# EUROPEAN PATENT SPECIFICATION

(11) **EP 1 670 773 B1**
(45) Date of publication and mention of the grant of the patent: **07.02.2007**
(21) Application number: 04766869.4
(22) Date of filing: 30.09.2004
(51) Int. Cl.: C07D 263/58

(54) **METHODS FOR THE PREPARATION OF BENZOXAZOLE SULFONAMIDE COMPOUNDS AND INTERMEDIATES THEREOF**
VERFAHREN ZUR HERSTELLUNG VON BENZOXAZOLSULFONAMIDVERBINDUNGEN UND ZWISCHENPRODUKTEN DAVON
METHODES POUR LA PREPARATION DES SULFONAMIDES DE LA BENZOXAZOLE ET PRODUITS INTERMEDIAIRES

(30) Priority: 30.09.2003 EP 03103630; 02.10.2003 US 507996 P
(43) Date of publication of application: 21.06.2006
(73) Proprietor: Tibotec Pharmaceuticals Ltd., Eastgate Little Island Co Cork (IE)
(72) Inventor: DE KOCK, Herman Augustinus, B-2370 Arendonk (BE); FILLIERS, Walter Ferdinand Maria, B-2531 Vremde (BE); AELTERMAN, Wim Albert Alex, B-2275 Gierle (BE)
(74) Representative: Daelemans, Frank F.R.
(86) International application number: PCT/EP2004/052382
(87) International publication number: WO 2005/030739

(56) References cited:
- WO-A-01/77092
- WO-A-02/092595

## Description

### Field of the invention

The present invention relates to methods for the preparation of benzoxazole sulfonamide compounds as well as novel intermediates for use in said method. More in particular the invention relates to methods for the preparation of 2-amino-benzoxazole sulfonamide compounds which make use of 2-mercapto-benzoxazole sulfonamide intermediates, more in particular methods employing the intermediate 1-Benzyl-2-hydroxy-3-[isobutyl-(2-methylsulfanyl-benzoxazole-6-sulfonyl)-amino]-propyl)-carbamic ester, and to methods amenable to industrial scaling up. Said benzoxazole sulfonamide compounds are particularly useful as HIV protease inhibitors.

### Background

The virus causing the acquired immunodeficiency syndrome (AIDS) is known by different names, including T-lymphocyte virus III (HTLV-III) or lymphadenopathy-associated virus (LAV) or AIDS-related virus (ARV) or human immunodeficiency virus (HIV). Up until now, two distinct families have been identified, i.e. HIV-1 and HTV-2. Hereinafter, HIV will be used to generically denote these viruses.

One of the critical pathways in a retroviral life cycle is the processing of polyprotein precursors by retroviral protease. For instance, during the replication cycle of the HIV virus, gag and gag-pol gene transcription products are translated as proteins, which are subsequently processed by a virally encoded protease to yield viral enzymes and structural proteins of the virus core. Most commonly, the gag precursor proteins are processed into the core proteins and the pol precursor proteins are processed into the viral enzymes, e.g., reverse transcriptase and retroviral protease. Correct processing of the precursor proteins by the retroviral protease is necessary for the assembly of infectious virions, thus making the retroviral protease an attractive target for antiviral therapy. In particular for HIV treatment, the HIV protease is an attractive target.

Several protease inhibitors are on the market or are being developed. Benzoxazole sulfonamide HIV protease inhibitors, for example 2-amino-benzoxazole sulfonamides, have been described to have favourable pharmacological and pharmacokinetic properties against wild-type and mutant HIV virus. The particular core structure, 2-amino-benzoxazole sulfonamide, can generally be prepared using procedures analogous to those procedures described in WO 95/06030, WO 96/22287, WO 96/28418, WO 96/28463, WO 96/28464, WO 96/28465 and WO 97/18205. In particular, methods for preparing 2-amino-benzoxazole sulfonamides have been described in WO 02/092595. However, such methods are in general complex, experiencing a burdensome halosulfonation, and providing insufficient yields for commercial purposes. Therefore, there is a need in the art for improved methods for preparing 2-amino benzoxazole sulfonamide protease inhibitors, which overcome at least some of the above-mentioned problems.

The present invention provides improved methods for preparing a retrovirus protease inhibitor, in particular for preparing 2-amino-benzoxazole sulfonamides. In particular, the present invention provides novel intermediate compounds of formula (6), 2-mercapto-benzoxazole sulfonamides, which are useful as precursors in the synthesis of 2-amino-benzoxazole sulfonamides.

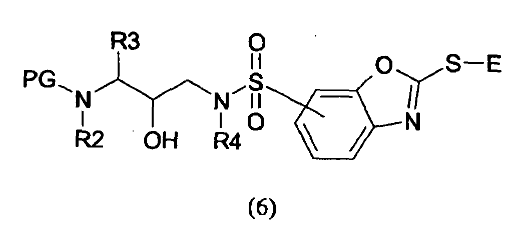

The use of compounds of formula (6) as intermediates allow the production of a broad and diverse range of 2-amino-benzoxazole sulfonamides, thus providing a broad range of HIV protease inhibitors starting from a single family of intermediates. Also, as exemplified below, the present method presents a convenient sulfonation and is consequently easy and cost-effective. Furthermore, another advantage of the present method is that acceptable yields for commercial purposes of 2-amino-benzoxazole sulfonamide protease inhibitors can be obtained. The present method has the further advantage of using commercially available starting material, such as a 2-mercaptobenzoxazole. The reagents further used in said method are safe and available in bulk. Furthermore, each step of said method provides with the desired compound in good yield. Moreover, each step of said method can be performed stereoselectively, which allows the synthesis of pure stereoisomeric forms of said compounds when using, where appropriate, optically pure starting material and reagents. Thus, the methods according to the present invention are amenable for industrial scaling up.

Other objects and advantages of the present invention will become apparent from the following detailed description taken in conjunction with the accompanying examples.

### Detailed description of the invention

The present invention involves methods for the synthesis of 2-amino-benzoxazole sulfonamides through the intermediates of formula (6) 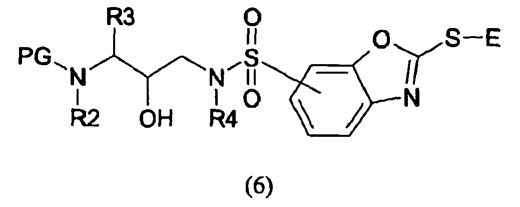 and salts, stereoisomeric forms, and racemic mixtures thereof, wherein
**PG** represents a protecting group;
**R₂** is hydrogen or C₁₋₆alkyl;
**R₃** is C₃₋₇cycloalkyl, aryl, Het¹, Het², or C₁₋₆alkyl optionally substituted with C₃₋₇cycloalkyl, aryl, Het¹, or Het²; wherein each C₃₋₇cycloalkyl, aryl, Het¹, and Het² may be optionally substituted with one or more groups selected from oxo, C₁₋₆alkyloxy, C₁₋₆alkyl, C₁₋₆alkylsulfonyl, aminosulfonyl, amino, C₁₋₆alkylcarbonylamino, hydroxyC₁₋₆alkyl, cyano, C₁₋₆alkyloxycarbonyl, aminocarbonyl, halogen or trifluoromethyl, wherein each amino maybe mono- or disubstitued with C₁₋₆alkyl;
**R₄** is selected from the group comprising hydrogen, C₁₋₄alkyloxycarbonyl, carboxyl, aminocarbonyl, mono- or di(C₁₋₄alkyl)aminocarbonyl, C₃₋₇cycloalkyl, C₂₋₆alkenyl, C₂₋₆alkynyl, or C₁₋₆alkyl optionally substituted with one or more substituents each independently selected from aryl, Het¹, Het², C₃₋₇cycloalkyl, C₁₋₄alkyloxycarbonyl, carboxyl, aminocarbonyl, mono- or di(C₁₋₄alkyl)aminocarbonyl, aminosulfonyl, C₁₋₄alkyl-S(=O)ₜ, hydroxy, cyano, halogen and amino optionally mono-or disubstituted where the substituents are each independently selected from C₁₋₄alkyl, aryl, arylC₁₋₄alkyl, C₃₋₇cycloalkyl, C₃₋₇cycloalkylC₁₋₄alkyl, Het¹, Het², Het¹C₁₋₄alkyl and Het²C₁₋₄alkyl;
**t** is zero, one or two; and
**E** represents an electrophilic moiety.
Intermediates of formula (6) may be prepared starting from compounds of formula (2), 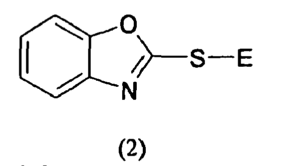 wherein
**E** is as described above;
transforming said intermediates of formula (2) into sulfonyl derivatives of formula (3), 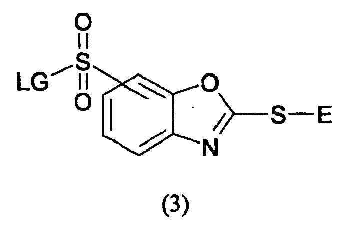 wherein
**LG** represents a leaving group;
subsequently reacting said sulfonyl derivatives with compounds of formula (5), 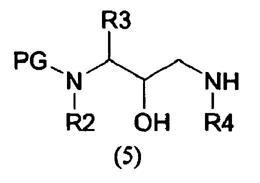 wherein
**PG, R₂, R₃,** and **R₄** are as described above;
thus obtaining intermediate compounds of formula (6). 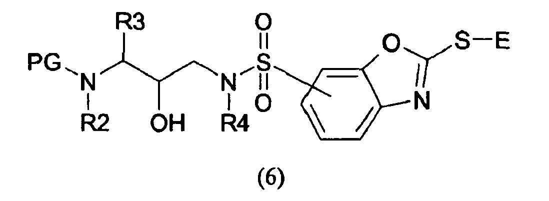 In a preferred embodiment, the present invention relates to a method for the synthesis of 2-amino-benzoxazole sulfonamides of formula (9), which comprises the steps of:
a) coupling an electrophilic moiety (**E**) to a 2-mercapto-benzoxazole of formula (1) 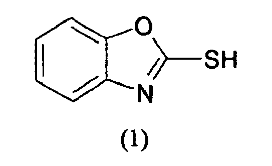 resulting into a compound of formula (2), wherein **E** is as described above; 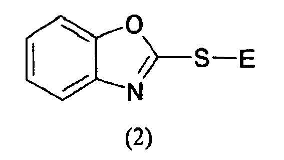
b) reacting said compound of formula (2) with a sulfonation agent and introducing a leaving group (**LG**), resulting in an intermediate of formula (3), wherein **LG** is as described above; 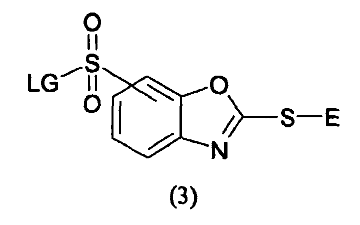
c) coupling said intermediate of formula (3) with a compound of formula (5), wherein **PG, R₂, R₃,** and **R₄** are as described above; 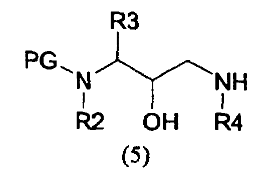 obtaining the intermediate of formula (6), 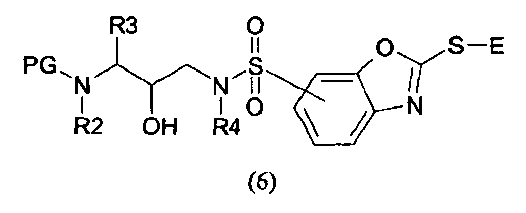
d) followed by an amination of compound of formula (6) to obtain 2-amino-benzoxazole sulfonamides of compound of formula (7), wherein 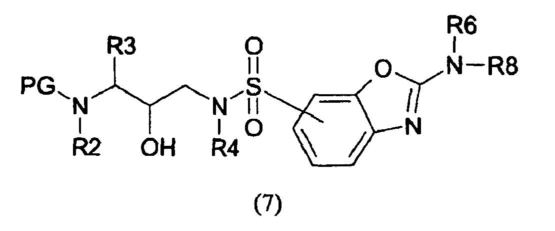
   **R₆** is hydrogen, hydroxy, C₁₋₆alkyl, Het¹C₁₋₆alkyl, Het²C₁₋₆alkyl, aminoC₁₋₆alkyl whereby the amino group may optionally be mono-or di-substituted with C₁₋₄alkyl;
   **R₈** is hydrogen, C₁₋₆alkyl, or **-A-R₇;**
   **A** is C₁₋₆alkanediyl, -C(=O)-, -C(=S)-, -S(=O)₂-, C₁₋₆alkanediyl-C(=O)-, C₁₋₆alkanediyl-C(=S)- or C₁₋₆alkanediyl-S(=O)₂-; whereby the point of attachment to the nitrogen atom is the C₁₋₆alkanediyl group in those moieties containing said group;
   **R₇** is C₁₋₆alkyloxy, Het¹, Het¹oxy, Het², Het²oxy, aryl, aryloxy, C₃₋₇cycloalkyl, or optionally mono- or disubstituted amino; and
   in case **-A-** is other than C₁₋₆alkanediyl then **R₇** may also be C₁₋₆alkyl, Het¹C₁₋₄alkyl, Het¹oxyC₁₋₄alkyl, Het²C₁₋₄alkyl, Het²oxyC₁₋₄alkyl, arylC₁₋₄alkyl, aryloxy-C₁₋₄alkyl or amino-C₁₋₆alkyl; whereby each of the amino groups in the definition of **R₇** may optionally be substituted with one or more substituents selected from C₁₋₄alkyl, C₁₋₄alkylcarbonyl, C₁₋₄alkyloxycarbonyl, aryl, arylcarbonyl, aryloxycarbonyl, Het¹, Het², arylC₁₋₄alkyl, Het¹-C₁₋₄alkyl or Het²C₁₋₄alkyl ; and
   **-A-R₇** may also be hydroxyC₁₋₆alkyl; and
   **R₆** and **-A-R₇** taken together with the nitrogen atom to which they are attached may also form Het¹ or Het²;
e) deprotecting compound of formula (7) to obtain 2-amino-benzoxazole sulfonamides of compound of formula (8), 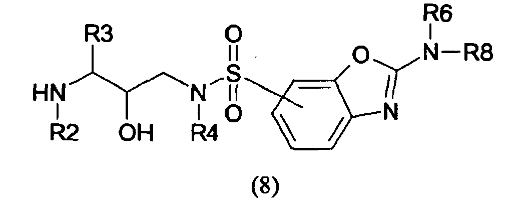
f) coupling a radical of formula **R₁-L-** to obtain the corresponding 2-amino-benzoxazole sulfonamide protease inhibitor of formula (9), 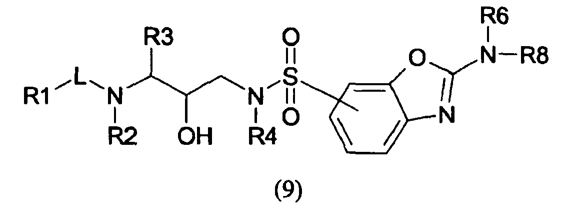 wherein
   **R₁** is selected from the group comprising hydrogen, C₁₋₆alkyl, C₂₋₆alkenyl, arylC₁₋₆alkyl, C₃₋₇cycloalkyl, C₃₋₇cycloalkylC₁₋₆alkyl, aryl, Het¹, Het¹C₁₋₆alkyl, Het², Het²C₁₋₆alkyl; and **R₁** may also be a radical of formula (10) 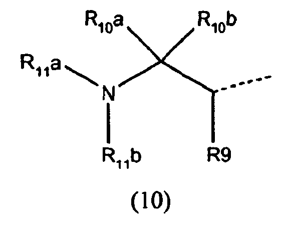
   wherein **R₉, R₁₀ₐ and R_{10b}** are, each independently, hydrogen, C₁₋₄alkyloxycarbonyl, carboxyl, aminocarbonyl, mono- or di(C₁₋₄alkyl)aminocarbonyl, C₃₋₇cycloalkyl, C₂₋₆alkenyl, C₂₋₆alkynyl or C₁₋₄alkyl optionally substituted with aryl, Het¹, Het², C₃₋₇cycloalkyl, C₁₋₄alkyloxycarbonyl, carboxyl, aminocarbonyl, mono- or di(C₁₋₄alkyl)aminocarbonyl, aminosulfonyl, C₁₋₄alkylS(O)ₜ, hydroxy, cyano, halogen or amino optionally mono- or disubstituted where the substituents are each independently selected from C₁₋₄alkyl, aryl, arylC₁₋₄alkyl, C₃₋₇cycloalkyl, C₃₋₇cycloaklC₁₋₄alkyl, Het¹, Het², Het¹C₁₋₄alkyl and
      Het²C₁₋₄alkyl; whereby R₉, R₁₀ₐ and the carbon atoms to which they are attached may also form a C₃₋₇cycloalkyl radical;
   **when L** is -O-C₁₋₆alkanediyl-C(=O)- or -NR₁₂-C₁₋₆alkanediyl-C(=O)-, **then R₉** may also be oxo;
   **R₁₁ₐ** is selected from the group comprising hydrogen, C₂₋₆alkenyl, C₂₋₆alkynyl, C₃₋₇cycloalkyl, aryl, aminocarbonyl optionally mono- or disubstituted, aminoC₁₋₄alkylcarbonyloxy optionally mono- or disubstituted, C₁₋₄alkyloxycarbonyl, aryloxycarbonyl, Het¹oxycarbonyl, Het²oxycarbonyl, aryloxycarbonylC₁₋₄alkyl, arylC₁₋₄alkyloxycarbonyl, C₁₋₄alkylcarbonyl, C₃₋₇cycloalkylcarbonyl, C₃₋₇cycloalkyl-C₁₋₄alkyloxycarbonyl, C₃₋₇cycloalkylcarbonyloxy, carboxylC₁₋₄alkylcarbonyloxy, C₁₋₄alkylcarbonyloxy, arylC₁₋₄alkylcarbonyloxy, arylcarbonyloxy, aryloxycarbonyloxy, Het¹carbonyl, Het¹carbonyloxy, Het¹C₁₋₄alkyloxycarbonyl, Het²carbonyloxy, Het²C₁₋₄alkylcarbonyloxy, Het²C₁₋₄alkyloxycarbonyloxy or C₁₋₄alkyl optionally substituted with aryl, aryloxy, Het² or hydroxy; wherein the substituents on the amino groups are each independently selected from C₁₋₄alkyl, aryl, arylC₁₋₄alkyl, C₃₋₇cycloalkyl, C₃₋₇cycloalkyl
      C₁₋₄alkyl, Het¹, Het², Het¹C₁₋₄alkyl and Het²C₁₋₄alkyl;
   **R_{11b}** is selected from the group comprising hydrogen, C₃₋₇cycloalkyl, C₂₋₆alkenyl,
      C₂₋₆alkynyl, aryl, Het¹, Het² or C₁₋₄alkyl optionally substituted with halogen, hydroxy, C₁₋₄alkylS(=O)ₜ, aryl, C₃₋₇cycloalkyl, Het¹, Het², amino optionally mono- or disubstituted where the substituents are each independently selected from C₁₋₄alkyl, aryl, arylC₁₋₄alkyl, C₃₋₇cycloalkyl, C₃₋₇cycloalkylC₁₋₄alkyl, Het¹, Het², Het¹C₁₋₄alkyl and Het²C₁₋₄alkyl;
   whereby **R_{11b}** may be linked to the remainder of the molecule via a sulfonyl group;
   **L** is selected from the group comprising -C(=O)-, -O-C(=O)-, -NR₁₂-C(=O)-, -O-C₁₋₆alkanediyl-C(=O)-, -NR₁₂-C₁₋₆alkanediyl-C(=O)-, -S(=O)₂-, -O-S(=O)₂-, - NR₁₂-S(=O)₂ whereby either the C(=O) group or the S(=O)₂ group is attached to the NR₂ moiety; whereby the C₁₋₆alkanediyl moiety is optionally substituted with a substituent selected from hydroxy, aryl, Het¹, and Het²; and
   **R₁₂** is hydrogen, C₁₋₆alkyl, C₂₋₆alkenyl, arylC₁₋₆alkyl, C₃₋₇cycloalkyl, C₃₋₇cycloalkylC₁₋₆alkyl, aryl, Het¹, Het¹C₁₋₆alkyl, Het², Het²C₁₋₆alkyl.

In a more preferred embodiment, the present invention relates to a method for the synthesis of 2-amino-benzoxazole sulfonamides of formula (9'), which comprises the steps of:
a) alkylating a 2-mercapto-benzoxazole of formula (1) 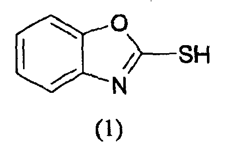 resulting into a 2-alkylthio-benzoxazole of formula (2), wherein **E** is C₁₋₆alkyl, preferably methyl; 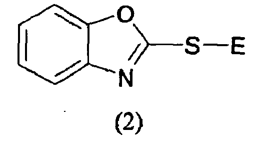
b) reacting said 2-alkylthio-benzoxazole of formula (2) with a sulfonation agent and introducing a leaving group (**LG**) resulting in an intermediate of formula (3), 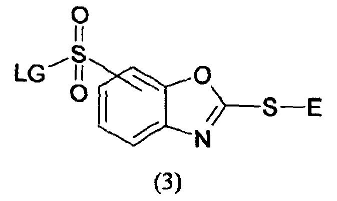
c) coupling said intermediate of formula (3) with a compound of formula (5'), wherein **R₂** is hydrogen, **R₃** is benzyl, and **R₄** is isobutyl; 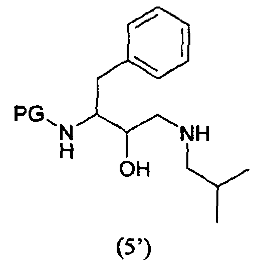 thus obtaining the intermediate of formula (6'), 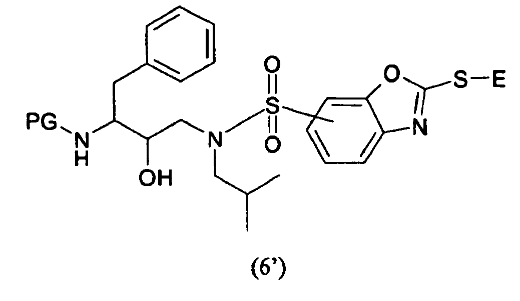
d) followed by an amination of compound of formula (6') to obtain 2-amino-benzoxazole sulfonamides of compound of formula (7'), 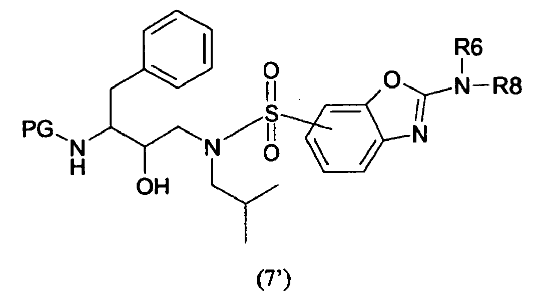 wherein **R₆** and **R₈** are as described above;
e) deprotecting compound of formula (7') to obtain 2-amino-benzoxazole sulfonamides of compound of formula (8'), 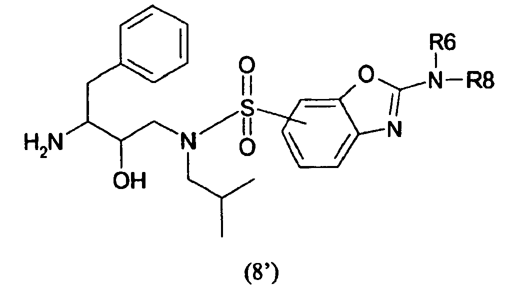
f) coupling a radical of formula **R₁-L-** for obtaining the corresponding 2-amino-benzoxazole sulfonamide protease inhibitor of formula (9'),
   wherein **R₁,** and **L** are as described above. 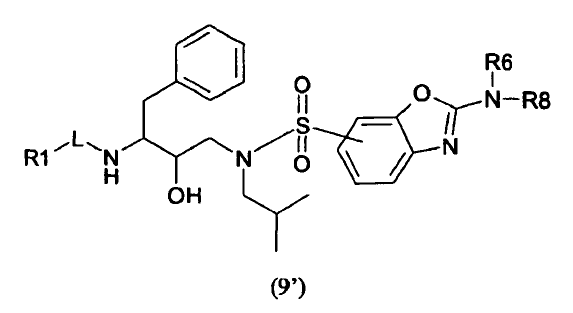 In a more preferred embodiment, said **R₁** is a Het¹, or a Het¹C₁₋₆alkyl, **L** is -O-C(=O)-, and **R₆** is hydrogen as indicated in formula (9") below.

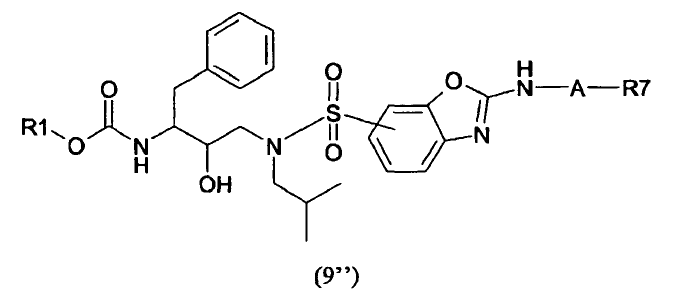

### Compound of formula (1)

Compound of formula (1), 2-mercapto-benzoxazole, may be directly purchased from commercially available sources, or may be prepared with procedures available in the art.

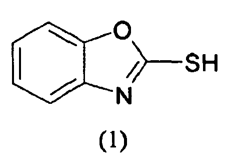

### Compounds of formula (2)

The 2-mercaptobenzoxazole, compound of formula (1), is subjected to a reaction with a suitable reagent to introduce an electrophilic moiety (**E**) which together with the -S-atom form a thiol-based leaving group (**-S-E**).

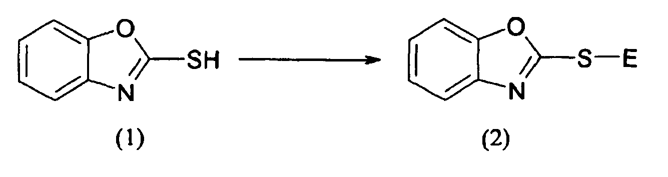

Said reagent is any material capable of providing to the reaction an electrophilic moiety (**E**) capable of reacting with the sulfur atom of the thiol (or mercapto or sulfhydryl) of the compound of formula (1) to form a new carbon sulfur bond thereby creating a thioether linkage, thus resulting in a thiol-based leaving group (**-S-E**).

The term "leaving group" is an atom or group of atoms which is displaceable upon reaction with an appropriate nucleophile. Such leaving groups are well known in the art. The term "electrophilic moiety" is so used to describe the electron deficient center moiety of an electrophile.

Preferred electrophiles for introducing electrophilic moieties are the alkylating agents which include, but are not limited to, C₁₋₆alkyl halides such as methyl iodide, ethyl iodide, n-propyl iodide, butyl iodide, methyl bromide, ethyl bromide, n-propyl bromide, and pentyl bromide; cycloC₃₋₇alkyl halides such as cyclohexyl bromide, and cyclopentylmethyl bromide; aryl-C₁₋₄alkyl halides such as 2-bromobenzyl bromide, 2-bromobenzyl chloride and the like; di-C₁₋₆alkyl sulfates such as dimethyl sulfate, diethyl sulfate, and di-n-propyl sulfate;
C₁₋₆alkylsulfonates such as ethyl methanesulfonate, n-propyl methanesulfonate; arylsulfonates; C₁₋₆alkyltoluenesulfonates such as methyl-p-toluenesulfonate; and the like. Other examples of electrophiles include acetic anhydride, trimethylacetyl chloride, butanoic anhydride, methyl succinoyl chloride, t-butyl succinoyl chloride, diethyldicarbonate, dimethyldicarbonate, benzoyl chloride, acetylacetoxy derivatives, haloacetamide derivatives, and the like. Other electrophiles include derivatives of epoxides, oxetanes, aziridines, azetidines, episulfides, maleimides, 2-oxazolin-5-ones, N-hydroxysuccinimides, mesylates, tosylates, nosylates, brosylates, isothiocyanates, electron-deficient aromatic rings, such as nitro-substituted pyrimidine rings, etc. Most preferred electrophiles are C₁₋₆alkylating agents. A particular suitable C₁₋₆alkylating agent is methyliodide which can be dissolved in customary solvents. Alternatively, ethyltosylate may be used as C₁₋₆alkylating agent.

In still other embodiments, the electrophile may be a group wherein, upon reaction with the nucleophilic S, an addition reaction takes place, leading to the formation of a covalent bond, for example with electron-deficient alkenes, such as α,β-unsaturated carbonyls, vinylsulfones.

The introduction of an electrophilic moiety (**E**) is carried out in the presence of conventional non-nucleophilic inorganic or organic bases. These include, for example, the hydrides, hydroxides, amides, alcoholates, acetates, carbonates, or hydrogen carbonates of alkaline earth metals or alkali metal hydrides such as, for example, sodium hydride, potassium hydride or calcium hydride, and metal amides, such as sodium amide, potassium amide, lithium diisopropylamide or potassium hexamethyldisilazide, and metal alkanes such as sodium methylate, sodium ethylate, potassium tert-butylate, sodium hydroxide, potassium hydroxide, ammonium hydroxide, sodium acetate, potassium acetate, calcium acetate, ammonium acetate, sodium carbonate, sodium bicarbonate, potassium carbonate, potassium bicarbonate, cesium carbonate, potassium hydrogen carbonate, sodium hydrogen carbonate, or ammonium carbonate, and also basic organic nitrogen compounds such as, trialkylamines, like trimethylamine, triethylamine, tributylamine, N,N-dimethylaniline, N,N- dimetbyl-benzylamine, N,N-diisopropylethylamine, pyridine, 1,4-diazabicyclo[2.2.2]-octane (DABCO), 1,5-diazabicyclo[4.3.0]-non-5-ene (DBN), or 1,8- diazabicyclo[5.4.0]-undec-7-ene (DBU), or an excess of an appropriate piperidine compound may be used. The base is preferably potassium carbonate, sodium carbonate, a sodium C₁₋₆alkoxide (e.g. sodium methoxide, sodium ethoxide, etc.), 1,1,3,3-tetramethylguanidine, sodium hydride, triethylamine and the like.

Suitable solvents for use in the introduction of an electrophilic moiety (E) include any one which does not disturb the reaction, such as aliphatic, alicyclic or aromatic, optionally halogenated hydrocarbons such as, for example, benzene, toluene, xylene, chlorobenzene, dichlorobenzene, petroleum ether, hexane, cyclohexane, dichloromethane, chloroform, tetrachloromethane; ethers such as diethyl ether, diisopropyl ether, dimethoxyethane, dioxane, tetrahydrofuran or ethylene glycol dimethyl ether or ethylene glycol diethyl ether; ketones such as acetone, butanone, or methyl isobutyl ketone; nitriles such as acetonitrile, propionitrile or benzonitrile; amides such as N,N-dimethylformamide, N,N- dimethylacetamide, N-methylformanilide, N-methylpyrrolidone, 1,3-dimethyl-2-imidazolidinone, or hexamethylphosphoric triamide; esters such as methyl acetate or ethyl acetate; sulfoxides such as dimethyl sulfoxide; alcohols such as methanol, ethanol, n- or i-propanol, n-, i-, s-, or t-butanol, ethylene glycol monomethyl ether, ethylene glycol monoethyl ether, diethylene glycol monomethyl ether, diethylene glycol monoethyl ether; or mixtures of these solvents. Preferably the alkylation reaction is carried out in suitable aprotic solvents such as dimethylformamide, acetonitrile, N-methylpyrrolidone, dimethylsulfoxide; ethers such as tetrahydrofuran, 2- methyltetrahydrofuran, methyl t-butyl ether, diethyl ether, dioxane; or esters such as ethyl acetate, or mixtures thereof.

In an embodiment, the introduction of an eletrophilic moiety is exemplified with an C₁₋₆alkylation reaction, which is suitably carried out at a temperature in the range from about -30°C to about 180°C, preferably at a temperature of from about 10°C to about 70°C, more preferably at a temperature of from about 10°C to about 55° C, even more preferably at a temperature of from about 15° C to about 40° C, being room temperature most preferred.

The ratios of equivalents between the 2-mercaptobenzoxazole and the C₁₋₆alkylating agent may range from 1:1 to 1:5, respectively. Preferably, the ratio of equivalents between the 2-mercaptobenzoxazole and the C₁₋₆alkylating agent is from 1:1 to 1:2, more preferably the ratio is around 1:1.1. The ratios of equivalents between the 2-mercaptobenzoxazole and the base may range from 1:1 to 1:5, preferably the ratio of equivalents ranges from 1:1.1 to 1:2, more preferably the ratio is around 1.3.

In an embodiment of the invention, the alkylation reaction is carried out in the presence of about 1.1 equivalents of methyl iodide, 1.3 equivalents of potassium carbonate and ethyl acetate, at ambient temperature, and stirring around 24 hours.

Alternative alkylating reactions encompass the use of Grignard reagents. Alkylating reactions are further described in Organic Synthesis, Vol. 31, pages 90-93, John Wiley & Sons, Inc., New York, New York.

### Compounds of formula (3)

Sulfonyl derivatives of formula (3) are prepared as illustrated in following scheme.

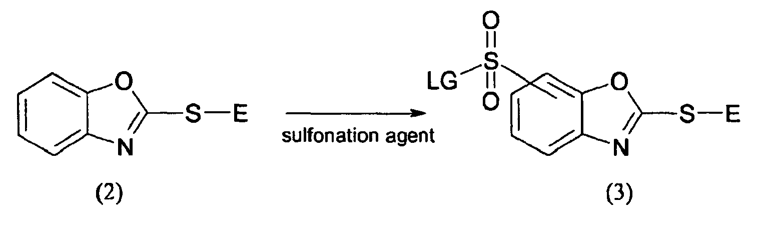

Sulfonation of an intermediate of formula (2) may be performed by any conventionally known method. As used herein, the term "sulfonation" means methods of introducing a sulfonyl moiety -SO₂- into a molecule.

Typical sulfonation agents are methanesulfonyl chloride, trifluoromethanesulfonyl chloride, trifluoromethanesulfonic anhydride, sulfonyl chloride, concentrated sulfuric acid (the sulfuric acid of about 70 wt % or higher is more preferable), sulfuric anhydride, fuming sulfuric acid, chlorosulfonic acid, sulfonated pyridine salt, sulfamic acid, amidosulfuric acid, fluorosulfuric acid, chlorosulfuric acid, sulfur trioxide, fuming sulfur, sulfuric acid, oleum, and sulfonation agents commonly employed in electrophilic aromatic substitutions, which can be used singly or in combinations of two or more types.

The sulfonation is simultaneously or subsequently followed with the insertion of a leaving group (**LG**), to form the moiety **LG-SO₂-.** Alternatively, the sulfonation agent has the leaving group already incorporated. Agents for the insertion of a leaving group are halogenating reagents such as, phosphorous chloride, phosphoric chloride, thionly chloride, phosphorus bromide, acetyl chloride, methyl chloroformate, methanesulfonyloxy chloride or an oxide.

Suitable leaving groups (LG) include alkoxy carbonyl groups such as ethoxy carbonyl; halogens such as iodine, bromine or chlorine, fluorine; substituted or unsubstituted saturated or unsaturated thiolates, such as thiomethyl or thiophenyl; substituted or unsubstituted saturated or unsaturated selenino compounds, such as phenyl selenide or alkyl selenide; or -OR_{z} where **R_{z}** is a substituted or unsubstituted saturated or unsaturated alkyl group, e.g., a C₁₋₆alkyl or alkenyl group such as methyl; a substituted or unsubstituted aliphatic or aromatic acyl group, e.g., a C₁₋₆aliphatic acyl group such as acetyl and an aromatic acyl group such as benzoyl; a substituted or unsubstituted saturated or unsaturated alkoxy carbonyl group, such as methyl carbonate and phenyl carbonate; substituted or unsubstituted sulphonyl imidazolide; substituted or unsubstituted carbonyl imidazolide; substituted or unsubstituted aliphatic or aromatic amino carbonyl group, such as phenyl carbamate; substituted or unsubstituted alkyl imidate group such as trichloroacetamidate; substituted or unsubstituted saturated or unsaturated phosphinoyl, such as diethylphosphinoyl; substituted or unsubstituted aliphatic or aromatic sulphonyl group, such as tosylate. Preferred leaving groups are halogen atoms such as bromo, fluoro and chloro, more preferably, chloro.

The treatment of compounds of formula (2) with the sulfonation agent can be carried out under heating (approximately between 25° to 250° C, preferably between 70° and 100°) and agitation. After the sulfonation treatment, the solvent and any remaining sulfonation agent are removed from the slurry thus obtained. This removal can be accomplished by repeated washing with water, ultrafiltration, reverse osmosis, centrifugation, and/or filtration or the like.

The sulfonation procedures applicable for the preparation of sulfonated benzoxazoles can also be found in "Sulfonation and Related Reactions", by E. E. Gilbert, R. E. Krieger Publishing Co. Huntington, N.Y. (1977), "Mechanistic Aspects of Aromatic Sulfonation and Desulfonation", by H. Cerfontain, Interscience Publishers, NY (1968), and in US6455738, "Process for the sulfonation of an aromatic compound", all incorporated herein by reference.

In particular, halosulfonyl benzoxazoles can be prepared by the reaction of a suitable Grignard or alkyl lithium reagent with sulfuryl chloride, or sulfur dioxide followed by oxidation with a halogen, preferably chlorine. Also, thiols may be oxidized to sulfonyl chlorides using chlorine in the presence of water under carefully controlled conditions. Additionally, sulfonic acids may be converted to sulfonyl halides using reagents such as PCl₅, and also to anhydrides using suitable dehydrating reagents. The sulfonic acids may in turn be prepared using procedures well known in the art. Such sulfonic acids are also commercially available.

Alternatively, the 2-amino-chlorosulfonylbenzoxazole derivative of formula (3) may be prepared following the procedure described in EP0445926.

Similar methods may be employed for the sulfonation of benzoxazole derivatives in the 4, 5, 6, and 7 positions. However, substitution of the sulfonyl group on the C-6 position of the benzoxazole derivative of formula (2) is preferred, as shown in formula (3"') below.

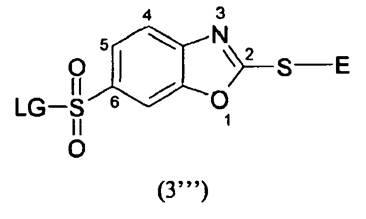

Conveniently, the ratios of equivalents between the compound of formula (2) and the sulfonation agent range from 1:2 to 1:8, respectively. Preferably, the ratio of equivalents between the compound of formula (2) and the sulfonation agent is from 1:3 to 1:5, more preferably the ratio is around 1:4.3. The ratios of equivalents between the compound of formula (2) and the agent for coupling a suitable leaving group range from 1:1 to 1:5, respectively. Preferably, the ratio of equivalents between the compound of formula (2) and the agent for coupling a suitable leaving group is from 1:1.1 to 1:3, more preferably the ratio is around 1:1.2.

In an embodiment of the invention, the sulfonation reaction is carried out in the presence of about 4.27 equivalents of chlorosulfonic acid, 1.2 equivalents of thionyl chloride and ethyl acetate, by stirring the chlorosulfonic acid under nitrogen, adding compound of formula (2) at a temperature below 60°, stirring overnight at around 85°C, cooling down to a temperature below 65°C and adding around 1.2 equivalents of thionyl chloride and stirring overnight at a temperature around 65°C.

In an embodiment the halogenating agent is sulfonylchloride, resulting in the sulfonylchloride of formula (3'), wherein E is selected from the group as defined above.

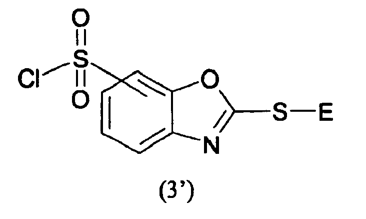

A preferred embodiment is the chlorosulfonation of intermediate of formula (2) by reacting the intermediate at a temperature of 50 to 130 °C in an organic solvent of dichloromethane, 1,2-dichloroethane, 1,1,2,2-tetrachloroethane, etc., or without organic solvent, in the presence of 2.5 to 5.0 equivalents of chlorosulfonic acid. Also, in the reaction, though it is variable depending on the E moiety, 2-substituted sulfonic acid is obtained as a product along with 2-substituted sulfonylchloride (formula 3') in the form of mixture. Without an isolating step, the mixture is preferably treated with a chlorination reagent of SOCl₂, to obtain 2-substituted sulfonylchloride (formula 3') only. Alternatively, the mixture can be isolated by recrystallization to give a pure 2-substituted sulfonic acid which is then treated with a chlorination reagent of SOCl₂ to be converted into 2-substituted sulfonylchloride (formula (3')).

In an embodiment the sulfonyl derivatives of formula (3) is a compound of formula (3"'), wherein E and LG are selected from the groups as defined above.

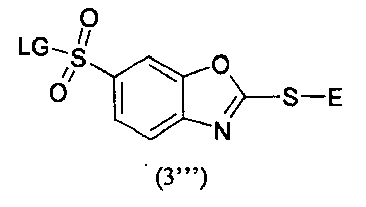

### Compounds of formula (5)

Compound of formula (5) may be obtained by amination of an epoxide-containing compound of formula (4) in the presence of a suitable solvent system. Compound of formula (4) additionally encompasses a protecting group moiety (**PG**) for protecting the amino moiety.

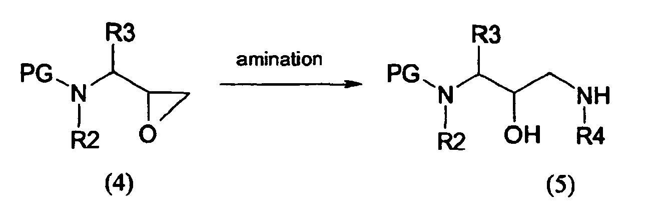

Compound of formula (4) may be prepared in several ways available in the literature, for example as described in WO95/06030, which is incorporated herein by reference.

The term "amination" as used herein refers to a process in which an amino group or substituted amine is introduced into an organic molecule. Amination of epoxides is described for instance in March, Advanced Organic Chemistry 368-69 (3rd Ed. 1985) and McManus et al., 3 Synth. Comm. 177 (1973), which are incorporated herein by reference. Suitably, compound of formula (5) may be prepared according to the procedure described in WO97/18205.

Amination agents which are used in the reaction include ammonia, ammonia generating compounds or organic amines. The ammonia generating compounds are inorganic compounds which generate ammonia gas on thermal decomposition thereof. Such inorganic compounds include ammonium carbonate, ammonium sulfate, etc. The organic amines include primary amine or secondary amines, such as methylamine, ethylamine, n-propylamine, butylamine, ethanolamine, dialkylamine such as dimethylamine, diethylamine, diisopropylamine, diethanolamine, methylethylamine, cyclohexylamine, aminopyridine, aniline, methylaniline, ethylaniline, n- propylaniline, isopropylaniline, dimethylaniline, diethylaniline, dipropylaniline, methylethylaniline, methylpropylanlline, etc. Tertiary amines may as well be employed for introducing strongly basic ion exchange groups, and are, for example, trialkylamines such as trimethylamine or triethylamine, or triethanolamine. Also diamines are useful such as alkylene diamines, preferably 1,3- diaminopropane, 1,4-diaminobutane or 1,6-diaminohexane. A preferred amination agent is a polyamine or oligoamine such as H-(NH-CH2- CH2)q-NH2, wherein q is a digit from I up to 10. Another preferred amination agent is isobutylamine.

Suitable solvent systems include protic, non-protic and dipolar aprotic organic solvents such as, for example, those wherein the solvent is an alcohol, such as methanol, ethanol, isopropanol, n-butanol, t-butanol, and the like, ethers such as tetrahydrofuran, dioxane and the like, toluene, N,N-dimethylformamide, dimethyl sulfoxide, and mixtures thereof. A preferred solvent is isopropanol.

Compounds of formula (4) additionally comprise an amino-protecting group. The term "amino-protecting group" as used herein refers to one or more selectively removable substituents on the amino group commonly employed to block or protect the amino functionality against undesirable side reactions during synthetic procedures and includes all conventional amino protecting groups. Examples of amino-protecting groups include the urethane blocking groups, such as t-butoxy-carbonyl ("Boc"), 2-(4-biphenylyl)propyl(2)oxycarbonyl ("Bpoc"), 2-phenylpropyl(2)oxycarbonyl ("Poc"), 2-(4-xenyl)isopropoxycarbonyl, isopropoxycarbonyl, 1,1-diphenyletbyl(1)-oxycarbonyl, 1,1-diphenylpropyl(1)oxycarbonyl, 2-(3,5-dimethoxyphenyl)propyl(2)-oxycarbonyl ("Ddz"), 2-(p-5-toluyl)propyl(2)oxycarbonyl, 1-methylcyclopentanyloxycarbonyl, cyclohexanyloxycarbonyl, 1-methylcyclohexanyloxycarbonyl, 2-methylcyclohexanyloxycarbonyl, ethoxycarbonyl, 2-(4-toluylsulfonyl)ethoxycarbonyl, 2-(methylsulfonyl)-ethoxycarbonyl, 2-(triphenylphosphino)-ethoxycarbonyl, 9-fluoroenylmethoxycarbonyl ("Fmoc"), 2-(trimethylsilyl)ethoxycarbonyl, allyloxycarbonyl, 1-(trimethylsilylmethyl)prop-1-enyloxycarbonyl, 5-benzisoxalylmethoxycarbonyl, 4-acetoxybenzyloxycarbonyl, 2,2,2- trichloroethoxycarbonyl, tribromoethoxycarbonyl, 2-ethynyl(2)propoxycarbonyl, cyclopropylmethoxycarbonyl, isobornyloxycarbonyl, 1-piperidyloxycarbonyl, benzyloxycarbonyl ("Z" or "Cbz"), 4-phenylbenzyloxycarbonyl, 2-methylbenzyloxy-carbonyl, α-2,4,5,-tetramethylbenzyloxycarbonyl ("Tmz"), 4-methoxybenzyloxycarbonyl, 4-fluorobenzyloxycarbonyl, 4-chlorobenzyloxycarbonyl, 3-chlorobenzyloxycarbonyl, 2-chlorobenzyloxycarbonyl, dichlorobenzyloxycarbonyl, 4-bromobenzyloxycarbonyl, ortho-bromobenzyloxycarbonyl, 3-bromobenzyloxycarbonyl, 4-nitrobenzyloxycarbonyl, 4-cyanobenzyloxycarbonyl, 4-(decyloxy)benzyloxycarbonyl, and the like; the benzoylmethylsulfonyl group, dithiasuccinoyl ("Dts") group, the 2-(nitro)phenylsulfenyl group ("Nps"), the diphenylphosphine oxide group, and the like. The species of amino-protecting group employed is usually not critical so long as the derivatized amino group is stable to the conditions of the subsequent reactions and can be removed at the appropriate point without disrupting the remainder of the compound.

Additional examples of amino protecting groups include phenylacetyl, formyl ("For"), trityl (Trt), acetyl, trifluoroacetyl (TFA), trichloroacetyl, dichloroacetyl, chloroacetyl, bromoacetyl, iodoacetyl, benzoyl, tert-amyloxycarbonyl, tert-butoxycarbonyl, 3,4-dimethoxybenzyloxycarbonyl, 4-(phenylazo)benzyloxycarbonyl, 2-furfuryloxycarbonyl, diphenylmethoxycarbonyl, 1,1-dimethylpropoxycarbonyl, phthalyl or phthalimido, succinyl, alanyl, leucyl, and 8-quinolyloxycarbonyl, benzyl, diphenylmethyl, 2-nitrophenylthio, 2,4- dinitrophenylthio, methanesulfonyl, paratoluenesulfonyl, N,N-dimethylaminomethylene, benzylidene, 2-hydroxybenzylidene, 2-hydroxy-5-chlorobenzylidene, 2-hydroxy-1-naphthylmethylene, 3-hydroxy-4-pyridylmethylene, cyclohexylidene, 2-ethoxycarbonylcyclohexylidene, 2-ethoxycarbonylcyclopentylidene, 2-acetylcyclohexylidene, 3,3-dimethyl-5-oxycyclohexylidene, diphenylphosphoryl, dibenzylphosphoryl, 5-methyl-2-oxo-2H-1,3-dioxol- 4-yl-methyl, trimethylsilyl, triethylsilyl, triphenylsilyl, 2-(p-biphenyl)-1-methylethoxycarbonyl, diisopropylmethoxycarbonyl, cyclopentyloxycarbonyl, adamantyloxycarbonyl, triphenylmethyl, trimethylsilane, phenylthiocarbonyl, paranitrobenzylcarbonyl.

Other amino protecting groups include 2,7-di-t-butyl-[9-(10,10-dioxo-10,10,10,10-tetrahydrothio-xanthyl)]methyloxycarbonyl; 2-trimethylsilylethyloxycarbonyl; 2-phenylethyloxycarbonyl; 1,1-dimethyl-2,2-dibromoethyloxycarbonyl; 1-methyl-1-(4-biphenylyl)ethyloxycarbonyl; p-nitrobenzyloxycarbonyl; 2-(p-toluenesulfonyl)-ethyloxycarbonyl; m-chloro-p-acyloxybenzyloxycarbonyl; 5-benzyisoxazolylmethyloxycarbonyl; p-(dihydroxyboryl)benzyloxycarbonyl; m-nitrophenyloxycarbonyl; o-nitrobenzyloxycarbonyl; 3,5-dimethoxybenzyloxycrbonyl; 3,4-dimethoxy-6-nitrobenzyloxycarbonyl; N'-p-toluenesulfonylaminocarbonyl; t-amyloxycarbonyl; p-decyloxybenzyloxycarbonyl; 2,2-dimethoxycarbonylvinyloxycarbonyl; di(2-pyridyl)methyloxycarbonyl; 2-furanylmethyloxycarbonyl; dithiasuccinimide; 2,5-dimethylpyrrole; 5-dibenzylsuberyl; and, methanesulfonamide. Preferred amino-protecting groups are Boc, Z / Cbz and Fmoc.

Further examples of amino-protecting groups are well known in organic synthesis and the peptide art and are described by, for example T. W. Greene and P. G. M. Wuts, *Protective Groups in Organic Synthesis,* 2nd ed., John Wiley and Sons, New York, Chapter 7, 1991; M. Bodanzsky, *Principles of Peptide Synthesis,* 1 st and 2nd revised ed., Springer-Verlag, New York, 1984 and 1993; Stewart and Young, Solid *Phase Peptide Synthesis,* 2nd ed., Pierce Chemical Co, Rockford, IL 1984; L. Fieser and M. Fieser, *Fieser and Fieser's Reagents for Organic Synthesis,* John Wiley and Sons (1994); L. Paquette, ed. *Encyclopedia of Reagents for Organic Synthesis,* John Wiley and Sons (1995). Suitable amino protecting groups are also given in e.g. WO 98/07685.

In an embodiment the intermediate having formula (5) can be prepared by reacting intermediate compound of formula (4) with an amine of formula **H₂N-R₄,** wherein **R₄** is selected from the group as defined above. Exemplary amines corresponding to the formula **H₂N-R₄** include benzylamine, isobutylamine, n-butylamine, pentylamine, isoamylamine, cyclohexanemethylamine, naphthylenemethylamine and the like.

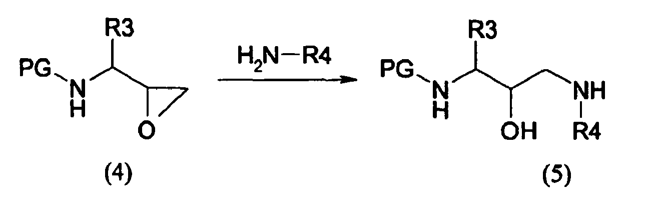

In this above scheme, enantiomerically pure compounds of formula (5) can only obtained if compound of formula (4) is enantiomerically pure. If compounds of formula (4) are a mixture of stereoisomers, then compounds of formula (5) will also consist of a mixture of stereoisomers.

Conveniently the reaction can be conducted over a wide range of temperatures, e.g., from about -20°C to about 200°C, but is preferably, but not necessarily, conducted at a temperature at which the solvent begins to reflux.

Suitably the ratios of equivalents between the compound of formula (4) and the amination agent may range from 1:1 to 1:99, respectively. Preferably, the ratio of equivalents between the compound of formula (4) and the amination agent is from 1:10 to 1:20, more preferably the ratio is around 1:14.

In an embodiment of the invention, the amination reaction is carried out in the presence of about 14 equivalents of isobutylamine, at ambient temperature, and stirring overnight at a temperature around 65°C.

### Compounds of formula (6)

Compound of formula (6) is obtained by coupling the intermediate of formula (3) with compound of formula (5), wherein the protecting group (**PG**), the substituents **R₂, R₃, R₄,** the leaving group (**LG**), and the electrophilic moiety (**E**) are as described above.

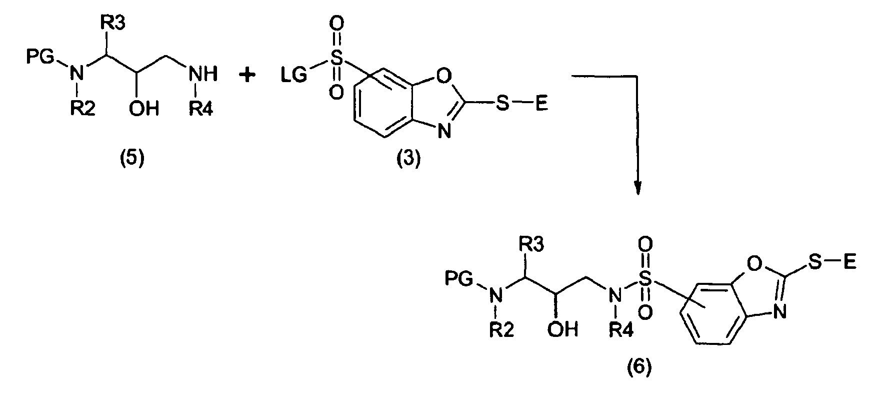

An alternative route to the preparation of formula (6) would consist of an amination of compound of formula (3) obtaining compound of formula (11) 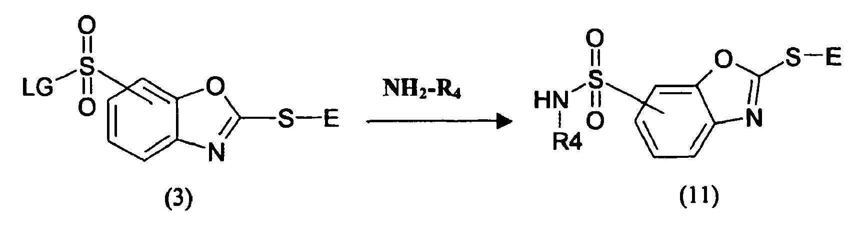 followed by attack by the amino function of compound of formula (11) onto the epoxide carbon atom of compound of formula (4) to yield compound of formula (6).

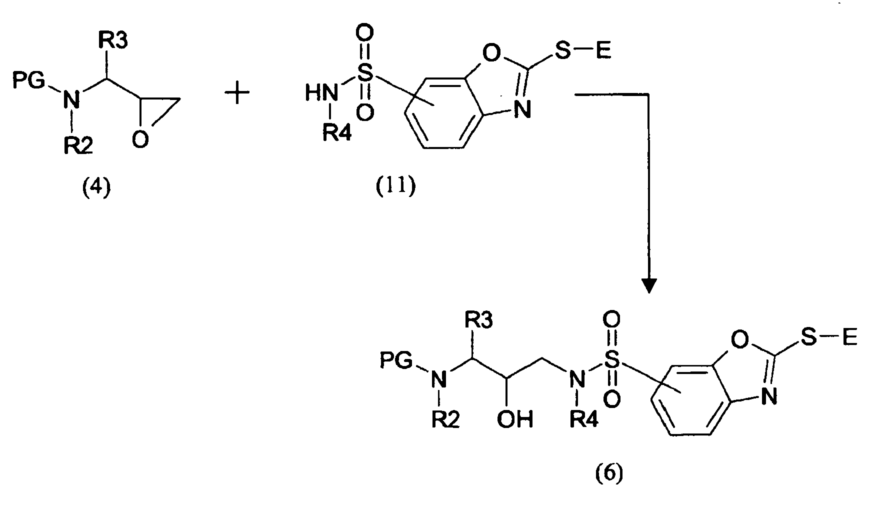

A particular group of compounds are those compounds of formula (6) wherein one or more of the following restrictions apply:
**R₂** is hydrogen;
**R₃** is arylC₁₋₄alkyl, in particular, arylmethyl, more in particular phenylmethyl;
**R₄** is unsubstituted C₁₋₆alkyl or C₁₋₆alkyl substituted with one or more substituents selected from aryl, Het¹, Het², C₃₋₇cycloalkyl and amino optionally mono-or disubstituted where the substituents are selected from C₁₋₄alkyl, aryl, Het¹ and Het².

A preferred group of compounds of formula (6) are those compounds where the sulfonamide group is attached to the benzoxazole group in the 6-position, as indicated in formula (6") below.

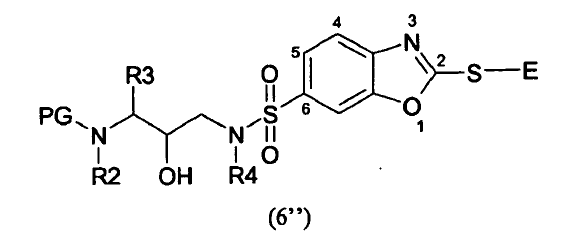

An interesting group of compounds are those of formula (6) wherein **R₂** is selected from the groups as defined above, wherein **R₃** is selected from the group comprising C₁₋₄alkyl, aryl, C₃₋₇cycloalkyl, C₃₋₇cycloalkylC₁₋₄alkyl, arylC₁₋₄akyl, and wherein **R₄** is hydrogen or C₁₋₄alkyl.

A suitable group of compounds are those compounds of formula (6), wherein **R₂** is hydrogen; **R₃** is arylC₁₋₄alkyl; and **R₄** is C₁₋₄alkyl; in particular, **R₂** is hydrogen; **R₃** is arylmethyl; and **R₄** is isobutyl.

A suitable group of compounds are those compounds of formula (6) as a salt, wherein the salt is selected from trifluoroacetate, fumarate, chloroacetate and methanesulfonate.

A particularly interesting compound according to the invention is the compound with formula (6"').

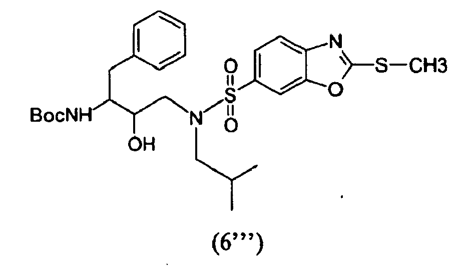

In a more preferred embodiment, the sulfonyl derivative of formula (3) is a sulfonylhalide of formula (3"), wherein X is fluoro, chloro, bromo, iodo, preferably chloro; said sulfonylhalide is reacted with an intermediate of formula (5'), wherein **R₂** is hydrogen, **R₃** is benzyl, and **R₄** is isobutyl, to yield a compound according to the invention having preferred formula (6"'), wherein **PG** is preferably Boc, and E is methyl.

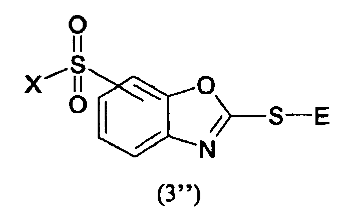

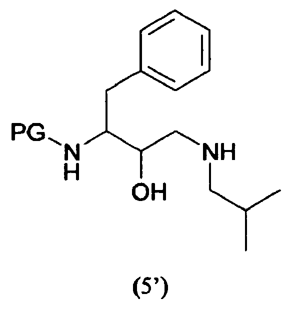

The present compounds according to the invention having general formula (6) are prepared by reacting a sulfonyl derivative of formula (3) with a suitable intermediate of formula (5) in suitable solvents under alkaline conditions. Suitable alkaline conditions include bases as the ones mentioned above and acid scavengers, such as triethylamine and pyridine. Suitable solvents have also been illustrated above, being inert solvents preferred, such as for example ethylacetate, methylene chloride, dichloromethane, and tetrahydrofuran.

The ratios of equivalents between the compound of formula (4) and compound of formula (3) may range from 1:1 to 1:8, respectively. Preferably, the ratio of equivalents between the compound of formula (4) and the compound of formula (3) is from 1:1.1 to 1:4, more preferably the ratio is around 1:1.2.

In an embodiment of the invention, the production of compound of formula (6) is carried out by stirring a solution of compound of formula (5) at a temperature above 65°C, adding the base, cooling down to 50°C and adding compound of formula (3) during 3 hours maintaining the reaction temperature between 40° and 50°C. In another embodiment, the synthesis of compound of formula (6) is performed at lower temperatures, for example from -20° to 150°C, preferably around room temperature.

Intermediates of formula (6) are also active inhibitors of retrovirus proteases.

### Compounds of formula (7)

Compound of formula (7) is obtained by amination of compound of formula (6) in the presence of an amination agent, and a solvent.

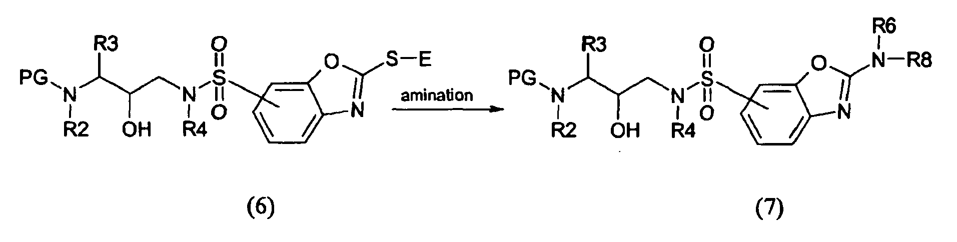

Suitable amination agents are as mentioned above, being methylamine preferred. Suitable solvents are as mentioned above, being isopropanol, and acetonitrile preferred.

The moieties **-R₆** and **-R₈** may be directly introduced by the amination agents, or subsequently introduced by a second reaction on the amino group.

The ratios of equivalents between the compound of formula (6) and the amination agent may range from 1:1.1 to 1:99, respectively. Preferably, the ratio of equivalents between the compound of formula (6) and the amination agent is around 1:35.

In an embodiment, compound of formula (7) is prepared by suspending compound of formula (6) in a solvent till complete dissolution. The amination agent is then added and the resulting solution is stirred and heated for 1 hour at a temperature between 20° and 180°C, preferably around 65°C.

### Compounds of formula (8)

Removal of the amino protecting group to obtain compound of formula (8) can be achieved using conditions which will not affect the remaining portion of the molecule. These methods are well known in the art and include acid hydrolysis, hydrogenolysis and the like, thus using commonly known acids in suitable solvents.

Examples of acids employed in the removal of the amino protecting group include inorganic acids such as hydrogen chloride, nitric acid, hydrochloric acid, sulfuric acid and phosphoric acid; organic acids such as acetic acid, trifluoroacetic acid methanesulfonic acid and p-toluenesulfonic acid; Lewis acids such as boron trifluoride; acidic cationic ion-exchange resins such as Dowex 50W^{™}. Of these acids, inorganic acids and organic acids are preferred. Hydrochloric acid, sulfuric acid, phosphoric acid and trifluoroacetic acid are more preferred, and hydrochloric acid is most preferred. Preferably, the acids employed are either 20% trifluoroacetic acid or hydrochloric acid, and the like, in methylene chloride or 4M HCl in dioxane.

The solvent employed is not particularly limited provided that it has no adverse effect on the reaction and dissolves the starting materials to at least some extent. Suitable solvents are aliphatic hydrocarbons such as hexane, heptane and petroleum ether; aromatic hydrocarbons such as benzene, toluene, xylene and mesitylene; halogenated hydrocarbons such as methylene chloride, chloroform, carbon tetrachloride and dichloroethane; ethers such as diethyl ether, tetrahydrofuran, 1,4-dioxane and 1,2-dimethoxyethane; alcohols such as methanol, ethanol, propanol, isopropanol and butanol; esters such as methyl acetate, ethyl acetate, methyl propionate and ethyl propionate; nitrites such as acetonitrile; amides such as N,N-dimethylformamide and N,N-dimethylacetamide; sulfoxides such as dimethyl sulfoxide and mixtures thereof. Aromatic hydrocarbons, alcohols and esters are preferred. Alcohols and esters are more preferred, and isopropanol, ethanol and ethyl acetate are particularly preferred. Alternatively, mixtures of ethanol and dioxane are also preferred.

The reaction temperature employed depends upon various factors such as the nature of the starting materials, solvents and acids. However it is usually between -20° C and 150° C, and is preferably between 10° C and 100° C. The reaction time employed depends on the reaction temperature and the like. It is typically from 5 minutes to 24 hours, and preferably from 10 minutes to 10 hours.

Examples of reagents and methods for deprotecting amines from amino protecting groups can additionally be found in *Protective Groups in Organic Synthesis* by Theodora W. Greene, New York, John Wiley and Sons, Inc., 1981, incorporated herein by reference.

As those skilled in the art will recognize, the choice of amino protecting group employed in a previous step of the process will dictate the reagents and procedures used in removing said amino protecting group.

The ratios of equivalents between the compound of formula (7) and the acid in solvent may range from 1:2 to 1:50, respectively. Preferably, the ratio of equivalents between the compound of formula (7) and the acid is from 1:2 to 1:8, more preferably the ratio is around 1:4.

In an embodiment of the invention, the removal of the amino protecting group of compound of formula (7) to generate compound of formula (8) is carried out by stirring a solution of compound of formula (7) in a suitable solvent at a temperature around 65°C, and adding the acid in solvent during 30 minutes. Preferably, prior to the stirring of a solution of compound of formula (7), an azeotropic distillation is applied in order to remove the content of water.

A preferred method involves removal of the protecting group, e.g., removal of a carbobenzoxy group, by hydrogenolysis utilizing palladium on carbon in a suitable solvent system such as an alcohol, acetic acid, and the like or mixtures thereof. Where the protecting group is a t-butoxycarbonyl group, it can be removed utilizing an inorganic or organic acid, e.g., HCl or trifluoroacetic acid, in a suitable solvent system, e.g., dioxane or methylene chloride. The resulting product is the amine salt derivative. Generally, the reaction is carried out at a temperature ranging from about 0° C to about 60° C. Generally, the reaction requires from about 1 to 24 hours. The deprotected amine of formula (8) may be isolated and purified by techniques well known in the art, such as extraction, evaporation, chromatography and recrystallization.

An alternative way of preparing compounds of formula (7), (8), and (9), wherein both **R₆** and **R₈** are hydrogen, can be performed by replacing one of **R₆** or **R₈** by a suitable amino-protecting group. In such a case, deprotection may occur simultaneously with the deprotection of the nitrogen atom on the left-hand side of the molecule.

### Compounds of formula (9)

Compound of formula (8) may be reacted with a suitable reagent to couple a radical of formula **R₁-L-,** thus obtaining the corresponding 2-amino-benzoxazole sulfonamide protease inhibitors.

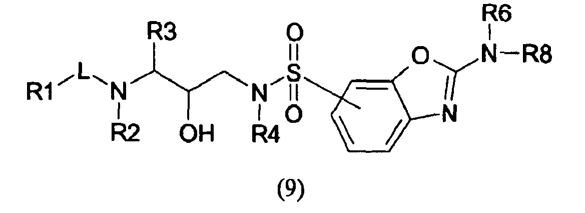

The coupling of a radical of formula **R₁-L-** may be performed in the presence of a base such as triethylamine (for alcohols to generate a carbamate) and optionally in the presence of 1-(3-dimethylaminopropyl)-3-ethylcarbodiimide hydrochloric acid (EDC) and 1-hydroxybenzotriazole (HOBT)(for carboxylic acids to generate an amide) or an alcohol such as *tert*-butanol, and in a suitable solvent such as dichloromethane. Reagents suitable to introduce radical of formula **R₁-L-** are reagents like **R₁-L-LG,** wherein **LG** is a leaving group, as described throughout the specification. Particularly, reagents of formula R₁-L-C(=O)-OH are suitable to couple radicals of formula **R₁-L-**into compounds of formula (8).

Compounds of formula (8) and (9) may as well be prepared as described in WO95/06030, and US-5,968,942, which are incorporated herein by reference.

An interesting group of compounds are those of formula (9) wherein
**R₁** is a radical of formula (10) 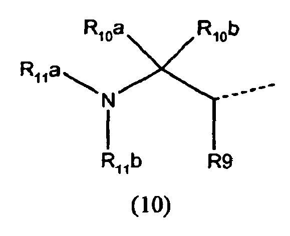
**R₉, R₁₀ₐ and R_{10b}** are, each independently, hydrogen, C₁₋₄alkyloxycarbonyl, carboxyl, aminocarbonyl, mono- or di(C₁₋₄alkyl)aminocarbonyl, C₃₋₇cycloalkyl, C₂₋₆alkenyl, C₂₋₆alkynyl or C₁₋₄alkyl optionally substituted with aryl, Het¹, Het², C₃₋₇cycloalkyl, C₁₋₄alkyloxycarbonyl, carboxyl, aminocarbonyl, mono- or di(C₁₋₄alkyl)aminocarbonyl, aminosulfonyl, C₁₋₄alkylS(O)ₜ, hydroxy, cyano, halogen or amino optionally mono- or disubstituted where the substituents are selected from C₁₋₄alkyl, aryl, arylC₁₋₄alkyl, C₃₋₇cycloalkyl, C₃₋₇cycloalkylC₁₋₄alkyl, Het¹, Het², Het¹C₁₋₄alkyl and Het²C₁₋₄alkyl; whereby R₉, R₁₀ₐ and the carbon atoms to which they are attached may also form a C₃₋₇cycloalkyl radical;
**R_{11b}** is hydrogen, C₃₋₇cycloalkyl, C₂₋₆alkenyl, C₂₋₆alkynyl, aryl, Het¹, Het² or C₁₋₄alkyl optionally substituted with halogen, hydroxy, C₁₋₄alkylS(=O)ₜ, aryl, C₃₋₇cycloalkyl, Het¹, Het², amino optionally mono- or disubstituted where the substituents are selected from C₁₋₄alkyl, aryl, arylC₁₋₄alkyl, C₃₋₇cycloalkyl, C₃₋₇cycloalkylC₁₋₄alkyl, Het¹, Het², Het¹C₁₋₄alkyl and Het²C₁₋₄alkyl; whereby R_{11b} may be linked to the remainder of the molecule via a sulfonyl group;
**t** is zero, one or two;
**L** is -C(=O)-, -O-C(=O)-, -NR₁₂-C(=_{O})-, -O-C₁₋₆alkanediyl-C(=O)-, -NR₁₂-C₁₋₆alkanediyl-C(=O)-, -S(=O)₂-, -O-S(=O)₂-, -NR₁₂-S(=O)₂ whereby either the C(=O) group or the S(=O)₂ group is attached to the NR₂ moiety;
**R₁₂** is hydrogen, C₁₋₆alkyl, C₂₋₆alkenyl, arylC₁₋₆alkyl, C₃₋₇cycloalkyl, C₃₋₇cycloalkyl-C₁₋₆alkyl, aryl, Het¹, Het¹C₁₋₆alkyl, Het², Het²C₁₋₆alkyl; and
**R₄** is hydrogen, C₁₋₄alkyloxycarbonyl, carboxyl, aminocarbonyl, mono- or di(C₁₋₄alkyl)aminocarbonyl, C₃₋₇cycloalkyl, C₂₋₆alkenyl, C₂₋₆alkynyl, or C₁₋₆alkyl optionally substituted with one or more substituents selected from aryl, Het¹, Het², C₃₋₇cycloalkyl, C₁₋₄alkyloxycarbonyl, carboxyl, aminocarbonyl, mono- or di(C₁₋₄alkyl)aminocarbonyl, aminosulfonyl, C₁₋₄alkylS(=O)ₜ, hydroxy, cyano, halogen and amino optionally mono- or disubstituted where the substituents are selected from C₁₋₄alkyl, aryl, arylC₁₋₄alkyl, C₃₋₇cycloalkyl, C₃₋₇cycloalkyl-C₁₋₄alkyl, Het¹, Het², Het¹C₁₋₄alkyl and Het²C₁₋₄alkyl.

A particular group of compounds are those compounds of formula (9) wherein one or more of the following restrictions apply:
**R₁** is hydrogen, Het¹, Het², aryl, Het¹C₁₋₆alkyl, Het²C₁₋₆alkyl, arylC₁₋₆alkyl, more in particular, R₁ is a saturated or partially unsaturated monocyclic or bicyclic heterocycle having 5 to 8 ring members, which contains one or more heteroatom ring members selected from nitrogen, oxygen or sulfur and which is optionally substituted, or phenyl optionally substituted with one or more substituents;
**R₂** is hydrogen;
**L** is -C(=O)-, -O-C(=O)-, -O-C₁₋₆alkanediyl-C(=O)-, more in particular, L is -O-C(=O)- or -O-C₁₋₆alkanediyl-C(=O)-, whereby in each case the C(=O) group is attached to the NR₂ moiety;
**R₃** is arylC₁₋₄alkyl, in particular, arylmethyl, more in particular phenylmethyl;
**R₄** is optionally substituted C₁₋₆alkyl, in particular unsubstituted C₁₋₆alkyl or C₁₋₆alkyl optionally substituted with one or more substituents selected from aryl, Het¹, Het², C₃₋₇cycloalkyl and amino optionally mono- or disubstituted where the substituents are selected from C₁₋₄alkyl, aryl, Het¹ and Het²;
**R₆** is hydrogen or methyl; and
**R₈** is hydrogen or methyl.

A special group of compounds are those compounds of formula (9) wherein **R₁-L** is Het¹-O-C(=O), Het²-C₁₋₆alkanediyl-O-C(=O), aryl-O-C₁₋₆alkanediyl-C(=O) or aryl-C(=O).

Also a special group of compounds are those compounds of formula (9) wherein **NR₆R₈** is amino, monomethylamino or dimethylamino.

Of particular interest are those compounds of formula (9) wherein **R₁** is hydrogen, C₁₋₆alkyl, C₂₋₆alkenyl, arylC₁₋₆alkyl, C₃₋₇cycloalkyl, C₃₋₇cycloalkylC₁₋₆alkyl, aryl, Het¹, Het¹C₁₋₆alkyl, Het², Het²C₁₋₆alkyl, in particular, **R₁** is hydrogen, C₁₋₆alkyl, C₂₋₆alkenyl, arylC₁₋₆alkyl, C₃₋₇cycloalkyl, C₃₋₇cycloalkylC₁₋₆alkyl, aryl, Het², Het²C₁₋₆alkyl.

An interesting group of compounds are those compounds of formula (9) wherein **R₁** is hydrogen, C₁₋₆alkyl, C₂₋₆alkenyl, arylC₁₋₆alkyl, C₃₋₇cycloalkyl, C₃₋₇cycloalkyl-C₁₋₆alkyl, aryl, Het¹, Het¹C₁₋₆alkyl, Het², Het²C₁₋₆alkyl; wherein Het¹ is a saturated or partially unsaturated monocyclic heterocycle having 5 or 6 ring members, which contains one or more heteroatom ring members selected from nitrogen, oxygen or sulfur and which is optionally substituted on one or more carbon atoms.

Another interesting group of compounds are those compounds of formula (9) wherein **L** is -O-C₁₋₆alkanediyl-C(=O)-.

A preferred group of compounds are those compounds where the sulfonamide group is attached to the benzoxazole group in the 6-position, as indicated in formula (9''') below.

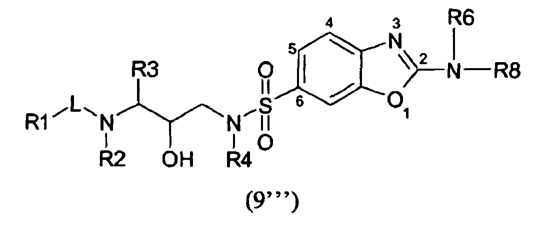

A suitable group of compounds are those compounds of formula (9) wherein **R₁** is aryl or arylC₁₋₆alkyl; in particular the aryl moiety of the **R₁** definition is further substituted on one or more ring members, whereby each substituent is independently selected from C₁₋₄alkyl, hydroxy, halogen, optionally mono- or disubstituted amino, optionally mono-or disubstituted aminoC₁₋₄alkyl, nitro and cyano; preferably the substituent is selected from methyl, ethyl, chlorine, iodine, bromine, hydroxy and cyano, in particular the aryl moiety contains 6 to 12 ring members, more in particular the aryl moiety in the definition of **R₁** contains 6 ring members.

A suitable group of compounds are those compounds of formula (9) wherein **R₁** is Het² or Het²C₁₋₆alkyl, wherein the Het² in the definition of **R₁** contains one or more heteroatoms each independently selected from nitrogen, oxygen and sulfur; in particular the Het² moiety of the R₁ definition is further substituted on one or more ring members, whereby each substituent is independently selected from C₁₋₄alkyl, hydroxy, halogen, optionally mono- or disubstituted amino and cyano; preferably the substituent is selected from methyl, ethyl, chlorine, iodine, bromine, hydroxy, amino and cyano.

Another group of compounds are those of formula (9) wherein **R₁** is Het² or Het²C₁₋₆alkyl, **L** is -C(-O)-, -O-C(=O)-, -O-C₁₋₆alkanediyl-C(=O)-; in particular the Het² moiety in the definition of **R₁** is an aromatic heterocycle having 5 or 6 ring members, which contain one or more heteroatom ring members each independently selected from nitrogen, oxygen or sulfur, more in particular the Het² moiety is an aromatic heterocycle having 5 or 6 ring members, which contain two or more heteroatom ring members each independently selected from nitrogen, oxygen or sulfur.

A suitable group of compounds are those compounds of formula (9) wherein **R₁** is Het¹C₁₋₆alkyl, Het¹, wherein said Het¹ in the definition of **R₁** is monocyclic having 5 or 6 ring members, wherein the Het¹ contains one or more heteroatoms each independently selected from nitrogen, oxygen and sulfur; in particular the Het¹ moiety of the **R₁** definition is further substituted on one or more carbon atoms, whereby each substituent is independently selected from C₁₋₄alkyl, hydroxy, halogen, optionally mono- or disubstituted amino and cyano; preferably the substituent is selected from methyl, ethyl, chlorine, iodine, bromine, hydroxy, amino and cyano.

A suitable group of compounds are those compounds of formula (9) wherein **R₁** is Het¹, wherein said Het¹ is bicyclic having 8 to 10 ring members, wherein the Het¹ contains one or more heteroatoms each independently selected from nitrogen, oxygen and sulfur; in particular the Het¹ moiety of the **R₁** definition is further substituted on one or more carbon atoms, whereby each substituent is independently selected from C₁₋₄alkyl, hydroxy, halogen, optionally mono- or disubstituted amino and cyano; preferably the substituent is selected from methyl, ethyl, chlorine, iodine, bromine, hydroxy, amino and cyano, in particular the Het¹ moiety contains 2 or more heteroatoms selected from nitrogen, sulfur and oxygen.

A suitable group of compounds are those compounds of formula (9) wherein **R₁** is Het¹, wherein said Het¹ is a satured bicyclic group having 5 to 10 ring members, wherein the Het¹ contains one or more heteroatoms each independently selected from nitrogen, oxygen and sulfur; in particular the Het¹ moiety of the **R₁** definition is further substituted on one or more carbon atoms, whereby each substituent is independently selected from C₁₋₄alkyl, hydroxy, halogen, optionally mono- or disubstituted amino and cyano; preferably the substituent is selected from methyl, ethyl, chlorine, iodine, bromine, hydroxy, amino and cyano; in particular Het¹ contains 5 to 8 ring members; in particular the Het¹ moiety has 6 to 8 ring members wherein Het¹ contains 2 or more heteroatoms selected from nitrogen, sulfur and oxygen.

An interesting group of compounds are those compounds of formula (9) wherein **R₁** is Het¹, Het², Het¹-C₁₋₆alkyl, or Het²-C₁₋₆alkyl, wherein Het¹ and Het² are selected from thiazolyl, imidazolyl, oxazolyl, oxadiazolyl, dioxazolyl, pyrazolyl, pyrazinyl, imidazolinonyl, quinolinyl, isoquinolinyl, indolyl, pyridazinyl, pyridinyl, pyrrolyl, pyranyl, pyrimidinyl, furanyl, triazolyl, tetrazolyl, benzofuranyl, benzoxazolyl, isoxazolyl, isothiazolyl, thiadiazolyl, thiophenyl, tetrahydrofurofuranyl, tetrahydropyranofuranyl, benzothiophenyl, carbazoyl, imidazolonyl, oxazolonyl, indolizinyl, triazinyl, quinoxalinyl, piperidinyl, piperazinyl, morpholinyl, thiamorpholinyl, pyrazinyl, thienyl, tetrahydroquinolinyl, tetrahydroisoquinolinyl, β-carbolinyl, dioxanyl, dithianyl, oxolanyl, dioxolanyl, tetrahydrothiophenyl, tetrahydropyranyl, tetrahydropyranyl; wherein Het¹ and Het² are optionally benzofused; wherein Het¹ and Het² are optionally further substituted on one or more ring members; preferably Het² is selected from thiazolyl, imidazolyl, oxazolyl, oxadiazolyl, pyrazolyl, pyridinyl, optionally substituted on one or more ring members.

A suitable group of compounds are those compounds of formula (9), wherein **R₂** is hydrogen; **R₃** is alkylaryl; and **R₄** is C₁₋₄alkyl; in particular, **R₂** is hydrogen; **R₃** is methylaryl; and **R₄** is isobutyl.

A suitable group of compounds are those compounds of formula (9) as a salt, wherein the salt is selected from trifluoroacetate, fumarate, chloroacetate and methanesulfonate.

A convenient way of preparing compounds of formula (9) wherein both **R₆** and **R₈** are hydrogen can be prepared analogously to the procedure described in scheme A, and whereby one of **R₆** or **R₈** is replaced by a suitable protecting group (**PG**) such as, for example, an acetyl or an alkyloxycarbonyl group, or any other as mentioned above. In such a case, deprotection may occur simultaneously with the deprotection of the nitrogen atom on the left-hand side of the molecule.

In a particular embodiment, the method for preparing a retrovirus protease inhibitor of the present invention, and in particular a 2-amino-benzoxazole sulfonamide protease inhibitor comprises the steps of
- reacting a compound of general formula (6) wherein **PG, R₂, R₃, R₄** and E are independently selected from the group as defined above, with ammonium to yield an intermediate of formula (7),
- deprotecting the obtained intermediate of formula (7) and
- reacting the deprotected intermediate of formula (8) in a suitable solvent with a suitable radical of formula **R₁-L-** for yielding a retrovirus protease inhibitor.

Example 2, provided below, illustrates the preparation of a 2-amino-benzoxazole sulfonamide protease inhibitor according to this method.

In another particular embodiment, the method for preparing a retrovirus protease inhibitor, and in particular a 2-amino-benzoxazole sulfonamide protease inhibitor comprises the steps of
- reacting a compound of general formula (6) wherein **PG, R₂, R₃, R₄** and **E** are independently selected from the group as defined above, with methyl amine to yield an intermediate of formula (7),
- deprotecting the obtained intermediate of formula (7), and
- reacting the deprotected intermediate of formula (8) in a suitable solvent with a suitable radical of formula **R₁-L-** for yielding a retrovirus protease inhibitor.
Example 3, provided below, illustrates the preparation of a 2-amino-benzoxazole sulfonamide protease inhibitor according to this method.

The compounds of formula (6), (7), (8) and (9) may also be converted to the corresponding N-oxide forms following art-known procedures for converting a trivalent nitrogen into its N-oxide form. Said N-oxidation reaction may generally be carried out by reacting the starting material of formulas (6), (7), (8) and (9) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chloro-benzenecarboperoxoic acid, peroxoalkanoic acids, e.g. peroxoacetic acid, alkylhydroperoxides, e.g. ter-butyl hydroperoxide. Suitable solvents are, for example, water, lower alkanols, e.g. ethanol and the like, hydrocarbons, e.g. toluene, ketones, e.g. 2-butanone, halogenated hydrocarbons, e.g. dichloromethane, and mixtures of such solvents.

In preparations presented above, the reaction products may be isolated from the reaction medium and, if necessary, further purified according to methodologies generally known in the art such as, for example, extraction, crystallization, distillation, trituration and chromatography.

Whenever the term "substituted" is used in the present invention, it is meant to indicate that one or more hydrogens on the atom indicated in the expression using "substituted" is replaced with a selection from the indicated group, provided that the indicated atom's normal valency is not exceeded, and that the substitution results in a chemically stable compound, i.e. a compound that is sufficiently robust to survive isolation to a useful degree of purity from a reaction mixture, and formulation into a therapeutic agent.

As used herein, the term "halo" or "halogen" as a group or part of a group is generic for fluoro, chloro, bromo or iodo.

The term "alkyl", alone or in combination, means straight and branched chained saturated hydrocarbon radicals containing from 1 to 10 carbon atoms, preferably from 1 to 8 carbon atoms, more preferably 1 to 6 carbon atoms. Examples of such radicals include methyl, ethyl, n-propyl, isopropyl, n-butyl, isobutyl, sec-butyl, tert-butyl, 2-methylbutyl, pentyl, iso-amyl, hexyl, 3-methylpentyl, octyl and the like.

The term "C₁₋₄alkyl" as a group or part of a group defines straight and branched chained saturated hydrocarbon radicals having from 1 to 4 carbon atoms, such as, for example, methyl, ethyl, propyl, butyl and 2-methyl-propyl.

The term "C₁₋₆alkyl" as a group or part of a group defines straight and branched chained saturated hydrocarbon radicals having from 1 to 6 carbon atoms such as the groups defined for C₁₋₄alkyl and pentyl, hexyl, 2-methylbutyl, 3-methylpentyl and the like.

The term "C₂₋₆alkenyl" as a group or part of a group defines straight and branched chained hydrocarbon radicals having from 2 to 6 carbon atoms containing at least one double bond such as, for example, ethenyl, propenyl, butenyl, pentenyl, hexenyl and the like.

The term "C₂₋₆alkynyl" as a group or part of a group defines straight and branched chained hydrocarbon radicals having from 2 to 6 carbon atoms containing at least one triple bond such as, for example, ethynyl, propynyl, butynyl, pentynyl, hexynyl and the like.

The term "C₁₋₆alkanediyl" as a group or part of a group defines bivalent straight and branched chained saturated hydrocarbon radicals having from 1 to 6 carbon atoms such as, for example, methylene, ethan-1,2-diyl, propan-1,3-diyl, propan-1,2-diyl, butan-1,4-diyl, pentan-1,5-diyl, hexan-1,6-diyl, 2-methylbutan-1,4-diyl, 3-methyl-pentan-1,5-diyl and the like.

The term "cycloalkyl" alone or in combination, means a saturated or partially saturated monocyclic, bicyclic or polycyclic alkyl radical wherein each cyclic moiety contains from about 3 to about 8 carbon atoms, more preferably from about 3 to about 7 carbon atoms. Examples of monocyclic cycloalkyl radicals include cyclopropyl, cyclobutyl, cyclopentyl, cyclohexyl, cycloheptyl, cyclooctyl and the like. Examples of polycyclic cycloalkyl radicals include decahydronaphthyl, bicyclo [5.4.0] undecyl, adamantyl, and the like.

The term "C₃₋₇cycloalkyl" as a group or part of a group is generic to cyclopropyl, cyclobutyl, cyclopentyl, cyclohexyl or cycloheptyl.

The term "aryl" as a group or part of a group is meant to include phenyl and naphtyl which both may be optionally substituted with one or more substituents independently selected from C₁₋₆alkyl, optionally mono- or disubstituted arninoC₁₋₆alkyl, C₁₋₆alkyloxy, halogen, hydroxy, hydroxyC₁₋₆alkyl, optionally mono- or disubstituted amino, nitro, cyano, haloC₁₋₆alkyl, carboxyl, C₁₋₆alkoxycarbonyl, C₃₋₇cycloalkyl, Het¹, optionally mono- or disubstituted aminocarbonyl, methylthio, methylsulfonyl, and phenyl optionally substituted with one or more substituents, each independently selected from C₁₋₆alkyl, optionally mono- or disubstituted aminoC₁₋₆alkyl, C₁₋₆alkyloxy, halogen, hydroxy, optionally mono- or disubstituted amino, nitro, cyano, haloC₁₋₆alkyl, carboxyl, C₁₋₆alkoxycarbonyl, C₃₋₇cycloalkyl, Het¹, optionally mono- or disubstituted aminocarbonyl, methylthio and methylsulfonyl; whereby the optional substituents on any amino function are independently selected from C₁₋₆alkyl, C₁₋₆alkyloxy-D-, Het¹-D-, Het¹C₁₋₆alkyl, Het¹C₁₋₆alkyl-D-, Het¹oxy-D-, Het¹oxy-C₁₋₄alkyl-D-, phenyl-D-, phenyl-oxy-D-, phenyloxyC₁₋₄alkyl-D-, phenylC₁₋₆alkyl-D-, C₁₋₆alkyloxycarbonylamino-D-, amino-D-, aminoC₁₋₆alkyl and aminoC₁₋₆alkyl-D- whereby each of the amino groups may optionally be mono- or where possible di-substituted with C₁₋₄alkyl and whereby D is defined as C₁₋₆alkanediyl, -C(=O)-, -C(=S)-, -S(=O)₂-, C₁₋₆alkanediyl-C(=O)-, C₁₋₆alkanediyl-C(=S)- or C₁₋₆alkanediyl-S(=O)₂- whereby the point of attachment ofD to the remainder of the molecule is the C₁₋₆alkanediyl group in those moieties containing said group.

The term "haloC₁₋₆alkyl" as a group or part of a group is defined as C₁₋₆alkyl substituted with one or more halogen atoms, preferably, chloro or fluoro atoms, more preferably fluoro atoms. Preferred haloC₁₋₆alkyl groups include for instance trifluoromethyl and difluoromethyl.

The term "hydroxyC₁₋₆alkyl" as a group or part of a group is defined as C₁₋₆alkyl substituted with one or more hydroxy groups.

The term "Het¹" as a group or part of a group is defined as a saturated or partially unsaturated monocyclic, bicyclic or tricyclic heterocycle having 3 to 14 ring members, preferably 5 to 10 ring members and more preferably 5 to 8 ring members, which contains one or more heteroatom ring members, each independently selected from nitrogen, oxygen or sulfur, and which is optionally substituted on one or more nitrogen ring atoms by C₁₋₆alkyl, and optionally substituted on one or more carbon atoms by C₁₋₆alkyl, optionally mono- or disubstituted aminoC₁₋₆alkyl, bydroxyC₁₋₆alkyl, C₁₋₆alkyloxy, halogen, hydroxy, oxo, optionally mono- or disubstituted amino, nitro, cyano, haloC₁₋₆alkyl, carboxyl, C₁₋₆alkoxycarbonyl, C₃₋₇cycloalkyl, optionally mono- or disubstituted aminocarbonyl, methylthio, methylsulfonyl, optionally substituted phenyl; whereby the optional substituents on any amino function are independently selected from C₁₋₆alkyl, C₁₋₆alkyloxy-D-, Het²-D-, Het²C₁₋₆akyl, Het²C₁₋₆alkyl-D-, Het²oxy-D-, Het²oxyC₁₋₄alkyl-D-, aryl-D-, aryloxy-D-, aryloxyC₁₋₄alkyl-D-, arylC₁₋₆alkyl-D-, C₁₋₆alkyloxycarbonylamino-D-, amino-D-, aminoC₁₋₆alkyl and aminoC₁₋₆alkyl-D-whereby each of the amino groups may optionally be mono- or where possible di-substituted with C₁₋₄alkyl and whereby D is as defined above.

The term "Het²" as a group or part of a group is defined as an aromatic monocyclic, bicyclic or tricyclic heterocycle having 5 to 14 ring members, preferably 5 to 10 ring members and more preferably 5 to 6 ring members, which contains one or more heteroatom ring members each independently selected from nitrogen, oxygen or sulfur, and which is optionally substituted on one or more nitrogen ring atoms by C₁₋₆alkyl, and optionally substituted on one or more carbon atoms by C₁₋₆alkyl, optionally mono-or disubstituted aminoC₁₋₆alkyl, hydroxyC₁₋₆alkyl, C₁₋₆alkyloxy, halogen, hydroxy, optionally mono- or disubstituted amino, nitro, cyano, haloC₁₋₆alkyl, carboxyl, C₁₋₆alkoxycarbonyl, C₃₋₇cycloalkyl, optionally mono- or disubstituted aminocarbonyl, methylthio, methylsulfonyl, phenyl; whereby the optional substituents on any amino function are independently selected from C₁₋₆alkyl, C₁₋₆alkyloxy-D-, Het¹-D-, Het¹C₁₋₆alkyl, Het¹C₁₋₆alkyl-D-, Het¹oxy-D-, Het¹oxyC₁₋₄alkyl-D-, aryl-D-, aryloxy-D-, aryloxyC₁₋₄alkyl-D-, arylC₁₋₆alkyl-D-, C₁₋₆alkyloxycarbonylamino-D-, amino-D-, aminoC₁₋₆alkyl and aminoC₁₋₆alkyl-D- whereby each of the amino groups may optionally be mono- or where possible di-substituted with C₁₋₄alkyl and whereby D is as defined above.

The term "alkoxy" or "alkyloxy", alone or in combination, means an alkyl ether radical wherein the term alkyl is as defined above. Examples of suitable alkyl ether radicals include methoxy, ethoxy, n-propoxy, isopropoxy, n-butoxy, iso-butoxy, sec-butoxy, tert-butoxy, hexanoxy and the like.

The term "alkylthio" means an alkyl thioether radical, wherein the term "alkyl" is defined as above. Examples of alkylthio radicals include methylthio (SCH₃), ethylthio (SCH₂CH₃), n-propylthio, isopropylthio, n-butylthio, isobutylthio, sec-butylthio, tert-butylthio, n-hexylthio, and the like.

As used herein the term (=O) forms a carbonyl moiety with the carbon atom to which it is attached. The term (=O) forms a sulfoxide with the sulfur atom to which it is attached. The term (=O)₂ forms a sulfonyl with the sulfur atom to which it is attached.

As used herein the term (=S) forms a thiocarbonyl moiety with the carbon atom to which it is attached.

As used herein before, the term "one or more" covers the possibility of all the available atoms, where appropriate, to be substituted, preferably, one, two or three.

When any variable (e.g. halogen or C₁₋₄alkyl) occurs more than one time in any constituent, each definition is independent.

Whenever used in the present invention the term "compounds of the invention" or "benzoxazole sulfonamide compounds" or a similar term is meant to include the compounds of general formulas (3), (6), (7), (8), and (9) and any subgroup thereof. This term also refers to their *N*-oxides, salts, stereoisomeric forms, racemic mixtures, pro-drugs, esters and metabolites, as well as their quaternized nitrogen analogues. The *N*-oxide forms of said compounds are meant to comprise compounds wherein one or several nitrogen atoms are oxidized to the so-called *N*-oxide.

For therapeutic use, the salts of the compounds according to the invention, are those wherein the counter-ion is pharmaceutically or physiologically acceptable. However, salts having a pharmaceutically unacceptable counterion may also find use, for example, in the preparation or purification of a pharmaceutically acceptable compound of the present invention. All salts, whether pharmaceutically acceptable or not are included within the ambit of the present invention.

The pharmaceutically acceptable salts of the compounds according to the invention, i.e. in the form of water-, oil-soluble, or dispersible products, include the conventional nontoxic salts or the quaternary ammonium salts which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such a sarginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl-bromides and others. Other pharmaceutically acceptable salts include the sulfate salt ethanolate and sulfate salts.

The compounds according to the invention may also exist in their tautomeric forms. Such forms, although not explicitly indicated in the compounds described herein, are intended to be included within the scope of the present invention.

The term stereochemically isomeric forms of compounds of the present invention, as used hereinbefore, defines all possible compounds made up of the same atoms bonded by the same sequence of bonds but having different three-dimensional structures which are not interchangeable, which the compounds of the present invention may possess.

Unless otherwise mentioned or indicated, the chemical designation of a compound encompasses the mixture of all possible stereochemically isomeric forms which said compound may possess. Said mixture may contain all diastereomers and/or enantiomers of the basic molecular structure of said compound. All stereochemically isomeric forms of the compounds of the present invention both in pure form or in admixture with each other are intended to be embraced within the scope of the present invention.

Pure stereoisomeric forms of the compounds and intermediates as mentioned herein are defined as isomers substantially free of other enantiomeric or diastereomeric forms of the same basic molecular structure of said compounds or intermediates. In particular, the term "stereoisomerically pure" concerns compounds or intermediates having a stereoisomeric excess of at least 80% (i.e. minimum 90% of one isomer and maximum 10% of the other possible isomers) up to a stereoisomeric excess of 100% (i. e. 100% of one isomer and none of the other), more in particular, compounds or intermediates having a stereoisomeric excess of 90% up to 100%, even more in particular having a stereoisomeric excess of 94% up to 100% and most in particular having a stereoisomeric excess of 97% up to 100%. The terms "enantiomerically pure" and "diastereomerically pure" should be understood in a similar way, but then having regard to the enantiomeric excess, respectively the diastereomeric excess of the mixture in question.

Pure stereoisomeric forms of the compounds and intermediates of this invention may be obtained by the application of art-known procedures. For instance, enantiomers may be separated from each other by the selective crystallization of their diastereomeric salts with optically active acids or bases. Examples thereof are tartaric acid, dibenzoyltartaric acid, ditoluoyltartaric acid and camphosulfonic acid. Alternatively, enantiomers may be separated by chromatographic techniques using chiral stationary phases. Said pure stereochemically isomeric forms may also be derived from the corresponding pure stereochemically isomeric forms of the appropriate starting materials, provided that the reaction occurs stereospecifically. Preferably, if a specific stereoisomer is desired, said compound will be synthesized by stereospecific methods of preparation. These methods will advantageously employ enantiomerically pure starting materials.

The diastereomeric racemates of the compounds and intermediates of this invention can be obtained separately by conventional methods. Appropriate physical separation methods which may advantageously be employed are, for example, selective crystallization and chromatography, e. g. column chromatography.

It is clear to a person skilled in the art that the compounds and intermediates of this invention contain at least two asymmetric centers and thus may exist as different stereoisomeric forms. These asymmetric centers are indicated with an asterisk (*) in the figures below.

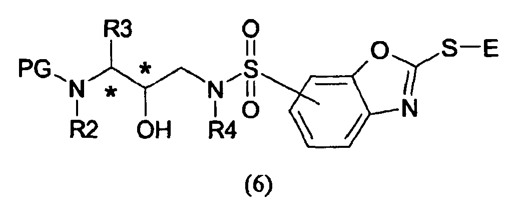

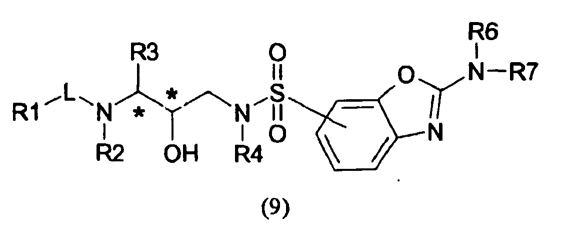

The absolute configuration of each asymmetric center that may be present in the compounds and intermediates of this invention may be indicated by the stereochemical descriptors R and S, this R and S notation corresponding to the rules described in Pure Appl. Chem. 1976, 45,11-30.

The present invention is also intended to include all isotopes of atoms occurring on the present compounds. Isotopes include those atoms having the same atomic number but different mass numbers. By way of general example and without limitation, isotopes of hydrogen include tritium and deuterium. Isotopes of carbon include C-13 and C-14.

The reagents and solvents used throughout the specification may be replaced by functional alternatives or functional derivatives thereof as they are known to a person skilled in the art. Also the reaction conditions such as stirring times, purification and temperature may be adjusted to optimise reaction conditions. Similarly, the reaction products may be isolated from the medium and, if necessary, further purified according to methodologies generally known in the art such as, for example, extraction, crystallization, trituration and chromatography. A number of intermediates and starting materials used in the foregoing preparations are known compounds, while others may be prepared according to methods known in the art of preparing said or similar compounds.

The chemical reactions described are generally disclosed in terms of their broadest application to the preparation of the compounds of this invention. Occasionally, the reactions may not be applicable as described to each compound included within the disclosed scope. The compounds for which this occurs will be readily recognized by those skilled in the art. In all such cases, either the reactions can be successfully performed by conventional modifications known to those skilled in the art, e.g., by appropriate protection of interfering groups, by changing to alternative conventional reagents, by routine modification of reaction conditions, and the like, or other reactions disclosed herein or otherwise conventional, will be applicable to the preparation of the corresponding compounds of this invention. In all preparative methods, all starting materials are known or readily preparable from known starting materials. Similarly, the order of the above mentioned steps in said processes may be different from the order cited above.

The compounds of formula (6) find their particular use in the preparation of a medicament. According to a preferred embodiment, the present compounds of formula (6) are used as precursor in the preparation of anti-viral drugs, in particular anti-HIV drugs, more in particular HIV protease inhibitors.

The compounds of formula (6) and all intermediates leading to the formation of stereoisomerically pure compounds are of particular interest in preparing 2-amino-benzoxazole sulfonamide compounds, as HIV protease inhibitors, as disclosed in WO 95/06030, WO 96/22287, WO 96/28418, WO 96/28463, WO 96/28464, WO 96/28465 WO 97/18205, and WO 02/092595 all incorporated herein by reference, and in particular, the following HIV-protease inhibitors of formula (9):
(3-[(2-Amino-benzoxazole-6-sulfonyl)-pyridin-2-ylmethyl-amino]-1-benzyl-2-hydroxy-propyl)-carbamie acid tetrahydro-furan-3-yl ester;
(3-[(2-Acetylamino-benzoxazole-6-sulfonyl)-isobutyl-amino]-1-benzyl-2-hydroxypropyl)-carbamic acid thiazol-5-ylmethyl ester;
(6-[[2-Hydroxy-4-phenyl-3-(thiazol-5-ylmethoxycarbonylamino)-butyl]-isobutylsulfamoyl]-benzoxazol-2-yl)-carbamic acid ethyl ester;
[1-Benzyl-2-hydroxy-3-((2-[(6-hydroxy-pyridine-3-carbonyl)-amino]-benzoxazole-6-sulfonyl)-isobutyl-amino)-propyl]-carbamic acid thiazol-5-ylmethyl ester;
[1-Benzyl-2-hydroxy-3-(isobutyl-(2-[(pyridine-3-carbonyl)-amino]-benzoxazole-6-sulfonyl)-amino)-propyl]-carbamic acid thiazol-5-ylmethyl ester;
(1-Benzyl-2-hydroxy-3-[isobutyl-(2-pyrrolidin-1-yl-benzoxazole-6-sulfonyl)-amino]-propyl)-carbamic acid thiazol-5-ylmethyl ester;
1-Benzyl-2-hydroxy-3-(isobutyl-(2-[methyl-(2-pyrrolidin-1-yl-ethyl)-amino]-benzoxazole-6-sulfonyl)-amino)-propyl]-carbamic acid thiazol-5-ylmethyl ester;
[1-Benzyl-2-hydroxy-3-(isobutyl-{2-[2-(4-methyl-piperazin-1-yl)-acetylamino]-benzooxazole-6-sulfonyl}-amino)-propyl]-carbamic acid thiazol-5-ylmethyl ester;
1-Benzy1-2-hydroxy-3-(isobutyl-(2-[methyl-(5-oxo-pyrrolidine-2-carbonyl)-amino]-benzoxazole-6-sulfonyl)-amino)-propyl]-carbamic acid thiazol-5-ylmethyl ester;
1-Benzyl-2-hydroxy-3-(isobutyl-(2-[methyl-(pyridine-4-carbonyl)-amino]-benzoxazole-6-sulfonyl)-amino)-propyl]-carbamic acid thiazol-5-ylmethyl ester;
1-Benzyl-2-hydroxy-3-(isobutyl-(2-[methyl-(pyridine-3-carbonyl)-amino]-benzoxazole-6-sulfonyl)-amino)-propyl]-carbamic acid thiazol-5-ylmethyl ester;
1-Benzyl-3-((2-[(furan-3-carbonyl)-methyl-amino]-benzoxazole-6-sulfonyl)-isobutylamino)-2-hydroxy-propyl]-carbamic acid thiazol-5-ylmethyl ester;
[1-Benzyl-2-hydroxy-3-(isobutyl-(2-[(1-methyl-pyrrolidine-2-carbonyl)-amino]-benzooxazole-6-sulfonyl)-amino)-propyl]-carbamic acid thiazol-5-ylmethyl ester;
(3-[(2-Amino-benzoxazole-6-sulfonyl)-isobutyl-amino]-1-benzyl-2-hydroxy-propyl)-carbamic acid tetrahydro-furan-3-yl ester;
(3-[(2-Amino-benzoxazole-6-sulfonyl)-isobutyl-amino]-1-benzyl-2-hydroxy-propyl)-carbamic acid hexahydro-furo[2,3-b]furan-3-yl ester;
(3-[(2-Arnino-benzoxazole-6-sulfonyl)-isobutyl-amino]-1-benryl-2-hydroxy-propyl)-carbamic acid thiazol-5-ylmethyl ester;
(1S,2R)-3-[(2-Amino-benzoxazole-6-sulfonyl)-isobutyl-amino]-1-benzyl-2-hydroxypropyl)-carbamic acid thiazol-5-ylmethyl ester;
(3-[(2-Amino-benzoxazole-6-sulfonyl)-isobutyl-amino]-1-benzyl-2-hydroxy-propyl)-carbamic acid pyridin-3-ylmethyl ester;
N-(3-[(2-Amino-benzoxazole-6-sulfonyl)-isobutyl-amino]-1-benzyl-2-hydroxypropyl)-2-(2,6-dimethyl-phenoxy)-acetamide;
3-Amino-N-(3-[(2-amino-benzoxazole-6-sulfonyl)-isobutyl-amino]-1-benzyl-2-hydroxy-propyl)-2-methyl-benzamide;
(3-[(2-Acetylamino-benzoxazole-6-sulfonyl)-isobutyl-amino]-1-benzyl-2-hydroxypropyl)-carbamic acid hexahydro-furo[2,3-b]furan-3-yl ester;
(3-[(2-Amino-benzoxazole-6-sulfonyl)-isobutyl-amino]-1-benzyl-2-hydroxy-propyl)-carbamic acid hexahydro-furo[2,3-b]furan-3-yl ester;
(1-Benzyl-3-([2-(2-dimethylamino-ethylamino)-benzoxazole-6-sulfonyl]-isobutylamino)-2-hydroxy-propyl)-carbamic acid hexahydro-furo[2,3-b]furan-3-yl ester;
(6-([3-(Hexahydro-furo[2,3-b]furan-3-yloxycarbonylamino)-2-hydroxy-4-phenylbutyl]-isobutyl-sulfamoyl)-benzoxazol-2-yl)-carbamic acid ethyl ester;
(1-Benzyl-2-hydroxy-3-[isobutyl-(2-methylamino-benzoxazole-6-sulfonyl)-amino]-propyl)-carbamic acid hexahydro-furo[2,3-b]furan-3-yl ester;
(1S,2R)-1-Benzyl-2-hydroxy-3-[isobutyl-(2-methylamino-benzoxazole-6-sulfonyl)-amino]-propyl}-carbamic acid (3R,3aS,6aR)-hexahydro-furo[2,3-b]furan-3-yl ester;
(1-Benzyl-2-hydroxy-3-(isobutyl-[2-(2-pyrrolidin-1-yl-ethylamino)-benzoxazole-6-sulfonyl]-amino)-propyl)-carbamic acid hexahydro-furo[2,3-b]furan-3-yl ester;
(1-Benzyl-2-hydroxy-3-[isobutyl-(2-methylamino-benzoxazole-6-sulfonyl)-amino]-propyl)-carbamic acid tetrahydro-furan-3-yl ester;
(1-Benzyl-2-hydroxy-3-[isobutyl-(2-methylamino-benzoxazole-6-sulfonyl)-amino]-propyl)-carbamic acid thiazol-5-ylmethyl ester;
N-(1-Benzyl-2-hydroxy-3-[isobutyl-(2-methylamino-benzoxazole-6-sulfonyl)-amino]-propyl)-3-hydroxy-2-methyl-benzamide;
3-Amino-N-(1-benzyl-2-hydroxy-3-[isobutyl-(2-methylamino-benzoxazole-6-sulfonyl)-amino]-propyl)-2-methyl-benzamide;
N-(1-Benzyl-2-hydroxy-3-[isobutyl-(2-methylamino-benzoxazole-6-sulfonyl)-amino]-propyl)-2-(2,6-dimethyl-phenoxy)-acetamide;
[6-((3-[2-(2,6-Dimethyl-phenoxy)-acetylamino]-2-hydroxy-4-phenyl-butyl)-isobutylsulfamoyl)-benzoxazol-2-yl]-carbamic acid ethyl ester;
N-(1-Benzyl-2-hydroxy-3-[isobutyl-(2-methylamino-benzoxazole-6-sulfonyl)-amino]-propyl)-2-(3,5-dichloro-pyridin-4-yloxy)-acetamide;
(1-Benzyl-2-hydroxy-3-[isobutyl-(2-methylamino-benzoxazole-6-sulfonyl)-amino]-propyl)-carbamic acid hexahydro-1,7-dioxa-4-aza-inden-3-yl ester;
5-Methyl-isoxazole-4-carboxylic acid (1-benzyl-2-hydroxy-3-[isobutyl-(2-methylamino-benzoxazole-6-sulfonyl)-amino]-propyl)-amide;
(1-Benzyl-2-hydroxy-3-[[2-(2-hydroxy-ethylamino)-benzooxazole-6-sulfonyl]-isobutyl-amino]-propyl)-carbamic acid hexahydro-furo[2,3-b]furan-3-yl ester;
N-(1-Benzyl-2-hydroxy-3-[isobutyl-(2-methylamino-benzoxazole-6-sulfonyl)-amino]-propyl)-2-(2,6-dimethyl-4-nitro-phenoxy)-acetamide;
2-(4-Amino-2,6-dimethyl-phenoxy)-N-(1-benzyl-2-hydroxy-3-[isobutyl-(2-methylamino-benzoxazole-6-sulfonyl)-amino]-propyl)-acetamide;
(3-[(2-Amino-benzoxazole-6-sulfonyl)-isobutyl-amino]-1-benzyl-2-hydroxy-propyl)-carbamic acid hexahydro-1,7-dioxa-4-aza-inden-3-yl ester;
N-(3-[(2-Amino-benzoxazole-6-sulfonyl)-isobutyl-amino]-1-benzyl-2-hydroxypropyl)-4-bromo-2-methyl-benzamide;
N-(3-[(2-Anvno-benzoxazole-6-sulfonyl)-isobutyl-amino]-1-benzyl-2-hydroxypropyl)-2-(4-cyano-2,6-dimethyl-phenoxy)-acetamide;
N-(3-[(2-Anvno-benzoxazole-6-sulfonyl)-isobutyl-amino]-1-benzyl-2-hydroxypropyl)-2-(2-amino-4,6-dimethyl-pyrimidin-5-yloxy)-acetamide;
(3-[(2-Amino-benzoxazole-6-sulfonyl)-pyridin-2-ylmethyl-amino]-1-benzyl-2-hydroxy-propyl)-carbamic acid hexahydro-furo[2,3-b]furan-3-yl ester;
N-(3-[(2-Amino-benzoxazole-6-sulfonyl)-isobutyl-ainino]-1-benzyl-2-hydroxypropyl)-2-(4,6-dimethyl-pyrimidin-5-yloxy)-acetamide;
N-(3-[(2-Amino-benzoxazole-6-sulfonyl)-isobutyl-amino]-1-benzyl-2-hydroxypropyl)-2-(2-amino-thiazol-4-yl)-acetamide;
(3-[(2-Amino-benzoxazole-6-sulfonyl)-pyridin-2-ylmethyl-amino]-1-benzyl-2-hydroxy-propyl)-carbamic acid pyridin-3-ylmethyl ester;
(3-[(2-Amino-benzoxazole-6-sulfonyl)-pyridin-2-ylmethyl-amino]-1-benzyl-2-hydroxy-propyl)-carbamic acid thiazol-5-ylmethyl ester;
N-(3-[(2-Amino-benzoxazole-6-sulfonyl)-pyridin-2-ylmethyl-amino]-1-benzyl-2-hydroxy-propyl)-2-(2,6-dimethyl-phenoxy)-acetamide;
N-(3-[(2-Amino-benzoxazole-6-sulfonyl)-pyridin-2-ylmethyl-amino]-1-benzyl-2-hydroxy-propyl)-3-hydroxy-2-methyl-benzamide;
(3-[(2-Amino-benzoxazole-6-sulfonyl)-isobutyl-amino]-1-benzyl-2-hydroxy-propyl)-carbamic acid 2,3-dihydro-[1,4]dioxino[2,3-b]pyridin-3-ylmethyl ester;
(3-[(2-Amino-benzoxazole-6-sulfonyl)-isobutyl-amino]-1-benzyl-2-hydroxy-propyl)-carbamic acid 2,3-dihydro-[1,4]dioxino[2,3-b]pyridin-2-ylmethyl ester;
N-(3-[(2-Amino-benzoxazole-6-sulfonyl)-isobutyl-amino]-1-benzyl-2-hydroxypropyl)-2-(3,4-diamino-2,6-dimethyl-phenoxy)-acetamide;
N-(3-[(2-Amino-benzoxazole-6-sulfonyl)-isobutyl-amino]-1-benzyl-2-hydroxypropyl)-2-(4,6-dimethyl-1H-benzoimidazol-5-yloxy)-acetamide;
N-(3-[(2-Amino-benzoxazole-6-sulfonyl)-isobutyl-amino]-1-benzyl-2-hydroxypropyl)-2-(2,6-dimethyl-phenoxy)-4-hydroxy-butyramide;
6-Methyl-imidazo[2,1-b]thiazole-5-carboxylic acid (3-[(2-amino-benzoxazole-6-sulfonyl)-isobutyl-amino]-1-benzyl-2-hydroxy-propyl)-amide;
N-(1-Benzyl-2-hydroxy-3-[isobutyl-(2-methylamino-benzoxazole-6-sulfonyl)-amino]-propyl)-4-bromo-2-methyl-benzamide;
(3-[(2-Amino-benzoxazole-6-sulfonyl)-isobutyl-amino]-1-benzyl-2-hydroxy-propyl)-carbamic acid 3H-imidazol-4-ylmethyl ester;
(3-[(2-Amino-benzoxazole-6-sulfonyl)-isobutyl-amino]-1-benzyl-2-hydroxy-propyl)-carbamic acid 2-hydroxymethyl-thiazol-4-ylmethyl ester;
N-(3-[(2-Amino-benzoxazole-6-sulfonyl)-isobutyl-amino]-1-benzyl-2-hydroxypropyl)-2-(4-aminomethyl-2,6-dimethyl-phenoxy)-acetamide;
[1-Benzyl-2-hydroxy-3-(isobutyl-(2-[methyl-(3-pyrrolidin-1-yl-propyl)-amino]-benzoxazole-6-sulfonyl)-amino)-propyl]-carbamic acid hexahydro-furo[2,3-b]furan-3-yl ester;
{3-[(2-Amino-benzooxazole-6-sulfonyl)-isobutyl-amino]-1-benzyl-2-hydroxy-propyl}-carbamic acid 2-(3,5-dimethyl-pyridin-4-yloxy)-ethyl ester
[1-Benzyl-2-hydroxy-3-(isobutyl-(2-[methyl-(2-pyrrolidin-1-yl-ethyl)-amino]-benzoxazole-6-sulfonyl)-amino)-propyl]-carbamic acid hexahydro-furo[2,3-b]furan-3-yl ester;
(1-Benzyl-2-hydroxy-3-[isobutyl-(2-methylamino-benzoxazole-6-sulfonyl)-amino]-propyl)-carbamic acid hexahydro-1,7-dioxa-4-aza-inden-3-yl ester;
(1-Benzyl-2-hydroxy-3-[isobutyl-(2-methylamino-benzoxazole-6-sulfonyl)-amino]-propyl)-carbamic acid hexahydro-1,7-dioxa-4-aza-inden-3-yl ester;
N-(3-[(2-Amino-benzoxazole-6-sulfonyl)-isobutyl-amino]-1-benzyl-2-hydroxypropyl)-2-(3,5-dimethyl-pyridin-4-yloxy)-acetamide;
(3-[(2-Amino-benzoxazole-6-sulfonyl)-isobutyl-amino]-1-benzyl-2-hydroxy-propyl)-carbamic acid 3-oxo-tetrahydro-pyrrolo[1,2-c]oxazol-7-yl ester;
(1-Benzyl-2-hydroxy-3-(isobutyl-[2-(4-methyl-piperazin-1-yl)-benzoxazole-6-sulfonyl]-amino)-propyl)-carbamic acid thiazol-5-ylmethyl ester;
(1-Benzyl-3-[(2-dimethylamino-benzoxazole-6-sulfonyl)-isobutyl-amino]-2-hydroxypropyl)-carbamic acid hexabydro-furo[2,3-b]furan-3-yl ester;
(6-([2-Hydroxy-4-phenyl-3-(thiazol-5-ylinethoxycarbonylamino)-butyl]-isobutylsulfamoyl)-benzoxazol-2-yl)-carbamic acid methyl ester;
[1-Benzyl-2-hydroxy-3-(isobutyl-(2-[(5-oxo-pyrrolidine-2-carbonyl)-amino]-benzoxazole-6-sulfonyl)-amino)-propyl]-carbamic acid thiazol-5-ylmethyl ester;
[1-Benzyl-3-((2-[(furan-3-carbonyl)-amino]-benzoxazole-6-sulfonyl)-isobutyl-amino)-2-hydroxy-propyl]-carbamic acid thiazol-5-ylmethyl ester;
[1-Benzyl-2-hydroxy-3-(isobutyl-(2-[(1-methyl-piperidine-4-carbonyl)-amino]-benzoxazole-6-sulfonyl)-amino)-propyl]-carbamic acid thiazol-5-ylmethyl ester;
[1-Benzyl-2-hydroxy-3-(isobutyl-(2-[(pyridine-2-carbonyl)-amino]-benzoxazole-6-sulfonyl)-amino)-propyl]-carbamic acid thiazol-5-ylmethyl ester;
(3-[(2-Amino-benzoxazole-6-sulfonyl)-isobutyl-amino]-1-benzyl-2-hydroxy-propyl)-carbamic acid 2-chloro-thiazol-5-ylmethyl ester;
(1-Benzyl-3-([2-(2-dimethylamino-acetylamino)-benzoxazole-6-sulfonyl]-isobutylamino)-2-hydroxy-propyl)-carbamic acid thiazol-5-ylmethyl ester;
(1-Benzyl-2-hydroxy-3-[isobutyl-(2-piperazin-1-yl-benzoxazole-6-sulfonyl)-amino]-propyl)-carbamic acid thiazol-5-ylmethyl ester;
(1-Benzyl-2-hydroxy-3-[isobutyl-(2-piperidin-1-yl-benzoxazole-6-sulfonyl)-amino]-propyl)-carbamic acid thiazol-5-ylmethyl ester;
(1-Benzyl-2-hydroxy-3-[isobutyl-(2-(2-[methyl-(2-pyrrolidin-1-yl-ethyl)-amino]-acetylamino)-benzoxazole-6-sulfonyl)-amino]-propyl)-carbamic acid thiazol- 5-ylmethyl ester;
(1-Benzyl-3-[(2-dimethylamino-benzoxazole-6-sulfonyl)-isobutyl-amino]-2-hydroxypropyl)-carbamic acid thiazol-5-ylmethyl ester;
(3-[(2-Amino-benzoxazole-6-sulfonyl)-isobutyl-amino]-1-benzyl-2-hydroxy-propyl)-carbamic acid oxazol-5-ylmethyl ester;
[1-Benzyl-2-hydroxy-3-(isobutyl-(2-[(pyridine-4-carbonyl)-amino]-benzoxazole-6-sulfonyl)-amino)-propyl]-carbamic acid thiazol-5-ylmethyl ester;
[1-Benzyl-2-hydroxy-3-(isobutyl-(2-[methyl-(pyridine-2-carbonyl)-amino]-benzoxazole-6-sulfonyl)-amino)-propyl]-carbamic acid thiazol-5-ylmethyl ester;
[1-Benzyl-2-bydroxy-3-(isobutyl-{2-[methyl-(1-methyl-piperidine-3-carbonyl)-amino]-benzooxazole-6-sulfonyl}-amino)-propyl]-carbamic acid thiazol-5-ylmethyl ester;
[1-Benzyl-2-hydroxy-3-(isobutyl-{2-[methyl-(1-methyl-piperidine-4-carbonyl)-amino]-benzooxazole-6-sulfonyl}-amino)-propyl]-carbamic acid thiazol-5-ylmethyl ester;
[1-Benzyl-3-((2-[(2-chloro-pyridine-4-carbonyl)-methyl-amino]-benzoxazole-6-sulfonyl)-isobutyl-amino)-2-hydroxy-propyl]-carbamic acid thiazol-5-ylmethyl ester;
[1-Benzyl-2-hydroxy-3-(isobutyl-{2-[methyl-(1-methyl-pyrrolidine-2-carbonyl)-amino]-benxooxazole-6-sulfonyl}-amino)-propyl]-carbamic acid thiazol-5-ylmethyl ester;
N-(3-[(2-Amino-benzoxazole-6-sulfonyl)-isobutyl-amino]-1-benzyl-2-hydroxypropyl)-3-hydroxy-2-methyl-4-nitro-benzamide;
4-Amino-N-(3-[(2-amino-benzoxazole-6-sulfonyl)-isobutyl-amino]-1-benzyl-2-hydroxy-propyl)-3-hydroxy-2-methyl-benzamide;
7-Methyl-benzoxazole-6-carboxylic acid (3-[(2-amino-benzoxazole-6-sulfonyl)-isobutyl-amino]-1-benzyl-2-hydroxy-propyl)-amide;
4-Methyl-benzo[1,3]dioxole-5-carboxylic acid (3-[(2-amino-benzoxazole-6-sulfonyl)-isobutyl-amino]-1-benzyl-2-hydroxy-propyl)-amide;
(3-[(2-Amino-benzoxazole-6-sulfonyl)-isobutyl-amino]-1-benzyl-2-hydroxy-propyl)-carbamic acid thiazol-5-ylmethyl ester;
or any stereoisomeric forms and pharmaceutically acceptable addition salts thereof.

Thus, the present invention also relates to HIV protease inhibitors of formula (9) or any pharmaceutically acceptable salt or prodrug thereof, obtained by using a compound of formula (6) as intermediate, wherein both compound of formula (6) and HIV protease inhibitors of formula (9) are prepared as described in the present invention.

The following examples are meant to illustrate the present invention. These examples are presented to exemplify the invention and are not to be considered as limiting the scope of the invention.

### Examples

Example I illustrates the preparation of a benzoxazole sulfonamide compound according to the invention corresponding to formula (6) by reacting a sulfonylchloride with an intermediate corresponding to formula (5). Example 2 and 3 illustrate the preparation of 2-amino-benzoxazole sulfonamide protease inhibitors using a benzoxazole sulfonamide compound according to the invention.

### Example 1: Preparation of a benzoxazole sulfonamide compound

A benzoxazole sulfonamide represented by compound c-6 in the below provided Scheme C, can be prepared as follows.

The intermediate c-2 was prepared by adding 2-mercaptobenzoxazole (c-1 which is equal to compound of formula (1) above) (1200 g; 7.94 mol) to 8500 ml ethylacetate in a 20 L flask. Then 1420 g (10.29 mol) potassium carbonate was added at rt. iodomethane (1243 g; 8.76 mol) was added dropwise to this reaction mixture maintaining the internal temperature below 40°C. This mixture was stirred for 24 hours while the internal temperature decreased to 20°C. The reaction mixture was then treated with 4000 ml water and 138 g NH₄OH at rt for about 20 minutes. The organic layer was separated and filtered. The aqueous phase was extracted with 1200 ml ethylacetate. The organic layers were collected and washed with 1500 ml water. The organic phase was evaporated under reduced pressure until a final volume of about 2000ml. Magnesium sulphate was added and the mixture was filtered. The filtrate was evaporated under reduced pressure yielding 1288 g of the intermediate c-2 (98 % yield / HPLC purity 99.6 %).

In order to prepare intermediate c-3, chlorosulfonic acid (3890 g; 33.3 mol) was stirred under nitrogen. Then intermediate c-2 (1288 g; 7.80 mol) was added portionwise maintaining the internal temperature below 60°C by external cooling. After complete addition of intermediate b-2 the reaction mixture was stirred overnight at 85°C. The heating was removed and the reaction mixture was cooled down until 65°C. SOCl₂ was added dropwise while maintaining a controlled release of gases by good stirring. This mixture was stirred overnight at 65°C. This reaction mixture was added to a well stirred mixture of EtOAc (6.9 kg) and ice (9.2kg) while maintained the temperature below 0°C. The organic layer was isolated. The aqueous phase was extracted with EtOAc (3.1 kg). The combined organic layer was washed with 7.5 % NaHCO₃ (210 g/2.8 L water). Because the pH of this water layer was still 1, therefore another 125 g NaHCO₃ was added. This mixture was stirred for 1 hour, then the phases were separated. The organic layer was dried with Na₂CO₃ (2.5 kg). After filtration 1935 g of intermediate c-3 was obtained (yield 94%, HPLC purity 94%) and used in the preparation of compound c-6.

Intermediate c-5 was prepared by reacting in a 20 L flask 1595 g of intermediate c-4 under inert conditions with 2400 g isopropanol. Then 6198 g isobutylamine was added at room temperature. The reaction mixture was heated and stirred overnight at an internal temperature of 65 °C. The excess of isobutylamine was removed as far as possible by distillation at 85°C. Then 3 L hexane was added and the solvents were removed by an azeotropic distillation at 90 °C. The azeotropic distillation with hexane was repeated 3 times. The remaining product in the 20 L flask was crystallised during cooling overnight upon rt. The solid white crude was solved by adding 3 L EtOAc and heated to 65°C. After complete dissolution of the white crude, 1.5 L EtOAc was distilled. The remained solution comprising intermediate c-5 was stirred at an internal temperature of 65°C and was used *in situ* for the synthesis of compound c-6.

For preparing compound c-6 the solution of intermediate c-5 was stirred at >65°C and triethylamine (1400 g) was added. Then the reaction mixture was cooled to 50°C and the EtOAc solution of intermediate c-3 was added in 3 hours while maintaining the reaction-temperature at 40°C - 50°C by cooling with water. TLC showed no starting material after 30 min. but the reaction was stirred overnight while the internal temperature decreased to 20°. The mixture was heated to 45°C and washed with 5 L water, with 4.2 L water plus 800 g 30% HCl and with 4.5 L water plus 250 g NaHCO₃. The organic layer was separated and crystallised by stirring overnight while the temperature decreased to 20°C. After further cooling to 0°C - 5°C, the solid was filtered and dried in the vacuumoven at 40°C, yielding 2585 g of compound c-1 (76 % yield, HPLC purity 98.2%).

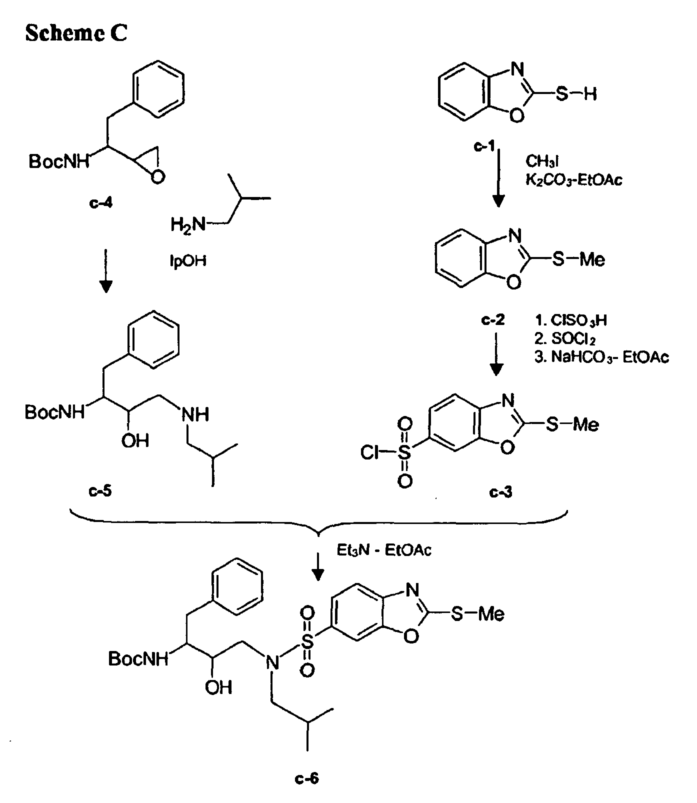

### Example 2: Preparation of a 2-amino-benzoxazole sulfonamide (compound d-5)

This example illustrates the preparation of a 2-amino benzoxazole sulfonamide protease inhibitor, represented as d-5 in the below provided Scheme D.

For preparing this protease inhibitor intermediate d-1 was charged into a 10 L sealed reactor and heated until 105°C. The pressure rose up to 2.2 bar. Then NH₃-gas (319 gram; 18.7 mol NH₃-gas) was added until a pressure of 7.5 - 8 bar was achieved. This reaction mixture was stirred for 15 hours at an internal temperature of 105°C - 110°C. Then the reaction mixture was cooled to an internal temperature of 35°C and the pressure was released carefully. The reaction mixture was collected in a 10 L drum. This procedure was repeated 3 times to end up with 3 different batches (batch 1,2 and 3). The purity on HPLC was about 75% for the 3 different batches. The 3 different batches were collected, pooled and evaporated on a Büchi apparatus to end up with a final volume of about 6 L iso-propanol. This residue was stirred and heated at 75 °C. Water (4.5 L) was added and the slurry was stirred for 30 min at 75 °C. Then the heating was removed and the mixture was stirred overnight at rt. The reaction mixture was filtered and washed with 400 ml iso-propanol. The product was dried for two days in the vacuum oven at 50 °C to yield 1514 gram (80% yield; HPLC purity 97.04%) of intermediate d-2.

Subsequently, 1514 gram of intermediate d-2, was stirred in 32 L ethylacetate and heated until 60°C. 2100 ml HCl/isopropanol 5N was added slowly and a white precipitate was formed and CO₂ gas was released. After adding all of the HCl/isopropanol 5 N the reaction mixture is stirred for 3 - 4 hours at an internal temperature of 55°C - 60 °C. Then the precipitate was filtered and washed with ethylacetate 400 ml. The wet precipitate was evaporated on a Büchi apparatus and then dried overnight in the vacuum oven at 50°C to yield 1265 gram 1 (83% yield; HPLC purity 98.58 %) of intermediate d-3.

Then, intermediate d-3 was further reacted with intermediate d-4, in the presence of triethylamine and dichloromethane in order to obtain d-5, which was further purified by ethanol extraction to yield d-5 (>85 % yield / HPLC purity 97 %).

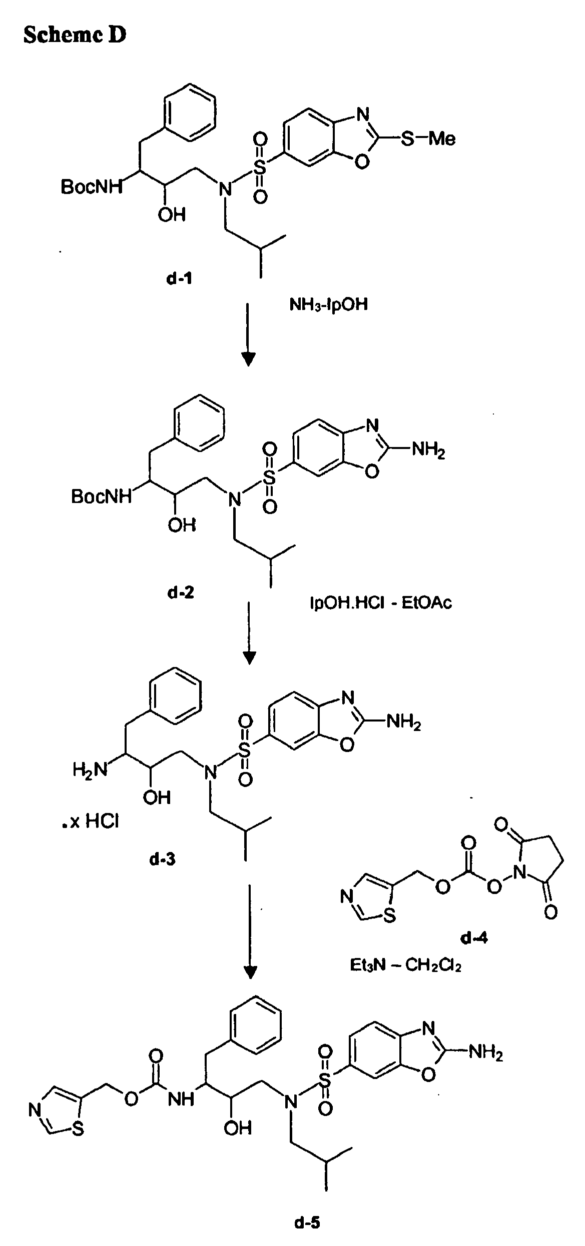

### Example 3: Preparation of a 2-amino-benzoxazole sulfonamide (compound e-5)

This example illustrates the preparation of a 2-amino benzoxazole sulfonamide protease inhibitor, represented as e-5 in the below provided Scheme E.

A suspension of intermediate e-1 (1000 g; 1.77 mol) in 6000 ml isopropanol was heated until complete dissolution (Ti 75°C). Over a period of 5 min. methylamine (4800 g, 40%wt in H₂O; 62 mol) was added (Ti after addition 65°C). The resulting solution was stirred and heated (Ti 65°C) for 1 hour. The 20 L reactor flask was opened and heated while stirring rapidly until most of excess methylamine was removed (Ti >70°C). At 70°C, 7500 ml hot water was added while maintaining the internal temperature > 70°C. Then the heating was removed and the reaction mixture was cooled to 30°C overnight. At Ti 66°C a bulky precipitate of intermediate e-2 was formed. At 30°C the precipitate was filtered. The precipitate was washed with 2000 ml isopropanol/water (1/1) and dried, yielding about 1000 g of intermediate e-2 (90 - 100% yield; HPLC purity; 96.9%).

Subsequently, a suspension of 16.6kg wet intermediate e-2 was charged into a 150 L reactor. Then 150 kg EtOAc was added and the suspension was stirred while heating to 78°C. The water in the reaction mixture was removed by an azeotropic distillation. The distillation was stopped when KF of the reaction mixture showed less then 0.15w/w% water. The reactor contained about 4800 g of intermediate e-2 TIC 1662 after the azeotropic distillation (± 95% yield). The remained clear solution (± 4800g intermediate e-2 in 80L EtOAc) was stirred at 65°C and 6.7 L HCl/iso-propanol (5 to 6 N solution in isopropanol) was added over 0.5 hour. The resulting mixture was further stirred at an internal temperature of 65°C. Another 1 L HCl/iso-propanol (5 to 6 N solution in isopropanol) was added at 65°C. This reaction mixture was further stirred overnight while the heating was removed. The reaction mixture was cooled to 15°C then filtered and washed with 5.2 kg EtOAc, yielding 8.5 kg intermediate e-3 wet, which was dried at rt under a nitrogen flow, providing 3.376 kg intermediate e-3 (74% yield; HPLC purity; 98.1%).

Then, intermediate e-3 was further reacted with intermediate e-4, in the presence of triethylamine and EtOAc in order to obtain e-5 (yield 75%, purity 98.8%).

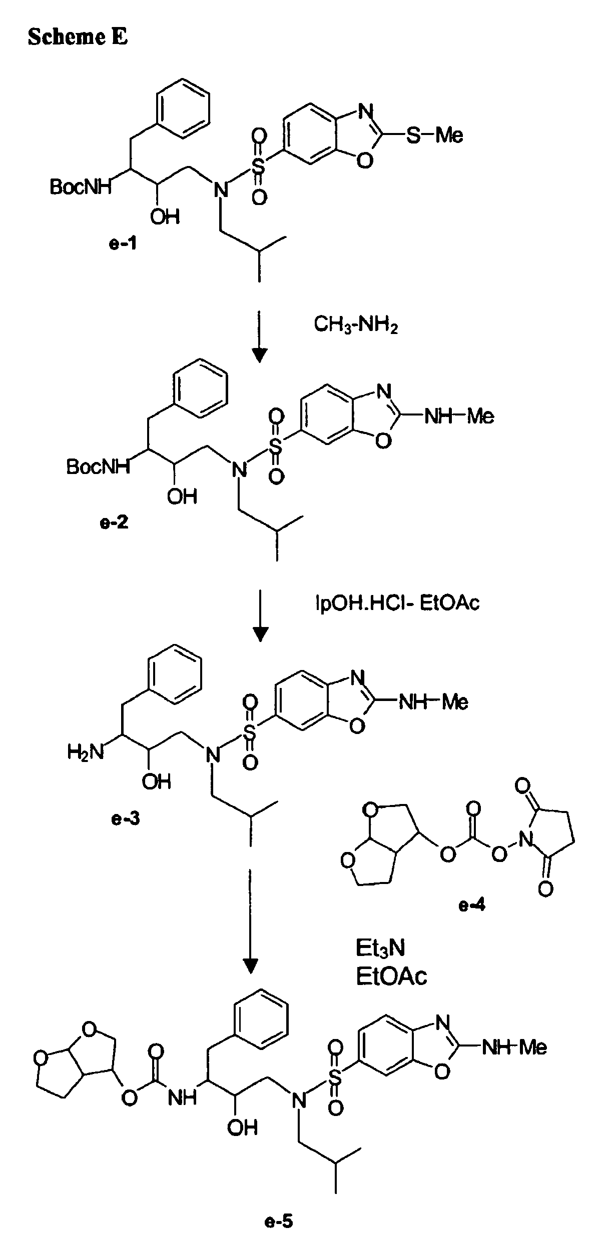

## Claims

1. A method for preparing a compound of formula (6), 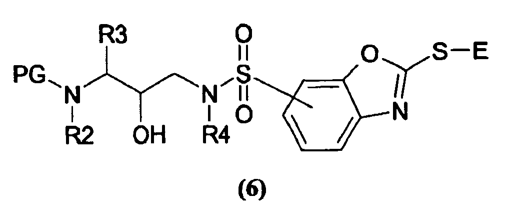 and salts, stereoisomeric forms, and racemic mixtures thereof, **characterized in that** said method starts from a compound of formula (2), 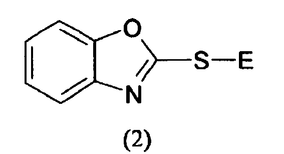 wherein
**E** is an electrophilic moiety;
transforming compound of formula (2) into a compound of formula (3), 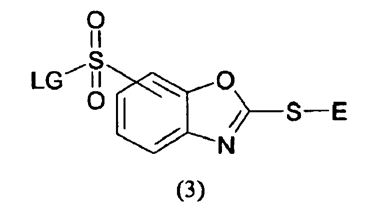 wherein
**LG** is a leaving group; and
reacting compound of formula (3) with a compound of formula (5), 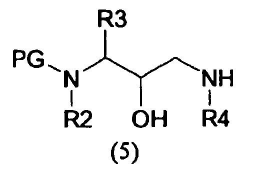 wherein
**PG** is a protecting group;
**R₂** is hydrogen or C₁₋₆alkyl;
**R₃** is C₃₋₇cycloalkyl, aryl, Het¹, Het², or C₁₋₆alkyl optionally substituted with C₃₋₇cycloalkyl, aryl, Het¹, or Het²; wherein each C₃₋₇cycloalkyl, aryl, Het¹, and Het² may be optionally substituted with one or more groups selected from oxo, C₁₋₆alkyloxy, C₁₋₆alkyl, C₁₋₆alkylsulfonyl, aminosulfonyl, amino, C₁₋₆alkylcarbonylamino, hydroxyC₁₋₆alkyl, cyano, C₁₋₆alkyloxycarbonyl, aminocarbonyl, halogen or trifluoromethyl, wherein each amino maybe mono- or disubstitued with C₁₋₆alkyl;
**R₄** is selected from the group comprising hydrogen, C₁₋₄alkyloxycarbonyl, carboxyl, aminocarbonyl, mono- or di(C₁₋₄alkyl)aminocarbonyl, C₃₋₇cycloalkyl, C₂₋₆alkenyl, C₂₋₆alkynyl, or C₁₋₆alkyl optionally substituted with one or more substituents each independently selected from aryl, Het¹, Het², C₃₋₇cycloalkyl, C₁₋₄alkyloxycarbonyl, carboxyl, aminocarbonyl, mono- or di(C₁₋₄alkyl)aminocarbonyl, aminosulfonyl, C₁₋₄alkyl-S(=O)ₜ, hydroxy, cyano, halogen and amino optionally mono-or disubstituted where the substituents are each independently selected from C₁₋₄alkyl, aryl, arylC₁₋₄alkyl, C₃₋₇cycloalkyl, C₃₋₇cycloalkylC₁₋₄alkyl, Het¹, Het², Het¹C₁₋₄alkyl and Het²C₁₋₄alkyl; and
**t** is zero, one or two.

2. A method according to claim 1 for preparing a compound of formula (6), **characterized in that** said method comprises the steps of:
alkylating a compound of formula (1) 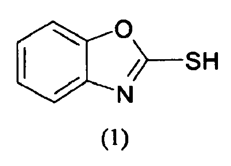
resulting into a compound of formula (2); 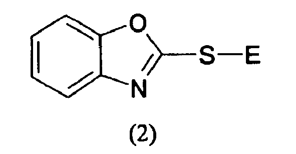 wherein
**E** is a C₁₋₆alkyl;
reacting compound of formula (2) with a sulfonation agent, resulting in a compound of formula (3); 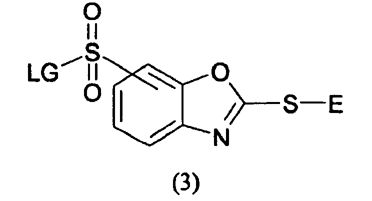 wherein
**LG** is a leaving group; and
coupling compound of formula (3) with a compound of formula (5). 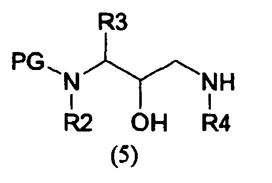 wherein
**PG** is a protecting group; and
wherein **R₂, R₃,** and **R₄** are as claimed in claim 1.

3. A method according to any one of claims 1 to 2, **characterized in that** compound of formula (3) is a compound of formula (3''').

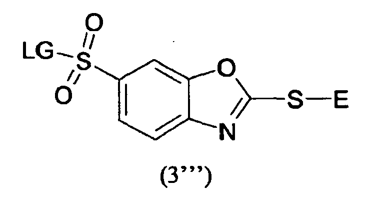

4. A method according to any one of claims 1 to 3, **characterized in that** compound of formula (5) is obtained by amination of an epoxide-containing compound of formula (4), and the amination reagent is H₂N-R₄, wherein R₄ is as claimed in any one of claims 1 to 3.

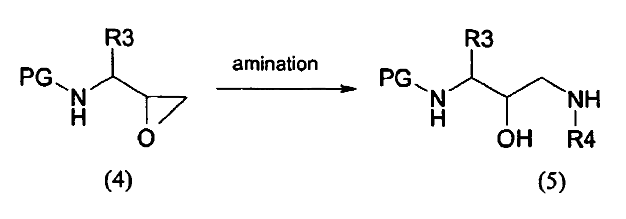

5. A method according to any one of claims 1 to 4, wherein compound of formula (5) is compound of formula (5').

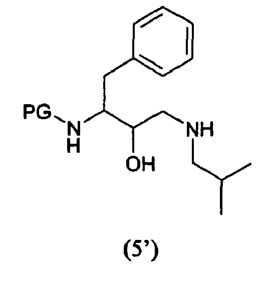

6. A compound having formula (6) 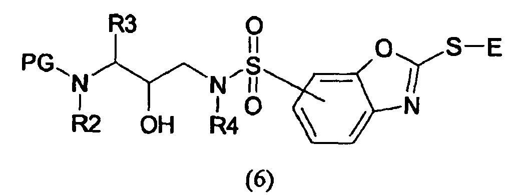 and salts, stereoisomeric forms, and racemic mixtures thereof, **characterized in that PG**, **R₂, R₃, R₄,** and **E** are as defined in any one of claims 1 to 5.

7. A compound according to claim 6, **characterized in that**
**R₂** is hydrogen;
**R₃** is arylC₁₋₄alkyl, arylmethyl, or phenylmethyl;
**R₄** is unsubstituted C₁₋₆alkyl or C₁₋₆alkyl substituted with one or more substituents selected from aryl, Het¹, Het², C₃₋₇cycloalkyl and amino optionally mono-or disubstituted where the substituents are selected from C₁₋₄alkyl, aryl, Het¹ and Het².

8. A compound according to any one of claims 6 to 7, **characterized in that**
**R₂** is hydrogen;
**R₃** is phenylmethyl; and
**R₄** is isobutyl.

9. A compound according to any one of claims 6 to 8, **characterized in that** the compound has formula (6").

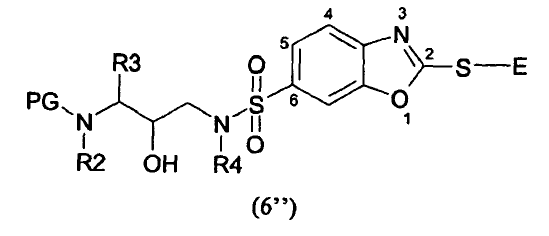

10. A compound according to any one of claims 6 to 9, **characterized in that** the compound has formula (6''').

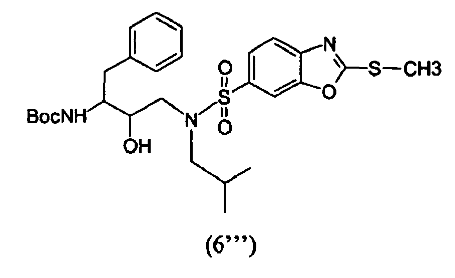

11. A compound according to any one of claims 6 to 10, **characterized in that** said compound is in the form of a salt selected from trifluoroacetate, fumarate, chloroacetate and methanesulfonate.

12. A method for preparing a compound of formula (9), wherein said method comprises the methods according to any one of claims 1 to 5, **characterised in that** said method further comprises
aminating compound of formula (6) to obtain compound of formula (7), wherein 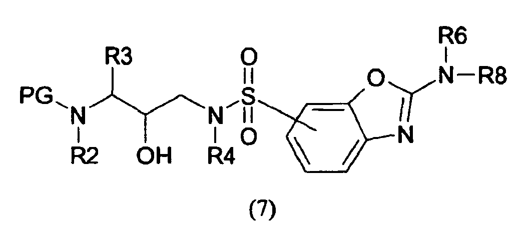
**R₆** is hydrogen, hydroxy, C₁₋₆alkyl, Het¹C₁₋₆alkyl, Het²C₁₋₆alkyl, aminoC₁₋₆alkyl whereby the amino group may optionally be mono-or di-substituted with C₁₋₄alkyl;
**R₈** is hydrogen, C₁₋₆alkyl, or **-A-R₇;**
**A** is C₁₋₆alkanediyl, -C(-O)-, -C(=S)-, -S(=O)₂-, C₁₋₆alkanediyl-C(=O)-, C₁₋₆alkanediyl-C(=S)- or C₁₋₆alkanediyl-S(=O)₂-; whereby the point of attachment to the nitrogen atom is the C₁₋₆alkanediyl group in those moieties containing said group;
**R₇** is C₁₋₆alkyloxy, Het¹, Het¹oxy, Het², Het²oxy, aryl, aryloxy, C₃₋₇cycloalkyl, or optionally mono- or disubstituted amino; and
in case **-A-** is other than C₁₋₆alkanediyl then **R₇** may also be C₁₋₆alkyl, Het¹C₁₋₄alkyl, Het¹oxyC₁₋₄alkyl, Het²C₁₋₄alkyl, Het²oxyC₁₋₄alkyl, arylC₁₋₄alkyl, aryloxyC₁₋₄alkyl or amino-C₁₋₆alkyl; whereby each of the amino groups in the definition of **R₇** may optionally be substituted with one or more substituents selected from C₁₋₄alkyl, C₁₋₄alkylcarbonyl, C₁₋₄alkyloxycarbonyl, aryl, arylcarbonyl, aryloxycarbonyl, Het¹, Het², arylC₁₋₄alkyl, Het¹-C₁₋₄alkyl or Het²C₁₋₄alkyl ; and
**-A-R₇** may also be hydroxyC₁₋₆alkyl; and
**R₆** and **-A-R₇** taken together with the nitrogen atom to which they are attached may also form Het¹ or Het²;
deprotecting compound of formula (7) to obtain compound of formula (8), 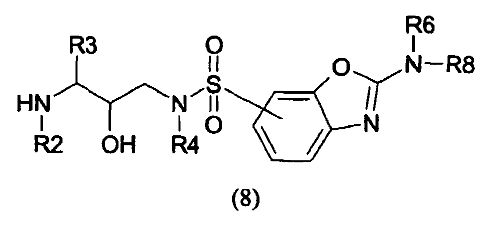 coupling a radical of formula **R₁-L-** to obtain compound of formula (9), 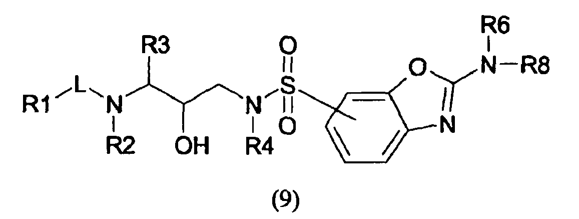 and *N*-oxides, salts, stereoisomeric forms, racemic mixtures, prodrugs, esters and metabolites thereof, wherein
**R₁** is selected from the group comprising hydrogen, C₁₋₆alkyl, C₂₋₆alkenyl, arylC₁₋₆alkyl, C₃₋₇cycloalkyl, C₃₋₇cycloalkylC₁₋₆alkyl, aryl, Het¹, Het¹C₁₋₆alkyl, Het², Het²C₁₋₆alkyl; and **R₁** may also be a radical of formula (10) 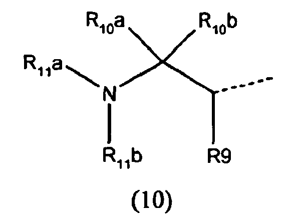
**R₉, R₁₀ₐ and R_{10b}** are, each independently, hydrogen, C₁₋₄alkyloxycarbonyl, carboxyl, aminocarbonyl, mono- or di(C₁₋₄alkyl)aminocarbonyl, C₃₋₇cycloalkyl, C₂₋₆alkenyl, C₂₋₆alkynyl or C₁₋₄alkyl optionally substituted with aryl, Het¹, Het², C₃₋₇cycloalkyl, C₁₋₄alkyloxycarbonyl, carboxyl, aminocarbonyl, mono- or di(C₁₋₄alkyl)-aminocarbonyl, aminosulfonyl, C₁₋₄alkylS(O)ₜ, hydroxy, cyano, halogen or amino optionally mono- or disubstituted where the substituents are each independently selected from C₁₋₄alkyl, aryl, arylC₁₋₄alkyl, C₃₋₇cycloalkyl, C₃₋₇cycloalkylC₁₋₄alkyl, Het¹, Het², Het¹C₁₋₄alkyl and Het²C₁₋₄alkyl; whereby R₉, R₁₀ₐ and the carbon atoms to which they are attached may also form a C₃₋₇cycloalkyl radical;
**when L** is -O-C₁₋₆alkanediyl-C(=O)- or -NR₁₂-C₁₋₆alkanediyl-C(=O)-, **then R₉** may also be oxo;
**R₁₁ₐ** is selected from the group comprising hydrogen, C₂₋₆alkenyl, C₂₋₆alkynyl, C₃₋₇cycloalkyl, aryl, aminocarbonyl optionally mono- or disubstituted, aminoC₁₋₄alkylcarbonyloxy optionally mono- or disubstituted, C₁₋₄alkyloxycarbonyl, aryloxycarbonyl, Het¹oxycarbonyl, Het²oxycarbonyl, aryloxycarbonylC₁₋₄alkyl, arylC₁₋₄alkyloxycarbonyl, C₁₋₄alkylcarbonyl, C₃₋₇cycloalkylcarbonyl, C₃₋₇cycloalkyl-C₁₋₄alkyloxycarbonyl, C₃₋₇cycloalkylcarbonyloxy, carboxylC₁₋₄alkylcarbonyloxy, C₁₋₄alkylcarbonyloxy, arylC₁₋₄alkylcarbonyloxy, arylcarbonyloxy, aryloxycarbonyloxy, Het¹carbonyl, Het¹carbonyloxy, Het¹C₁₋₄alkyloxycarbonyl, Het²carbonyloxy, Het²C₁₋₄alkylcarbonyloxy, Het²C₁₋₄alkyloxycarbonyloxy or C₁₋₄alkyl optionally substituted with aryl, aryloxy, Het² or hydroxy; wherein the substituents on the amino groups are each independently selected from C₁₋₄alkyl, aryl, arylC₁₋₄alkyl, C₃₋₁cycloalkyl, C₃₋₇cycloalkyl
C₁₋₄alkyl, Het¹, Het², Het¹C₁₋₄alkyl and Het²C₁₋₄alkyl;
**R_{11b}** is selected from the group comprising hydrogen, C₃₋₇cycloalkyl, C₂₋₆alkenyl,
C₂₋₆alkynyl, aryl, Het¹, Het² or C₁₋₄alkyl optionally substituted with halogen, hydroxy, C₁₋₄alkylS(=O)ₜ, aryl, C₃₋₇cycloalkyl, Het¹, Het², amino optionally mono- or disubstituted where the substituents are each independently selected from C₁₋₄alkyl, aryl, arylC₁₋₄alkyl,C₃₋₇cycloalkyl, C₃₋₇cycloalkylC₁₋₄alkyl, Het¹, Het², Het¹C₁₋₄alkyl and Het²C₁₋₄alkyl;
whereby **R_{11b}** may be linked to the remainder of the molecule via a sulfonyl group; and
**L** is selected from the group comprising -C(=O)-, -O-C(=O)-, -NR₁₂-C(=O)-, -O-C₁₋₆alkanediyl-C(=O)-, -NR₁₂-C₁₋₆alkanediyl-C(=O)-, -S(=O)₂-, -O-S(=O)₂-, - NR₁₂-S(=O)₂ whereby either the C(=O) group or the S(=O)₂ group is attached to the NR₂ moiety; whereby the C₁₋₆alkanediyl moiety is optionally substituted with a substituent selected from hydroxy, aryl, Het¹, and Het²;
**R₁₂** is hydrogen, C₁₋₆alkyl, C₂₋₆alkenyl, arylC₁₋₆alkyl, C₃₋₇cycloalkyl, C₃₋₇cycloalkylC₁₋₆alkyl, aryl, Het¹, Het¹C₁₋₆alkyl, Het², Het²C₁₋₆alkyl;
**R₂** is hydrogen or C₁₋₆alkyl;
**R₃** is C₃₋₇cycloalkyl, aryl, Het¹, Het², or C₁₋₆alkyl optionally substituted with C₃₋₇cycloalkyl, aryl, Het¹, or Het²; wherein each C₃₋₇cycloalkyl, aryl, Het¹, and Het² may be optionally substituted with one or more groups selected from oxo, C₁₋₆alkyloxy, C₁₋₆alkyl, C₁₋₆alkylsulfonyl, aminosulfonyl, amino, C₁₋₆alkylcarbonylamino, hydroxyC₁₋₆alkyl, cyano, C₁₋₆alkyloxycarbonyl, aminocarbonyl, halogen or trifluoromethyl, wherein each amino maybe mono- or disubstitued with C₁₋₆alkyl;
**R₄** is selected from the group comprising hydrogen, C₁₋₄alkyloxycarbonyl, carboxyl, aminocarbonyl, mono- or di(C₁₋₄alkyl)aminocarbonyl, C₃₋₇cycloalkyl, C₂₋₆alkenyl, C₂₋₆alkynyl, or C₁₋₆alkyl optionally substituted with one or more substituents each independently selected from aryl, Het¹, Het², C₃₋₇cycloalkyl, C₁₋₄alkyloxycarbonyl, carboxyl, aminocarbonyl, mono- or di(C₁₋₄alkyl)aminocarbonyl, aminosulfonyl, C₁₋₄alkyl-S(=O)ₜ, hydroxy, cyano, halogen and amino optionally mono-or disubstituted where the substituents are each independently selected from C₁₋₄alkyl, aryl, arylC₁₋₄alkyl, C₃₋₇cycloalkyl, C₃₋₇cycloalkylC₁₋₄alkyl, Het¹, Het², Het¹C₁₋₄alkyl and Het²C₁₋₄alkyl; and
**t** is zero, one or two.

13. The method according to claim 12, wherein
**R₁** is a radical of formula (10) 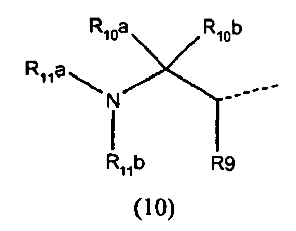
**R₉, R₁₀ₐ** and **R_{10b}** are, each independently, hydrogen, C₁₋₄alkyloxycarbonyl, carboxyl, aminocarbonyl, mono- or di(C₁₋₄alkyl)aminocarbonyl, C₃₋₇cycloalkyl, C₂₋₆alkenyl, C₂₋₆alkynyl or C₁₋₄alkyl optionally substituted with aryl, Het¹, Het², C₃₋₇cycloalkyl, C₁₋₄alkyloxycarbonyl, carboxyl, aminocarbonyl, mono- or di(C₁₋₄alkyl)-aminocarbonyl, aminosulfonyl, C₁₋₄alkylS(O)ₜ, hydroxy, cyano, halogen or amino optionally mono- or disubstituted where the substituents are each independently selected from C₁₋₄alkyl, aryl, arylC₁₋₄alkyl, C₃₋₇cycloalkyl, C₃₋₇cycloalkyl-C₁₋₄alkyl, Het¹, Het², Het¹C₁₋₄alkyl and Het²C₁₋₄alkyl;
whereby **R₉, R₁₀ₐ** and the carbon atoms to which they are attached may also form a C₃₋₇cycloalkyl radical;
**R_{11b}** is hydrogen, C₃₋₇cycloalkyl, C₂₋₆alkenyl, C₂₋₆alkynyl, aryl, Het¹, Het² or C₁₋₄alkyl optionally substituted with halogen, hydroxy, C₁₋₄alkylS(=O)ₜ, aryl, C₃₋₇cycloalkyl, Het¹, Het², amino optionally mono- or disubstituted where the substituents are each independently selected from C₁₋₄alkyl, aryl, arylC₁₋₄alkyl, C₃₋₇cycloalkyl, C₃₋₇cycloalkylC₁₋₄alkyl, Het¹, Het², Het¹C₁₋₄alkyl and Het²C₁₋₄alkyl;
whereby **R**_{11b} may be linked to the remainder of the molecule via a sulfonyl group;
**t** is zero, one or two;
**L** is -C(=O)-, -O-C(=O)-, -NR₁₂-C(=O)-, -O-C₁₋₆alkanediyl-C(=O)-, -NR₁₂-C₁₋₆alkanediyl-C(=O)-, -S(=O)₂-, -O-S(=O)₂-, -NR₁₂-S(=O)₂ whereby either the C(=O) group or the S(=O)₂ group is attached to the NR₂ moiety; whereby the C₁₋₆alkanediyl moiety is optionally substituted with a substituent selected from hydroxy, aryl, Het¹, and Het²;
**R₁₂** is hydrogen, C₁₋₆alkyl, C₂₋₆alkenyl, arylC₁₋₆alkyl, C₃₋₇cycloalkyl, C₃₋₇cycloalkylC₁₋₆alkyl, aryl, Het¹, Het¹C₁₋₆alkyl, Het², Het²C₁₋₆alkyl; and
**R₄** is hydrogen, C₁₋₄alkyloxycarbonyl, carboxyl, aminocarbonyl, mono- or di(C₁₋₄alkyl)aminocarbonyl, C₃₋₇cycloalkyl, C₂₋₆alkenyl, C₂₋₆alkynyl, or C₁₋₆alkyl optionally substituted with one or more substituents selected from aryl, Het¹, Het², C₃₋₇cycloalkyl, C₁₋₄alkyloxycarbonyl, carboxyl, aminocarbonyl, mono- or di(C₁₋₄alkyl)-aminocarbonyl, aminosulfonyl, C₁₋₄alkylS(=O)ₜ, hydroxy, cyano, halogen and amino optionally mono- or disubstituted where the substituents are selected from C₁₋₄alkyl, aryl, arylC₁₋₄alkyl, C₃₋₇cycloalkyl, C₃₋₇cycloalkyl-C₁₋₄alkyl, Het¹, Het², Het¹C₁₋₄alkyl and Het²C₁₋₄alkyl.

14. The method according to any one of claims 12 to 13, wherein one or more of the following restrictions apply:
**R₁** is hydrogen, Het¹, Het², aryl, Het¹C₁₋₆alkyl, Het²C₁₋₆alkyl, arylC₁₋₆alkyl, more in particular, R₁ is a saturated or partially unsaturated monocyclic or bicyclic heterocycle having 5 to 8 ring members, which contains one or more heteroatom ring members selected from nitrogen, oxygen or sulfur and which is optionally substituted, or phenyl optionally substituted with one or more substituents;
**R₂** is hydrogen;
**L** is -C(=O)-, -O-C(=O)-, -O-C₁₋₆alkanediyl-C(=O)-, more in particular, L is -O-C(=O)- or -O-C₁₋₆alkanediyl-C(=O)-, whereby in each case the C(=O) group is attached to the NR₂ moiety;
**R₃** is arylC₁₋₄alkyl, in particular, arylmethyl, more in particular phenylmethyl;
**R₄** is optionally substituted C₁₋₆alkyl, in particular unsubstituted C₁₋₆alkyl or C₁₋₆alkyl optionally substituted with one or more substituents selected from aryl, Het¹, Het², C₃₋₇cycloalkyl and amino optionally mono- or disubstituted where the substituents are selected from C₁₋₄alkyl, aryl, Het¹ and Het²;
**R₆** is hydrogen or methyl; and
**R₈** is hydrogen or methyl.

15. The method according to any one of claims 12 to 14, wherein
**R₁-L** is Het¹-O-C(=O), Het²-C₁₋₆alkanediyl-O-C(=O), aryl-O-C₁₋₆alkanediyl-C(=O) or aryl-C(=O).

16. The method according to any one of claims 12 to 15, wherein
**NR₆R₈** is amino, monomethylamino or dimethylamino.

17. The method according to to any one of claims 12 to 16, wherein
**R₁** is a Het¹, or a Het¹C₁₋₆alkyl, and
**L** is -O-C(=O)-;
**R₂** is hydrogen;
**R₃** is phenylmethyl;
**R₄** is isobutyl;
**R₆** is hydrogen; and
**R₈** is hydrogen or methyl.

18. The method according to any one of claims 12 to 17, wherein compound (9) has formula (9''').

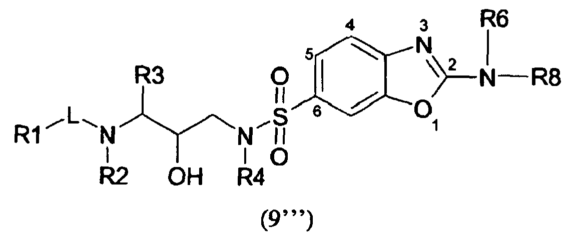

19. The method according to any one of claims 12 to 18, **characterized in that** compound of formula (9) is in the form of a salt selected from trifluoroacetate, fumarate, chloroacetate and methanesulfonate.

20. Use of a compound as claimed in any of claims 7 to 11 as an intermediate for preparing a retrovirus protease inhibitor of formula (9).

## Patentansprüche

1. Verfahren zur Herstellung einer Verbindung der Formel (6), 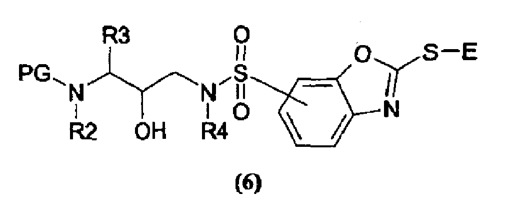 sowie Salze, stereoisomere Formen und racemische Gemische davon, **dadurch gekennzeichnet, daß** man in dem Verfahren von einer Verbindung der Formel (2), 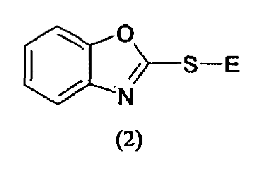 worin
**E** für eine elektrophile Gruppierung steht,
ausgeht, die Verbindung der Formel (2) in eine Verbindung der Formel (3), 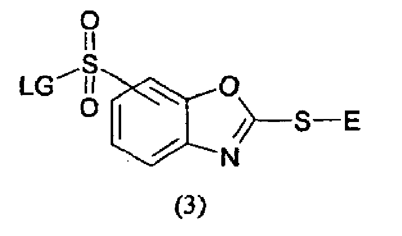 worin
**LG** für eine Abgangsgruppe steht,
überführt und die Verbindung der Formel (3) mit einer Verbindung der Formel (5), 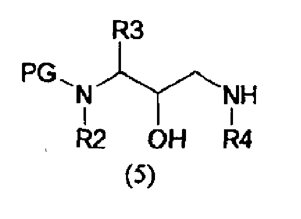 worin
**PG** für eine Schutzgruppe steht,
**R₂** für Wasserstoff oder C₁₋₆-Alkyl steht,
**R₃** für C₃₋₇-Cycloalkyl, Aryl, Het¹, Het² oder gegebenenfalls mit C₃₋₇-Cycloalkyl, Aryl, Het¹ oder Het² substituiertes C₁₋₆-Alkyl, worin C₃₋₇-Cycloalkyl, Aryl, Het¹ und Het² jeweils gegebenenfalls mit einer oder mehreren Gruppen, ausgewählt aus Oxo, C₁₋₆-Alkyloxy, C₁₋₆-Alkyl, C₁₋₆-Alkylsulfonyl, Aminosulfonyl, Amino, C₁₋₆-Alkylcarbonylamino, Hydroxy-C₁₋₆-alkyl, Cyano, C₁₋₆-Alkyloxycarbonyl, Aminocarbonyl, Halogen oder Trifluormethyl, substituiert sein kann, worin Amino jeweils ein- oder zweifach mit C₁₋₆-Alkyl substituiert sein kann, steht,
**R₄** ausgewählt ist aus der Gruppe Wasserstoff, C₁₋₄-Alkyloxycarbonyl, Carboxyl, Aminocarbonyl, Mono- oder Di(C₁₋₄-alkyl)aminocarbonyl, C₃₋₇-Cycloalkyl, C₂-₆-Alkenyl, C₂₋₆-Alkinyl oder C₁₋₆-Alkyl, gegebenenfalls substituiert mit einem oder mehreren Substituenten, jeweils unabhängig ausgewählt aus Aryl, Het¹, Het², C₃₋₇-Cycloalkyl, C₁₋₄-Alkyloxycarbonyl, Carboxyl, Aminocarbonyl, Mono- oder Di(C₁₋₄-alkyl)aminocarbonyl, Aminosulfonyl, C₁₋₄-Alkyl-S(=O)ₜ, Hydroxy, Cyano, Halogen und gegebenenfalls ein- oder zweifach substituiertem Amino, wobei die Substituenten jeweils unabhängig ausgewählt sind aus C₁₋₄-Alkyl, Aryl, Aryl-C₁₋₄-alkyl, C₃₋₇-Cycloalkyl, C₃₋₇-Cycloalkyl-C₁₋₄-alkyl, Het¹, Het², Het¹-C₁₋₄-alkyl und Het²-C₁₋₄-alkyl, und **t** gleich null, eins oder zwei ist, umsetzt.

2. Verfahren nach Anspruch 1 zur Herstellung einer Verbindung der Formel (6), **dadurch gekennzeichnet, daß** man in dem Verfahren die folgenden Schritte durchführt:
Alkylieren einer Verbindung der Formel (1) 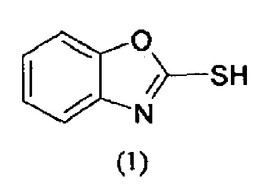
unter Erhalt einer Verbindung der Formel (2) 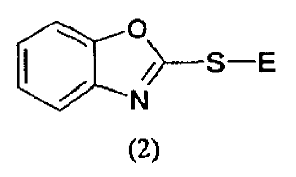 worin
**E** für ein C₁₋₆-Alkyl steht;
Umsetzen der Verbindung der Formel (2) mit einem Sulfonierungsmittel unter Erhalt einer Verbindung der Formel (3) 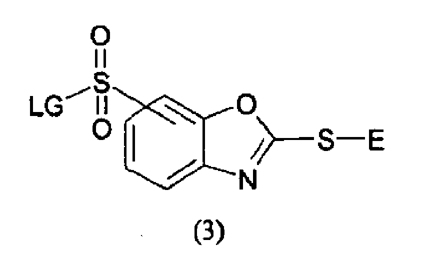 worin
**LG** für eine Abgangsgruppe steht; und
Kuppeln der Verbindung der Formel (3) mit einer Verbindung der Formel (5) 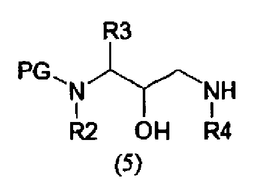 worin
**PG** für eine Schutzgruppe steht und
worin **R₂, R₃** und **R₄** die in Anspruch 1 angegebene Bedeutung aufweisen.

3. Verfahren nach einem der Ansprüche 1 bis 2, **dadurch gekennzeichnet, daß** es sich bei der Verbindung der Formel (3) um eine Verbindung der Formel (3''') handelt.

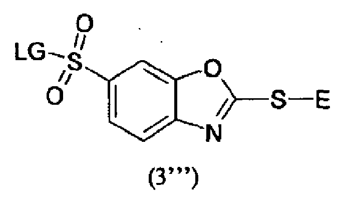

4. Verfahren nach einem der Ansprüche 1 bis 3, **dadurch gekennzeichnet, daß** man die Verbindung der Formel (5) durch Aminierung einer epoxidhaltigen Verbindung der Formel (4) gewinnt und es sich bei dem Aminierungsreagenz um H₂N-R₄ handelt, worin R₄ die in einem der Ansprüche 1 bis 3 angegebene Bedeutung aufweist.

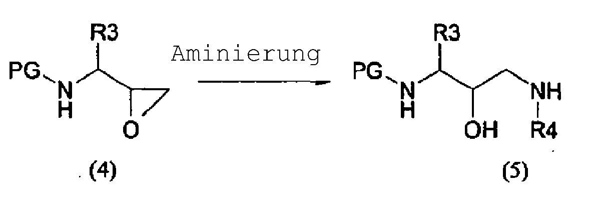

5. Verfahren nach einem der Ansprüche 1 bis 4, wobei es sich bei der Verbindung der Formel (5) um eine Verbindung der Formel (5') handelt.

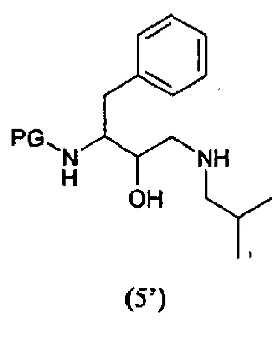

6. Verbindung mit der Formel (6) 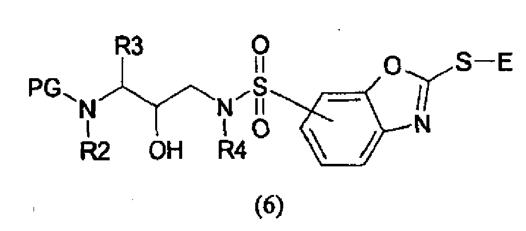 sowie Salze, stereoisomere Formen und racemische Gemische davon, **dadurch gekennzeichnet, daß PG, R₂, R₃, R₄** und **E** die in einem der Ansprüche 1 bis 5 angegebene Definition besitzen.

7. Verbindung nach Anspruch 6, **dadurch gekennzeichnet, daß**
**R₂** für Wasserstoff,
**R₃** für Aryl-C₁₋₄-alkyl, Arylmethyl oder Phenylmethyl, **R₄** für unsubstituiertes C₁₋₆-Alkyl oder mit einem oder mehreren Substituenten, ausgewählt aus Aryl, Het¹, Het², C₃₋₇-Cycloalkyl und gegebenenfalls ein- oder zweifach substituiertem Amino, wobei die Substituenten jeweils unabhängig ausgewählt sind aus C₁₋₄-Alkyl, Aryl, Het¹ und Het², substituiertes C₁₋₆-Alkyl steht.

8. Verbindung nach einem der Ansprüche 6 bis 7, **dadurch gekennzeichnet, daß**
**R₂** für Wasserstoff,
**R₃** für Phenylmethyl und
**R₄** für Isobutyl steht.

9. Verbindung nach einem der Ansprüche 6 bis 8, **dadurch gekennzeichnet, daß** die Verbindung die Formel (6") aufweist.

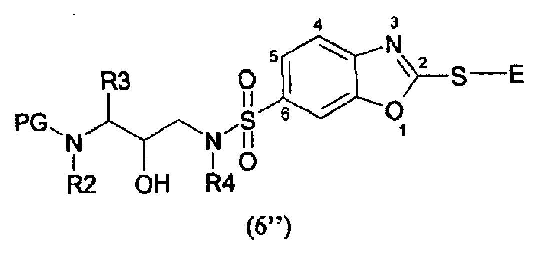

10. Verbindung nach einem der Ansprüche 6 bis 9, **dadurch gekennzeichnet, daß** die Verbindung die Formel (6''') aufweist.

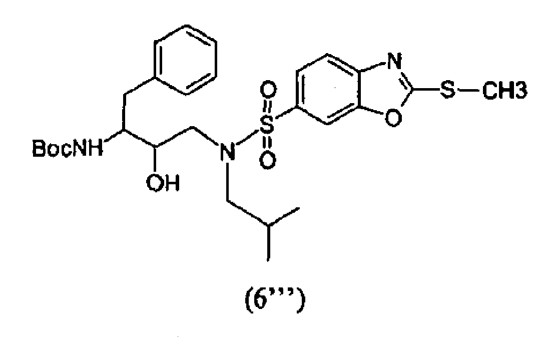

11. Verbindung nach einem der Ansprüche 6 bis 10, **dadurch gekennzeichnet, daß** die Verbindung in Form eines Salzes, ausgewählt aus Trifluoracetat, Fumarat, Chloracetat und Methansulfonat, vorliegt.

12. Verfahren zur Herstellung einer Verbindung der Formel (9), das die Verfahren nach einem der Ansprüche 1 bis 5 umfaßt, **dadurch gekennzeichnet, daß** man in dem Verfahren ferner
die Verbindung der Formel (6) unter Erhalt der Verbindung der Formel (7) aminiert, worin 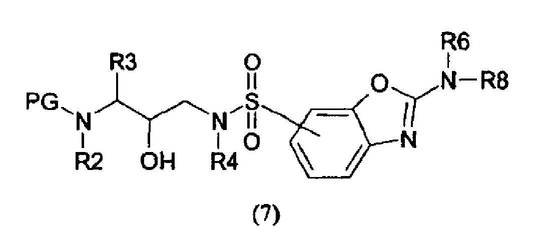
**R₆** für Wasserstoff, Hydroxy, C₁₋₆-Alkyl, Het¹-C₁₋₆-alkyl, Het²-C₁₋₆-alkyl, Amino-C₁₋₆-alkyl, wobei die Aminogruppe gegebenenfalls ein- oder zweifach mit C₁₋₄-Alkyl substituiert sein kann, steht,
**R₆** für Wasserstoff C₁₋₆-Alkyl oder **-A-R₇** steht,
**A** für C₁₋₆-Alkandiyl, -C(=O)-, -C(=S)-, -S(=O)₂-, C₁-₆-Alkandiyl-C(=O)-, C₁₋₆-Alkandiyl-C(=S)- oder C₁₋₆-Alkandiyl-S(=O)₂- steht, wobei die C₁₋₆-Alkandiylgruppe die Verknüpfungsstelle mit dem Stickstoffatom in denjenigen Gruppierungen, die diese Gruppe enthalten, darstellt,
**R₇** für C₁₋₆-Alkyloxy, Het¹, Het¹oxy, Het², Het²oxy, Aryl, Aryloxy, C₃₋₇-Cycloalkyl oder gegebenenfalls ein- oder zweifach substituiertes Amino steht und, falls **-A-** nicht C₁₋₆-Alkandiyl ist, auch für C₁₋₆-Alkyl, Het¹-C₁₋₄-alkyl, Het¹oxy-C₁₋₄-alkyl, Het²-C₁₋₄-alkyl, Het²oxy-C₁₋₄-alkyl, Aryl-C₁₋₄-alkyl, Aryloxy-C₁₋₄-alkyl oder Amino-C₁₋₆-alkyl stehen kann, wobei die Aminogruppen in der Definition von **R₇** gegebenenfalls mit einem oder mehreren Substituenten, ausgewählt aus C₁₋₄-Alkyl, C₁₋₄-Alkylcarbonyl, C₁₋₄-Alkyloxycarbonyl, Aryl, Arylcarbonyl, Aryloxycarbonyl, Het¹, Het², Aryl-C₁₋₄-alkyl, Het¹-C₁₋₄-alkyl oder Het²-C₁₋₄-alkyl, substituiert sein können, und
**-A-R₇** auch für Hydroxy-C₁₋₆-alkyl stehen kann und
**R₆** und **-A-R₇** zusammengenommen mit dem Stickstoffatom, mit dem sie verknüpft sind, auch Het¹ oder Het² bilden können,
die Verbindung der Formel (7) unter Erhalt der Verbindung der Formel (8) entschützt, 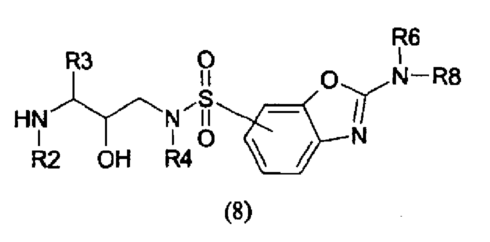 einen Rest der Formel R₁-L- unter Erhalt der Verbindung der Formel (9) 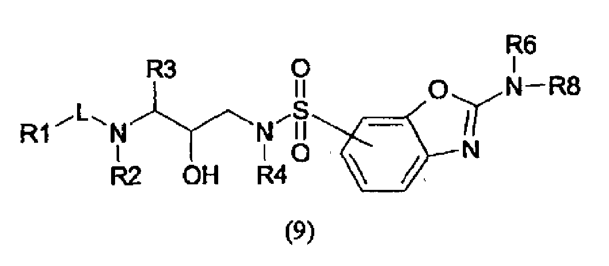 sowie von N-Oxiden, Salzen, stereoisomeren Formen, racemischen Gemischen, Arzneistoffvorstufen (Prodrugs), Estern und Metaboliten davon ankuppelt, worin
**R₁** ausgewählt ist aus der Gruppe Wasserstoff, C₁₋₆-Alkyl, C₂₋₆-Alkenyl, Aryl-C₁₋₆-alkyl, C₃₋₇-Cycloalkyl, C₃₋₇-Cycloalkyl-C₁₋₆-alkyl, Aryl, Het¹, Het¹-C₁₋₆-alkyl, Het², Het²-C₁₋₆-alkyl und auch für einen Rest der Formel (10) 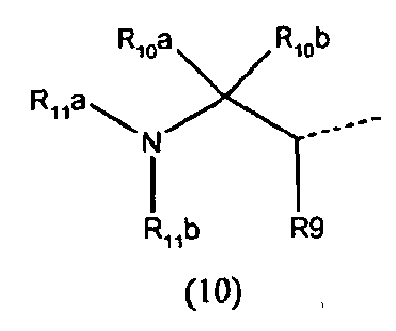
stehen kann,
**R₉, R₁₀ₐ und R_{10b}** jeweils unabhängig für Wasserstoff, C₁₋₄-Alkyloxycarbonyl, Carboxyl, Aminocarbonyl, Mono- oder Di(C₁₋₄-alkyl)aminocarbonyl, C₃₋₇-Cycloalkyl, C₂-₆-Alkenyl, C₂₋₆-Alkinyl oder C₁₋₄-Alkyl, gegebenenfalls substiuiert mit Aryl, Het¹, Het², C₃₋₇-Cycloalkyl, C₁₋₄-Alkyloxycarbonyl, Carboxyl, Aminocarbonyl, Mono- oder Di(C₁₋₄-alkyl)aminocarbonyl, Aminosulfonyl, C₁₋₄-AlkylS(=O)ₜ, Hydroxy, Cyano, Halogen oder gegebenenfalls ein- oder zweifach substituiertem Amino, wobei die Substituenten jeweils unabhängig ausgewählt sind aus C₁₋₄-Alkyl, Aryl, Aryl-C₁₋₄-alkyl, C₃₋₇-Cycloalkyl, C₃₋₇-Cycloalkyl-C₁₋₄-alkyl, Het¹, Het², Het¹-C₁₋₄-alkyl und Het²-C₁₋₄-alkyl, stehen, wobei R₉, R₁₀ₐ und die Kohlenstoffatome, mit denen sie verknüpft sind, auch einen C₃₋₇-Cycloalkyl-Rest bilden können,
**R₉** auch für Oxo stehen kann, **wenn L** für -O-C₁₋₆-Alkandiyl-C(=O)- oder NR₁₂-C₁₋₆-Alkandiyl-C(=O)-steht,
**R₁₁ₐ** ausgewählt ist aus der Gruppe Wasserstoff, C₂₋₆-Alkenyl, C₂₋₆-Alkinyl, C₃₋₇-Cycloalkyl, Aryl, gegebenenfalls einfach oder zweifach substituiertes Aminocarbonyl, gegebenenfalls einfach oder zweifach substituiertes Amino-C₁₋₄-alkylcarbonyloxy, C₁₋₄-Alkyloxycarbonyl, Aryloxycarbonyl, Het¹oxycarbonyl, Het²oxycarbonyl, Aryloxycarbonyl-C₁₋₄-alkyl, Aryl-C₁₋₄-alkyloxycarbonyl, C₁₋₄-Alkylcarbonyl, C₃₋₇-Cycloalkylcarbonyl, C₃₋₇-Cycloalkyl-C₁₋₄-alkyloxycarbonyl, C₃₋₇-Cycloalkylcarbonyloxy, Carboxyl-C₁₋₄-alkylcarbonyloxy, C₁₋₄-Alkylcarbonyloxy, Aryl-C₁₋₄-alkylcarbonyloxy, Arylcarbonyloxy, Aryloxycarbonyloxy, Het¹carbonyl, Het¹carbonyloxy, Het¹-C₁₋₄-alkyloxycarbonyl, Het²carbonyloxy, Het²-C₁₋₄-alkylcarbonyloxy, Het²-C₁₋₄-alkyloxycarbonyloxy oder gegebenenfalls mit Aryl, Aryloxy, Het² oder Hydroxy substituiertes C₁₋₄-Alkyl, worin die Substituenten an den Aminogruppen jeweils unabhängig ausgewählt sind aus C₁₋₄-Alkyl, Aryl, Aryl-C₁₋₄-alkyl, C₃₋₇-Cycloalkyl, C₃₋₇-Cycloalkyl-C₁₋₄-alkyl, Het¹, Het², Het¹-C₁₋₄-alkyl und Het²-C₁₋₄-alkyl,
**R_{11b}** ausgewählt ist aus der Gruppe Wasserstoff, C₃₋₇-Cycloalkyl, C₂₋₆-Alkenyl, C₂₋₆-Alkinyl Aryl, Het¹, Het² oder C₁₋₄-Alkyl, gegebenenfalls substiuiert mit Halogen, Hydroxy, C₁₋₄-Alkyl-S (=O)ₜ, Aryl, C₃₋₇-Cycloalkyl, Het¹, Het², gegebenenfalls ein- oder zweifach substituiertem Amino, wobei die Substituenten jeweils unabhängig ausgewählt sind aus C₁₋₄-Alkyl, Aryl, Aryl-C₁₋₄-alkyl, C₃₋₇-Cycloalkyl, C₃₋₇-Cycloalkyl-C₁₋₄-alkyl, Het¹, Het², Het¹-C₁₋₄-alkyl und Het²-C₁₋₄-alkyl,
wobei **R_{11b}** mit dem Rest des Moleküls über eine Sulfonylgruppe verbunden sein kann, und
**L** ausgewählt ist aus der Gruppe -C(=O)-, -O-C(=O)-, -NR₁₂-C (=O) -, -O-C₁₋₆-Alkandiyl-C(=O)-, -NR₁₂-C₁₋₆-Alkandiyl-C(=O)-, -S(=O)₂-, -O-S(=O)₂-, -NR₁₂-S(=O)₂, wobei entweder die C(=O)-Gruppe oder die S(=O)₂-Gruppe mit der NR₂-Gruppierung verknüpft ist, wobei die C₁₋₆-Alkandiyl-Gruppierung gegebenenfalls mit einem Substituenten, ausgewählt aus Hydroxy, Aryl, Het¹ und Het², substituiert ist,
**R₁₂** für Wasserstoff, C₁₋₆-Alkyl, C₂₋₆-Alkenyl, Aryl-C₁₋₆-alkyl, C₃₋₇-Cycloalkyl, C₃₋₇-Cycloalkyl-C₁₋₆-alkyl, Aryl, Het¹, Het¹-C₁₋₆-alkyl, Het², Het²-C₁₋₆-alkyl, steht,
**R₂** für Wasserstoff oder C₁₋₆-Alkyl steht,
**R₃** für C₃₋₇-Cycloalkyl, Aryl, Het¹, Het² oder gegebenenfalls mit C₃₋₇-Cycloalkyl, Aryl, Het¹ oder Het² substituiertes C₁₋₆-Alkyl, worin C₃₋₇-Cycloalkyl, Aryl, Het¹ und Het² jeweils gegebenenfalls mit einer oder mehreren Gruppen, ausgewählt aus Oxo, C₁₋₆-Alkyloxy, C₁₋₆-Alkyl, C₁₋₆-Alkylsulfonyl, Aminosulfonyl, Amino, C₁₋₆-Alkylcarbonylamino, Hydroxy-C₁₋₆-alkyl, Cyano, C₁₋₆-Alkyloxycarbonyl, Aminocarbonyl, Halogen oder Trifluormethyl, substituiert sein kann, worin Amino jeweils ein- oder zweifach mit C₁₋₆-Alkyl substituiert sein kann, steht,
**R₄** ausgewählt ist aus der Gruppe Wasserstoff, C₁₋₄-Alkyloxycarbonyl, Carboxyl, Aminocarbonyl, Mono- oder Di(C₁₋₄-alkyl)aminocarbonyl, C₃₋₇-Cycloalkyl, C₂-₆-Alkenyl, C₂₋₆-Alkinyl oder C₁₋₆-Alkyl, gegebenenfalls substituiert mit einem oder mehreren Substituenten, jeweils unabhängig ausgewählt aus Aryl, Het¹, Het², C₃₋₇-Cycloalkyl, C₁₋₄-Alkyloxycarbonyl, Carboxyl, Aminocarbonyl, Mono- oder Di(C₁₋₄-alkyl)aminocarbonyl, Aminosulfonyl, C₁₋₄-Alkyl-S(=O)ₜ, Hydroxy, Cyano, Halogen und gegebenenfalls ein- oder zweifach substituiertem Amino, wobei die Substituenten jeweils unabhängig ausgewählt sind aus C₁₋₄-Alkyl, Aryl, Aryl-C₁₋₄-alkyl, C₃₋₇-Cycloalkyl, C₃₋₇-Cycloalkyl-C₁₋₄-alkyl, Het¹, Het², Het¹-C₁₋₄-alkyl und Het²-C₁₋₄-alkyl, und
**t** gleich null, eins oder zwei ist.

13. Verfahren nach Anspruch 12, bei dem
**R₁** für einen Rest der Formel (10) 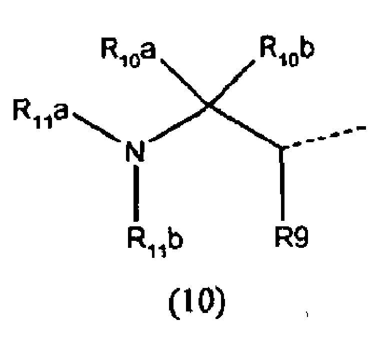 steht,
**R₉, R₁₀ₐ und R_{10b}** jeweils unabhängig für Wasserstoff, C₁₋₄-Alkyloxycarbonyl, Carboxyl, Aminocarbonyl, Mono- oder Di(C₁₋₄-alkyl)aminocarbonyl, C₃₋₇-Cycloalkyl, C₂₋₆-Alkenyl, C₂₋₆-Alkinyl oder C₁₋₄-Alkyl, gegebenenfalls substiuiert mit Aryl, Het¹, Het², C₃₋₇-Cycloalkyl, C₁₋₄-Alkyloxycarbonyl, Carboxyl, Aminocarbonyl, Mono- oder Di(C₁₋₄-alkyl)aminocarbonyl, Aminosulfonyl, C₁₋₄-AlkylS(=O)ₜ, Hydroxy, Cyano, Halogen oder gegebenenfalls ein- oder zweifach substituiertem Amino, wobei die Substituenten jeweils unabhängig ausgewählt sind aus C₁₋₄-Alkyl, Aryl, Aryl-C₁₋₄-alkyl, C₃₋₇-Cycloalkyl, C₃₋₇-Cycloalkyl-C₁₋₄-alkyl, Het¹, Het², Het¹-C₁₋₄-alkyl und Het²-C₁₋₄-alkyl, stehen,
wobei **R₉, R₁₀ₐ** und die Kohlenstoffatome, mit denen sie verknüpft sind, auch einen C₃₋₇-Cycloalkyl-Rest bilden können,
**R_{11b}** für Wasserstoff, C₃₋₇-Cycloalkyl, C₂₋₆-Alkenyl, C₂₋₆-Alkinyl, Aryl, Het¹, Het² oder C₁₋₄-Alkyl, gegebenenfalls substiuiert mit Halogen, Hydroxy, C₁-₄-Alkyl-S(=O)ₜ, Aryl, C₃₋₇-Cycloalkyl, Het¹, Het², gegebenenfalls ein- oder zweifach substituiertem Amino, wobei die Substituenten jeweils unabhängig ausgewählt sind aus C₁₋₄-Alkyl, Aryl, Aryl-C₁₋₄-alkyl, C₃₋₇-Cycloalkyl, C₃₋₇-Cycloalkyl-C₁₋₄-alkyl, Het¹, Het², Het¹-C₁₋₄-alkyl und Het²-C₁₋₄-alkyl, steht, wobei **R_{11b}** mit dem Rest des Moleküls über eine Sulfonylgruppe verbunden sein kann,
**t** gleich null, eins oder zwei ist,
**L** für -C(=O)-, -O-C(=O)-, -NR₁₂-C(=O)-, -O-C₁₋₆-Alkandiyl-C(=O)-, -NR₁₂-C₁₋₆-Alkandiyl-C(=O)-, -S(=O)₂-, -O-S(=O)₂-, -NR₁₂-S(=O)₂- steht, wobei entweder die C(=O)-Gruppe oder die S(=O)₂-Gruppe mit der NR₂-Gruppierung verknüpft ist, wobei die C₁₋₆-Alkandiyl-Gruppierung gegebenenfalls mit einem Substituenten, ausgewählt aus Hydroxy, Aryl, Het¹ und Het², substituiert ist,
**R₁₂** für Wasserstoff, C₁₋₆-Alkyl, C₂₋₆-Alkenyl, Aryl-C₁₋₆-alkyl, C₃₋₇-Cycloalkyl, C₃₋₇-Cycloalkyl-C₁₋₆-alkyl, Aryl, Het¹, Het¹-C₁₋₆-alkyl, Het², Het²-C₁₋₆-alkyl, steht und
**R₄** für Wasserstoff, C₁₋₄-Alkyloxycarbonyl, Carboxyl, Aminocarbonyl, Mono- oder Di(C₁₋₄-alkyl)aminocarbonyl, C₃₋₇-Cycloalkyl, C₂₋₆-Alkenyl, C₂₋₆-Alkinyl oder C₁₋₆-Alkyl, gegebenenfalls substituiert mit einem oder mehreren Substituenten, ausgewählt aus Aryl, Het¹, Het², C₃₋₇-Cycloalkyl, C₁₋₄-Alkyloxycarbonyl, Carboxyl, Aminocarbonyl, Mono- oder Di(C₁₋₄-alkyl)aminocarbonyl, Aminosulfonyl, C₁₋₄-Alkyl-S(=O)ₜ, Hydroxy, Cyano, Halogen und gegebenenfalls ein- oder zweifach substituiertem Amino, wobei die Substituenten ausgewählt sind aus C₁₋₄-Alkyl, Aryl, Aryl-C₁₋₄-alkyl, C₃₋₇-Cycloalkyl, C₃₋₇-Cycloalkyl-C₁₋₄-alkyl, Het¹, Het², Het¹-C₁₋₄-alkyl und Het²-C₁₋₄-alkyl, steht.

14. Verfahren nach einem der Ansprüche 12 bis 13, bei dem eine oder mehrere der folgenden Einschränkungen gelten:
**R₁** steht für Wasserstoff, Het¹, Het², Aryl, Het¹-C₁₋₆-alkyl, Het²-C₁₋₆-alkyl, Aryl-C₁₋₆-alkyl , ganz besonders für einen gesättigten oder teilweise ungesättigten monocyclischen oder bicyclischen Heterocyclus mit 5 bis 8 Ringgliedern, der ein oder mehrere Heteroatomringglieder, ausgewählt aus Stickstoff, Sauerstoff oder Schwefel, enthält und gegebenenfalls substituiert ist, oder gegebenenfalls mit einem oder mehreren Substituenten substituiertes Phenyl;
**R₂** steht für Wasserstoff;
**L** steht für -C(=O)-, -O-C(=O)-, -O-C₁₋₆-Alkandiyl-C(=O)-, ganz besonders für -O-C(=O)- oder -O-C₁₋₆-Alkandiyl-C(=O)-, wobei jeweils die C(=O)-Gruppe mit der -NR₂-Gruppierung verknüpft ist;
**R₃** steht für Aryl-C₁₋₄-alkyl, insbesondere für Arylmethyl, ganz besonders für Phenylmethyl;
**R₄** steht für gegebenenfalls substituiertes C₁₋₆-Alkyl, insbesondere für Arylmethyl, ganz besonders für unsubstituiertes C₁₋₆-Alkyl oder gegebenenfalls mit einem oder mehreren Substituenten, ausgewählt aus Aryl, Het¹, Het², C₃₋₇-Cycloalkyl und gegebenenfalls ein- oder zweifach substituiertem Amino, wobei die Substituenten ausgewählt sind aus C₁₋₄-Alkyl, Aryl, Het¹ und Het², substituiertes C₁₋₆-Alkyl;
**R₆** steht für Wasserstoff oder Methyl und
**R₈** steht für Wasserstoff oder Methyl.

15. Verfahren nach einem der Ansprüche 12 bis 14, bei dem
**R₁-L** für Het¹-O-C(=O), Het²-C₁₋₆-alkandiyl-O-C(=O), Aryl-O-C₁₋₆-alkandiyl-C(=O) oder Aryl-C(=O) steht.

16. Verfahren nach einem der Ansprüche 12 bis 15, bei dem
**NR₆R₈** für Amino, Monomethylamino oder Dimethylamino steht.

17. Verfahren nach einem der Ansprüche 12 bis 16, bei dem
**R₁** für Het¹ oder ein Het¹-C₁₋₆-alkyl und
**L** für -O-C(=O)-,
**R₂** für Wasserstoff,
**R₃** für Phenylmethyl,
**R₄** für Isobutyl,
**R₆** für Wasserstoff und
**R₈** für Wasserstoff oder Methyl steht.

18. Verfahren nach einem der Ansprüche 12 bis 17, bei dem Verbindung (9) die Formel (9''') aufweist.

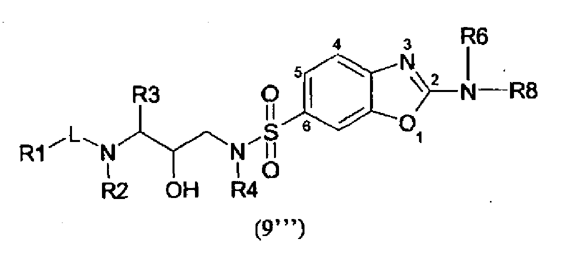

19. Verfahren nach einem der Ansprüche 12 bis 18, **dadurch gekennzeichnet, daß** die Verbindung der Formel (9) in Form eines Salzes, ausgewählt aus Trifluoracetat, Fumarat, Chloracetat und Methansulfonat, vorliegt.

20. Verwendung einer Verbindung gemäß einem der Ansprüche 7 bis 11 als Zwischenstufe bei der Herstellung eines Retrovirusproteaseinhibitors der Formel (9).

## Revendications

1. Méthode de préparation d'un composé de formule (6) 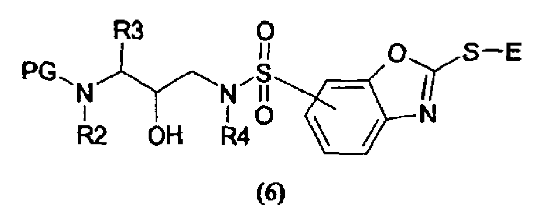 et de sels, de formes stéréoisomères et de mélanges racémiques de celui-ci, **caractérisée en ce que** ladite méthode part d'un composé de formule (2), 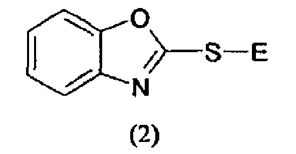 dans laquelle
**E** est un fragment électrophile ;
en transformant un composé de formule (2) en un composé de formule (3) 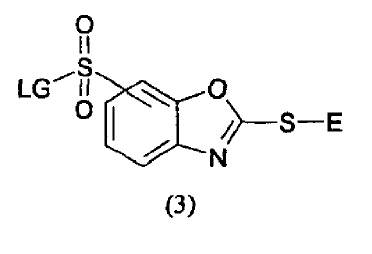 dans laquelle
**LG** est un groupe partant ; et
en faisant réagir un composé de formule (3) avec un composé de formule (5), 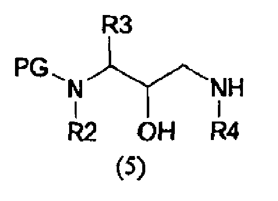 dans laquelle :
**PG** est un groupe protecteur ;
**R₂** est un atome d'hydrogène ou un groupe alkyle en C₁₋₆ ;
**R₃** est un groupe cycloalkyle en C₃₋₇, un groupe aryle, un groupe Het¹, un groupe Het² ou un groupe alkyle en C₁₋₆ éventuellement substitué par un groupe cycloalkyle en C₃₋₇, un groupe aryle, un groupe Het¹ ou un groupe Het², où chaque groupe cycloalkyle en C₃₋₇, chaque groupe aryle, chaque groupe Het¹ et chaque groupe Het² peuvent être éventuellement substitués par un ou plusieurs groupes choisis parmi un groupe oxo, un groupe alkyloxy en C₁₋₆, un groupe alkyle en C₁₋₆, un groupe alkylsulfonyle en C₁₋₆, un groupe aminosulfonyle, un groupe amino, un groupe C₁₋₆-alkylcarbonylamino, un groupe hydroxy-C₁₋₆-alkyle, un groupe cyano, un groupe C₁₋₆-alkyloxycarbonyle, un groupe aminocarbonyle, un atome d'halogène ou un groupe trifluorométhyle, où chaque groupe amino peut être mono- ou disubstitué par un groupe alkyle en C₁₋₆ ;
**R₄** est choisi parmi le groupe comprenant un atome d'hydrogène, un groupe C₁₋₄-alkyloxycarbonyle, un groupe carboxyle, un groupe aminocarbonyle, un groupe mono- ou di-(C₁₋₄-alkyl)aminocarbonyle, un groupe cycloakyle en C₃₋₇, un groupe alcényle en C₂₋₆, un groupe alcynyle en C₂₋₆, ou un groupe alkyle en C₁₋₆ éventuellement substitué par un ou plusieurs substituants choisis chacun indépendamment parmi un groupe aryle, un groupe Het¹, un groupe Het², un groupe cycloalkyle en C₃₋₇, un groupe C₁₋₄-alkyloxycarbonyle, un groupe carboxyle, un groupe aminocarbonyle, un groupe mono- ou di-(C₁₋₄-alkyl) aminocarbonyle, un groupe aminosulfonyle, un groupe C₁₋₄-alkyl-S(=O)ₜ, un groupe hydroxy, un groupe cyano, un atome d'halogène et un groupe amino éventuellement mono- ou disubstitué, où les substituants sont choisis chacun indépendamment parmi un groupe alkyle en C₁₋₄, un groupe aryle, un groupe aryl-C₁₋₄-alkyle, un groupe cycloakyle en C₃₋₇, un groupe C₃₋₇-cycloalkyl-C₁₋₄-alkyle, un groupe Het¹, un groupe Het², un groupe Het¹-C₁₋₄-alkyle et un groupe Het²-C₁₋₄-alkyle ; et
**t** vaut zéro, un ou deux.

2. Méthode selon la revendication 1 pour la préparation d'un composé de formule (6), **caractérisée en ce que** ladite méthode comprend les étapes consistant à :
alkyler un composé de formule (1) 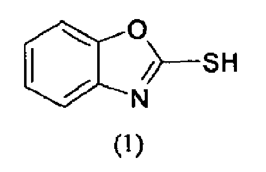
en donnant lieu à un composé de formule (2) 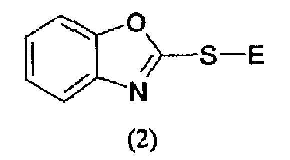 dans laquelle
**E** est un groupe alkyle en C₁₋₆ ;
faire réagir un composé de formule (2) avec un agent de sulfonation, en donnant lieu à un composé de formule (3) 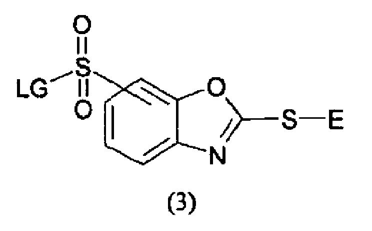 dans laquelle
**LG** est un groupe partant ; et
coupler un composé de formule (3) avec un composé de formule (5) 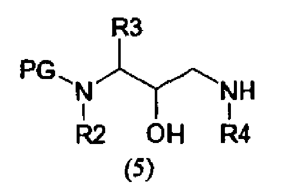 dans laquelle :
**PG** est un groupe protecteur ; et
dans laquelle **R₂, R₃** et **R₄** sont tels que revendiqués dans la revendication 1.

3. Méthode selon l'une quelconque des revendications 1 à 2, **caractérisée en ce qu'**un composé de formule (3) est un composé de formule (3''').

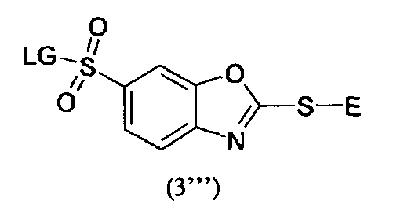

4. Méthode selon l'une quelconque des revendications 1 à 3, **caractérisée en ce qu'**un composé de formule (5) est obtenu par l'amination d'un composé renfermant un groupe époxide de formule (4), et le réactif d'amination est un H₂N-R₄, dans lequel R₄ est tel que revendiqué dans l'une quelconque des revendications 1 à 3.

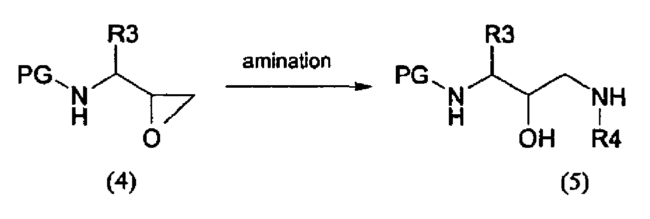

5. Méthode selon l'une quelconque des revendications 1 à 4, dans laquelle un composé de formule (5) est un composé de formule (5').

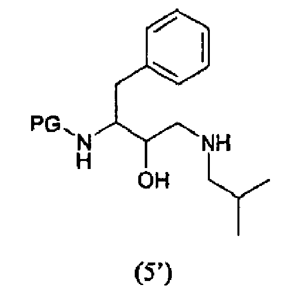

6. Composé de formule (6) 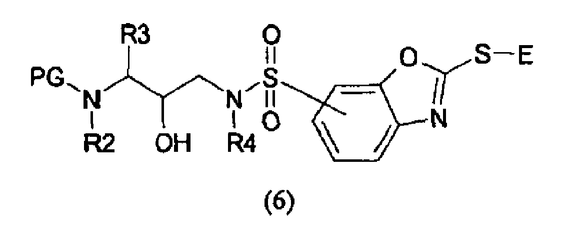 et des sels, des formes stéréoisomères et des mélanges racémiques de celui-ci, **caractérisé en ce que PG, R₂, R₃, R₄** et **E** sont tels que définis dans l'une quelconque des revendications 1 à 5.

7. Composé selon la revendication 6, **caractérisé en ce que** :
**R₂** est un atome d'hydrogène ;
**R₃** est un groupe aryl-C₁₋₄-alkyle, un groupe arylméthyle ou un groupe phénylméthyle ;
**R₄** est un groupe alkyle en C₁₋₆ non-substitué ou un groupe alkyle en C₁₋₆ substitué par un ou plusieurs substituants choisis parmi un groupe aryle, un groupe Het¹, un groupe Het², un groupe cycloalkyle en C₃₋₇ et un groupe amino éventuellement mono- ou di-substitué, où les substituants sont choisis parmi un groupe alkyle en C₁₋₄, un groupe aryle, un groupe Het¹ et un groupe Het².

8. Composé selon l'une quelconque des revendications 6 à 7, **caractérisé en ce que** :
**R₂** est un atome d'hydrogène ;
**R₃** est un groupe phénylméthyle ; et
**R₄** est un groupe isobutyle.

9. Composé selon l'une quelconque des revendications 6 à 8, **caractérisé en ce que** le composé est de formule (6'').

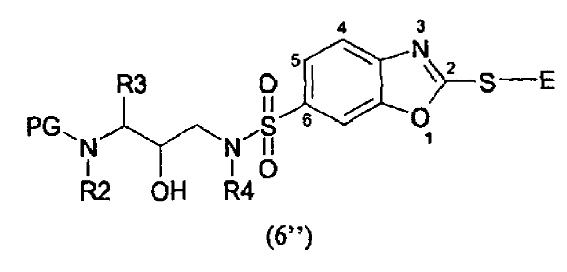

10. Composé selon l'une quelconque des revendications 6 à 9, **caractérisé en ce que** le composé est de formule (6''').

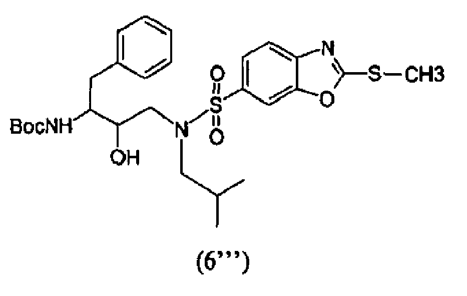

11. Composé selon l'une quelconque des revendications 6 à 10, **caractérisé en ce que** ledit composé est sous la forme d'un sel choisi parmi un trifluoroacétate, un fumarate, un chloroacétate et un méthane-sulfonate.

12. Méthode de préparation d'un composé de formule (9), où ladite méthode comprend les méthodes selon l'une quelconque des revendications 1 à 5, **caractérisée en ce que** ladite méthode comprend en outre :
l'amination d'un composé de formule (6) en vue d'obtenir un composé de formule (7), 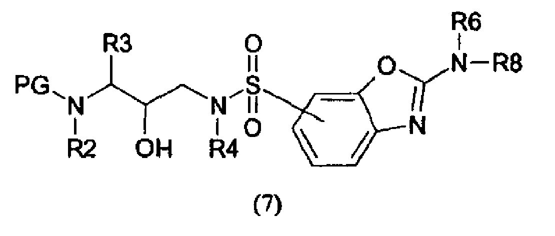 dans laquelle :
**R₆** est un atome d'hydrogène, un groupe hydroxy, un groupe alkyle en C₁₋₆, un groupe Het¹-C₁₋₆-alkyle, un groupe Het²-C₁₋₆-alkyle, un groupe amino-C₁₋₆-alkyle dans lequel le groupe amino peut être éventuellement mono- ou disubstitué par un groupe alkyle en C₁₋₄ ;
**R₈** est un atome d'hydrogène, un groupe alkyle en C₁₋₆ ou un groupe **-A-R₇ ;**
**A** est un groupe C₁₋₆-alcanediyle, un groupe -C(=O)-, un groupe -C(=S)-, un groupe -S (=O)₂-, un groupe C₁₋₆-alcanediyle-C(=O)-, un groupe C₁₋₆-alcanediyl-C(=S)- ou un groupe C₁₋₆-alcanediyl-S(=O)₂- ; où le point de fixation à l'atome d'azote est le groupe C₁₋₆-alcanediyle dans les fragments renfermant ledit groupe ;
**R₇** est un groupe alkyloxy en C₁₋₆, un groupe Het¹, un groupe Het¹oxy, un groupe Het², un groupe Het²oxy, un groupe aryle, un groupe aryloxy, un groupe cycloalkyle en C₃₋₇, ou un groupe amino éventuellement mono- ou di-substitué ; et
dans le cas où **-A-** est autre qu'un groupe C₁₋₆-alcanediyle, alors **R₇** peut également être un groupe alkyle en C₁₋₆, un groupe Het¹-C₁₋₄-alkyle, un groupe Het¹oxy-C₁₋₄-alkyle, un groupe Het²-C₁₋₄-alkyle, un groupe Het²oxy-C₁₋₄-alkyle, un groupe aryl-C₁₋₄-alkyle, un groupe aryloxy-C₁₋₄-alkyle ou un groupe amino-C₁₋₆-alkyle ; où chacun des groupes amino dans la définition de **R₇** peut être éventuellement substitué par un ou plusieurs substituants choisis parmi un groupe alkyle en C₁₋₄, un groupe C₁₋₄-alkylcarbonyle, un groupe C₁₋₄-alkyloxycarbonyle, un groupe aryle, un groupe arylcarbonyle, un groupe aryloxycarbonyle, un groupe Het¹, un groupe Het², un groupe aryl-C₁₋₄-alkyle, un groupe Het¹-C₁₋₄-alkyle ou un groupe Het²-C₁₋₄-alkyle ; et
**-A-R₇** peut également être un groupe hydroxy-C₁₋₆-alkyle ; et
**R₆** et **-A-R₇,** pris conjointement avec l'atome d'azote auquel ils sont fixés, peuvent également former un groupe Het¹ ou un groupe Het² ;
la déprotection d'un composé de formule (7) en vue d'obtenir un composé de formule (8) 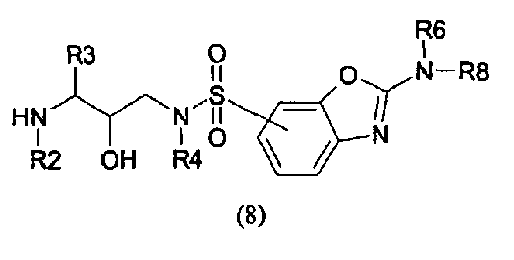
le couplage d'un radical de formule **R₁-L-** en vue d'obtenir un composé de formule (9) 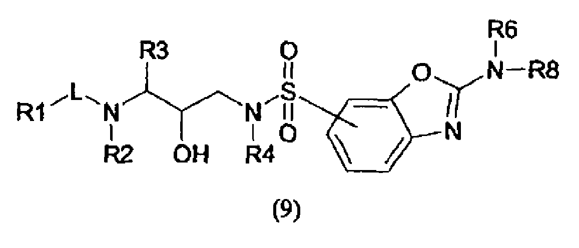
et des N-oxydes, des sels, des formes stéréoisomères, des mélanges racémiques, des pro-médicaments, des esters et des métabolites de celui-ci, où
**R₁** est choisi parmi le groupe comprenant un atome d'hydrogène, un groupe alkyle en C₁₋₆, un groupe alcényle en C₂₋₆, un groupe aryl-C₁₋₆-alkyle, un groupe cycloalkyle en C₃₋₇, un groupe C₃₋₇-cycloalkyl-C₁₋₆-alkyle, un groupe aryle, un groupe Het¹, un groupe Het¹-C₁₋₆-alkyle, un groupe Het², un groupe Het²-C₁₋₆-alkyle ; et **R₁** peut également être un radical de formule (10) 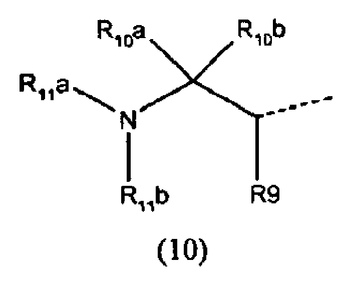
**R9, R₁₀ₐ** et **R_{10b}** sont, chacun indépendamment, un atome d'hydrogène, un groupe C₁₋₄-alkyloxycarbonyle, un groupe carboxyle, un groupe aminocarbonyle, un groupe mono- ou di-(C₁₋₄-alkyl)aminocarbonyle, un groupe cycloalkyle en C₃₋₇, un groupe alcényle en C₂₋₆, un groupe alcynyle en C₂₋₆ ou un groupe alkyle en C₁₋₄ éventuellement substitué par un groupe aryle, un groupe Het¹, un groupe Het², un groupe cycloalkyle en C₃₋₇, un groupe C₁₋₄-alkyloxycarbonyle, un groupe carboxyle, un groupe aminocarbonyle, un groupe mono- ou di-(C₁₋₄-alkyl) aminocarbonyle, un groupe aminosulfonyle, un groupe C₁₋₄-alkyl-S(O)ₜ, un groupe hydroxy, un groupe cyano, un atome d'halogène ou un groupe amino éventuellement mono- ou di-substitué, où les substituants sont choisis chacun indépendamment parmi un groupe alkyle en C₁₋₄, un groupe aryle, un groupe aryl-C₁₋₄-alkyle, un groupe cycloalkyle en C₃₋₇, un groupe C₃₋₇-cycloalkyl-C₁₋₄-alkyle, un groupe Het¹, un groupe Het², un groupe Het¹-C₁₋₄-alkyle et un groupe Het²-C₁₋₄-alkyle ;
où **R₉, R₁₀ₐ** et les atomes de carbone auxquels ils sont fixés peuvent également former un radical cycloalkyle en C₃₋₇ ;
lorsque **L** est un groupe -O-C₁₋₆-alcanediyl-C(=O)- ou un groupe -NR₁₂-C₁₋₆-alcanediyl-C(=O)-, alors **R₉** peut également être un groupe oxo ;
**R₁₁ₐ** est choisi parmi le groupe comprenant un atome d'hydrogène, un groupe alcényle en C₂₋₆, un groupe alcynyle en C₂₋₆, un groupe cycloalkyle en C₃₋₇, un groupe aryle, un groupe aminocarbonyle éventuellement mono- ou disubstitué, un groupe amino-C₁₋₄-alkylcarbonyloxy éventuellement mono- ou di-substitué, un groupe C₁₋₄-alkyloxycarbonyle, un groupe aryloxycarbonyle, un groupe Het¹oxycarbonyle, un groupe Het²oxycarbonyle, un groupe aryloxycarbonyl-C₁₋₄-alkyle, un groupe aryl-C₁₋₄-alkyloxycarbonyle, un groupe C₁₋₄-alkylcarbonyle, un groupe C₃₋₇-cycloalkylcarbonyle, un groupe C₃₋₇-cycloalkyl-C₁₋₄-alkyloxycarbonyle, un groupe C₃₋₇-cycloalkylcarbonyloxy, un groupe carboxyl-C₁₋₄-alkylcarbonyloxy, un groupe C₁₋₄-alkylcarbonyloxy, un groupe aryl-C₁₋₄-alkylcarbonyloxy, un groupe arylcarbonyloxy, un groupe aryloxycarbonyloxy, un groupe Het¹carbonyle, un groupe Het¹carbonyloxy, un groupe Het¹-C₁₋₄-alkyloxycarbonyle, un groupe Het²carbonyloxy, un groupe Het²-C₁₋₄-alkylcarbonyloxy, un groupe Het²-C₁₋₄-alkyloxycarbonyloxy ou un groupe alkyle en C₁₋₄ éventuellement substitué par un groupe aryle, un groupe aryloxy, un groupe Het² ou un groupe hydroxy ; où les substituants sur les groupes amino sont choisis chacun indépendamment parmi un groupe alkyle en C₁₋₄, un groupe aryle, un groupe aryl-C₁₋₄-alkyle, un groupe cycloalkyle en C₃₋₇, un groupe C₃₋₇-cycloalkyl-C₁₋₄-alkyle, un groupe Het¹, un groupe Het², un groupe Het¹-C₁₋₄-alkyle et un groupe Het²-C₁₋₄-alkyle ;
**R_{11b}** est choisi parmi le groupe comprenant un atome d'hydrogène, un groupe cycloalkyle en C₃₋₇, un groupe alcényle en C₂₋₆, un groupe alcynyle en C₂₋₆, un groupe aryle, un groupe Het¹, un groupe Het² ou un groupe alkyle en C₁₋₄ éventuellement substitué par un atome d'halogène, un groupe hydroxy, un groupe C₁₋₄-alkylS(=O)ₜ, un groupe aryle, un groupe cycloalkyle en C₃₋₇, un groupe Het¹, un groupe Het², un groupe amino éventuellement mono- ou disubstitué, où les substituants sont choisis chacun indépendamment parmi un groupe alkyle en C₁₋₄, un groupe aryle, un groupe aryl-C₁₋₄-alkyle, un groupe cycloalkyle en C₃₋₇, un groupe C₃₋₇-cycloalkyl-C₁₋₄-alkyle, un groupe Het¹, un groupe Het², un groupe Het¹-C₁₋₄-alkyle et un groupe Het²-C₁₋₄-alkyle ;
où **R_{11b}** peut être lié au reste de la molécule par l'intermédiaire d'un groupe sulfonyle ; et
**L** est choisi parmi le groupe comprenant un groupe -C(=O)-, un groupe -O-C(=O)-, un groupe -NR₁₂-C(=O)-, un groupe -O-C₁₋₆-alcanediyl-C(=O)-, un groupe -NR₁₂-C₁₋₆-alcanediyl-C(=O)-, un groupe -S(=O)₂-, un groupe -O-S(=O)₂-, un groupe -NR₁₂-S(=O)₂, où soit le groupe C(=O), soit le groupe S(=O)₂ est fixé au fragment NR₂ ; où le fragment C₁₋₆-alcanediyle est éventuellement substitué par un substituant choisi parmi un groupe hydroxy, un groupe aryle, un groupe Het¹ et un groupe Het² ;
**R₁₂** est un atome d'hydrogène, un groupe alkyle en C₁₋₆, un groupe alcényle en C₂₋₆, un groupe aryl-C₁₋₆-alkyle, un groupe cycloalkyle en C₃₋₇, un groupe C₃₋₇-cycloalkyl-C₁₋₆-alkyle, un groupe aryle, un groupe Het¹, un groupe Het¹-C₁₋₆-alkyle, un groupe Het², un groupe Het²-C₁₋₆-alkyle ;
**R₂** est un atome d'hydrogène ou un groupe alkyle en C₁₋₆ ;
**R₃** est un groupe cycloalkyle en C₃₋₇, un groupe aryle, un groupe Het¹, un groupe Het² ou un groupe alkyle en C₁₋₆ éventuellement substitué par un groupe cycloalkyle en C₃₋₇, un groupe aryle, un groupe Het¹ ou un groupe Het², où chaque groupe cycloalkyle en C₃₋₇, chaque groupe aryle, chaque groupe Het¹ et chaque groupe Het² peuvent être éventuellement substitués par un ou plusieurs groupes choisis parmi un groupe oxo, un groupe alkyloxy en C₁₋₆, un groupe alkyle en C₁₋₆, un groupe alkylsulfonyle en C₁₋₆, un groupe aminosulfonyle, un groupe amino, un groupe C₁₋₆-alkylcarbonylamino, un groupe hydroxy-C₁₋₆-alkyle, un groupe cyano, un groupe C₁₋₆-alkyloxycarbonyle, un groupe aminocarbonyle, un atome d'halogène ou un groupe trifluorométhyle, où chaque groupe amino peut être mono- ou disubstitué par un groupe alkyle en C₁₋₆ ;
**R₄** est choisi parmi le groupe comprenant un atome d'hydrogène, un groupe C₁₋₄-alkyloxycarbonyle, un groupe carboxyle, un groupe aminocarbonyle, un groupe mono- ou di-(C₁₋₄-alkyl)aminocarbonyle, un groupe cycloakyle en C₃₋₇, un groupe alcényle en C₂₋₆, un groupe alcynyle en C₂₋₆, ou un groupe alkyle en C₁₋₆ éventuellement substitué par un ou plusieurs substituants choisis chacun indépendamment parmi un groupe aryle, un groupe Het¹, un groupe Het², un groupe cycloalkyle en C₃₋₇, un groupe C₁₋₄-alkyloxycarbonyle, un groupe carboxyle, un groupe aminocarbonyle, un groupe mono- ou di-(C₁₋₄-alkyl) aminocarbonyle, un groupe aminosulfonyle, un groupe C₁₋₄-alkyl-S(=O)ₜ, un groupe hydroxy, un groupe cyano, un atome d'halogène et un groupe amino éventuellement mono- ou disubstitué, où les substituants sont choisis chacun indépendamment parmi un groupe alkyle en C₁₋₄, un groupe aryle, un groupe aryl-C₁₋₄-alkyle, un groupe cycloakyle en C₃₋₇, un groupe C₃₋₇-cycloalkyl-C₁₋₄-alkyle, un groupe Het¹, un groupe Het², un groupe Het¹-C₁₋₄-alkyle et un groupe Het²-C₁₋₄-alkyle ; et
t vaut zéro, un ou deux.

13. Méthode selon la revendication 12, dans laquelle :
**R₁** est un radical de formule (10) 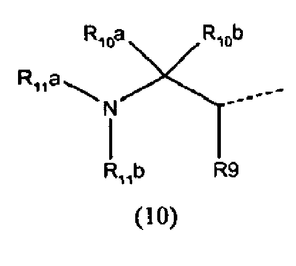
**R9, R₁₀ₐ** et **R_{10b}** sont, chacun indépendamment, un atome d'hydrogène, un groupe C₁₋₄-alkyloxycarbonyle, un groupe carboxyle, un groupe aminocarbonyle, un groupe mono- ou di-(C₁₋₄-alkyl)aminocarbonyle, un groupe cycloalkyle en C₃₋₇, un groupe alcényle en C₂₋₆, un groupe alcynyle en C₂₋₆ ou un groupe alkyle en C₁₋₄ éventuellement substitué par un groupe aryle, un groupe Het¹, un groupe Het², un groupe cycloalkyle en C₃₋₇, un groupe C₁₋₄-alkyloxycarbonyle, un groupe carboxyle, un groupe aminocarbonyle, un groupe mono- ou di-(C₁₋₄-alkyl) aminocarbonyle, un groupe aminosulfonyle, un groupe C₁₋₄-alkyl-S(O)ₜ, un groupe hydroxy, un groupe cyano, un atome d'halogène ou un groupe amino éventuellement mono- ou di-substitué, où les substituants sont choisis chacun indépendamment parmi un groupe alkyle en C₁₋₄, un groupe aryle, un groupe aryl-C₁₋₄-alkyle, un groupe cycloalkyle en C₃₋₇, un groupe C₃₋₇-cycloalkyl-C₁₋₄-alkyle, un groupe Het¹, un groupe Het², un groupe Het¹-C₁₋₄-alkyle et un groupe Het²-C₁₋₄-alkyle ;
où **R₉, R₁₀ₐ** et les atomes de carbone auxquels ils sont fixés peuvent également former un radical cycloalkyle en C₃₋₇ ;
**R_{11b}** est un atome d'hydrogène, un groupe cycloalkyle en C₃₋₇, un groupe alcényle en C₂₋₆, un groupe alcynyle en C₂₋₆, un groupe aryle, un groupe Het¹, un groupe Het² ou un groupe alkyle en C₁₋₄ éventuellement substitué par un atome d'halogène, un groupe hydroxy, un groupe C₁₋₄-alkyl-S(=O)ₜ, un groupe aryle, un groupe cycloalkyle en C₃₋₇, un groupe Het¹, un groupe Het², un groupe amino éventuellement mono- ou disubstitué, où les substituants sont choisis chacun indépendamment parmi un groupe alkyle en C₁₋₄, un groupe aryle, un groupe aryl-C₁₋₄-alkyle, un groupe cycloalkyle en C₃₋₇, un groupe C₃₋₇-cycloalkyl-C₁₋₄-alkyle, un groupe Het¹, un groupe Het², un groupe Het¹-C₁₋₄-alkyle et un groupe Het²-C₁₋₄-alkyle ;
où **R_{11b}** peut être lié au reste de la molécule par l'intermédiaire d'un groupe sulfonyle ;
**t** vaut zéro, un ou deux ;
**L** est un groupe -C(=O)-, un groupe -O-C(=O)-, un groupe -NR₁₂-C(=O)-, un groupe -O-C₁₋₆-alcanediyl-C(=O)-, un groupe -NR₁₂-C₁₋₆-alcanediyl-C(=O)-, un groupe -S(=O)₂-, un groupe -O-S(=O)₂-, un groupe -NR₁₂-S(=O)₂, où soit le groupe C(=O), soit le groupe S(=O)₂ est fixé au fragment NR₂ ; où le fragment C₁₋₆-alcanediyle est éventuellement substitué par un substituant choisi parmi un groupe hydroxy, un groupe aryle, un groupe Het¹ et un groupe Het² ;
**R₁₂** est un atome d'hydrogène, un groupe alkyle en C₁₋₆, un groupe alcényle en C₂₋₆, un groupe aryl-C₁₋₆-alkyle, un groupe cycloalkyle en C₃₋₇, un groupe C₃₋₇-cycloalkyl-C₁₋₆-alkyle, un groupe aryle, un groupe Het¹, un groupe Het¹-C₁₋₆-alkyle, un groupe Het², un groupe Het²-C₁₋₆-alkyle ; et
**R₄** est un atome d'hydrogène, un groupe C₁₋₄-alkyloxy-carbonyle, un groupe carboxyle, un groupe aminocarbonyle, un groupe mono- ou di-(C₁₋₄-alkyl)aminocarbonyle, un groupe cycloakyle en C₃₋₇, un groupe alcényle en C₂₋₆, un groupe alcynyle en C₂₋₆, ou un groupe alkyle en C₁₋₆ éventuellement substitué par un ou plusieurs substituants choisis parmi un groupe aryle, un groupe Het¹, un groupe Het², un groupe cycloalkyle en C₃₋₇, un groupe C₁₋₄-alkyloxycarbonyle, un groupe carboxyle, un groupe aminocarbonyle, un groupe mono- ou di-(C₁₋₄-alkyl) aminocarbonyle, un groupe aminosulfonyle, un groupe C₁₋₄-alkyl-S(=O)ₜ, un groupe hydroxy, un groupe cyano, un atome d'halogène et un groupe amino éventuellement mono- ou di-substitué, où les substituants sont choisis parmi un groupe alkyle en C₂₋₄, un groupe aryle, un groupe aryl-C₁₋₄-alkyle, un groupe cycloakyle en C₃₋₇, un groupe C₃₋₇-cycloalkyl-C₁₋₄-alkyle, un groupe Het¹, un groupe Het², un groupe Het¹-C₁₋₄-alkyle et un groupe Het²-C₁₋₄-alkyle.

14. Méthode selon l'une quelconque des revendications 12 à 13, dans laquelle l'une ou plusieurs des restrictions suivantes s'appliquent :
**R₁** est un atome d'hydrogène, un groupe Het¹, un groupe Het², un groupe aryle, un groupe Het¹-C₁₋₆-alkyle, un groupe Het²-C₁₋₆-alkyle, un groupe aryl-C₁₋₆-alkyle, plus particulièrement, **R₁** est un hétérocycle monocyclique ou bicyclique, saturé ou partiellement insaturé, renfermant de 5 à 8 chaînons du noyau, lequel renferme un ou plusieurs chaînons du noyau hétéroatomiques choisis parmi un atome d'azote, un atome d'oxygène ou un atome de soufre, et lequel est éventuellement substitué, ou un groupe phényle éventuellement substitué par un ou plusieurs substituants ;
**R₂** est un atome d'hydrogène ;
**L** est un groupe -C(=O)-, un groupe -O-C(=O)-, un groupe -O-C₁₋₆-alcanediyl-C(=O)-, plus particulièrement, **L** est un groupe -O-C(=O)- ou un groupe -O-C₁₋₆-alcanediyl-C(=O)-, où, dans chaque cas, le groupe C(=O) est fixé au fragment NR₂ ;
**R₃** est un groupe aryl-C₁₋₄-alkyle, en particulier, un groupe arylméthyle, plus particulièrement un groupe phénylméthyle ;
**R₄** est un groupe alkyle en C₁₋₆ éventuellement substitué, un particulier un groupe alkyle en C₁₋₆ non-substitué ou un groupe alkyle en C₁₋₆ éventuellement substitué par un ou plusieurs substituants choisis parmi un groupe aryle, un groupe Het¹, un groupe Het², un groupe cycloalkyle en C₃₋₇ et un groupe amino éventuellement mono- ou di-substitué, où les substituants sont choisis parmi un groupe alkyle en C₁₋₄, un groupe aryle, un groupe Het¹ et un groupe Het² ;
**R₆** est un atome d'hydrogène ou un groupe méthyle ; et
**R₈** est un atome d'hydrogène ou un groupe méthyle.

15. Méthode selon l'une quelconque des revendications 12 à 14, dans laquelle :
**R₁-L** est un groupe Het¹-O-C(=O), un groupe Het²-C₁₋₆-alcanediyl-O-C(=O), un groupe aryl-O-C₁₋₆-alcanediyl-C(=O) ou un groupe aryl-C(=O).

16. Méthode selon l'une quelconque des revendications 12 à 15, dans laquelle :
**NR₆R₈** est un groupe amino, un groupe monométhylamino ou un groupe diméthylamino.

17. Méthode selon l'une quelconque des revendications 12 à 16, dans laquelle :
**R₁** est un groupe Het¹ ou un groupe Het¹-C₁₋₆-alkyle, et
**L** est un groupe -O-C(=O)- ;
**R₂** est un atome d'hydrogène ;
**R₃** est un groupe phénylméthyle ;
**R₄** est un groupe isobutyle ;
**R₆** est un atome d'hydrogène ; et
**R₈** est un atome d'hydrogène ou un groupe méthyle.

18. Méthode selon l'une quelconque des revendications 12 à 17, dans laquelle le composé (9) est de formule (9''').

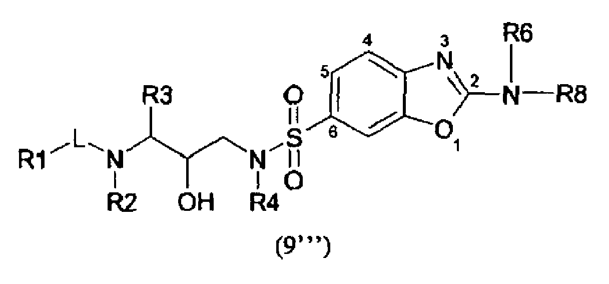

19. Méthode selon l'une quelconque des revendications 12 à 18, **caractérisée en ce que** le composé de formule (9) est sous la forme d'un sel choisi parmi un trifluoroacétate, un fumarate, un chloroacétate et un méthanesulfonate.

20. Utilisation d'un composé selon l'une quelconque des revendications 7 à 11 en tant qu'intermédiaire pour la préparation d'un inhibiteur de protéase de rétrovirus de formule (9).
